# EUROPEAN PATENT APPLICATION

(11) **EP 4 840 999 A1**
(43) Date of publication of application: **18.11.2026**
(21) Application number: 26162832.5
(22) Date of filing: 06.03.2026
(51) Int. Cl.: D04B 1/24, A41C 1/00, A61F 13/00, D04B 1/10, D04B 1/18

(54) **PANTS-TYPE COMPRESSION GARMENT**

(30) Priority: 13.05.2025 JP 2025080580
(71) Applicant: Trestech Co., Ltd., Ama-shi, Aichi 497-0013 (JP)
(72) Inventor: SASAKI, Toshiya, Ama-shi, Aichi, 497-0013 (JP)
(74) Representative: TBK

(57) **Abstract**

A pants-type compression garment (1) can be worn comfortably and effectively promotes lymph flow in the lower abdominal, groin, and genital regions.

A pants-type compression garment (1) includes the pair of left and right leg-side knitted fabric portions (10R, 10L) each having a small-diameter tubular shape, the crotch-side knitted fabric portion (20) connecting the pair of left and right leg-side knitted fabric portions (10R, 10L), and the body-side knitted fabric portion (30) having a large-diameter tubular shape and continuously extending from the pair of left and right leg-side knitted fabric portions (10R, 10L) and the crotch-side knitted fabric portion (20). The pair of left and right leg-side knitted fabric portions (10R, 10L), the crotch-side knitted fabric portion (20), and the body-side knitted fabric portion (30) are integrally knitted by a seamless flat knitting machine.

## Description

### TECHNICAL FIELD

The disclosure relates to pants-type compression garments, such as girdles and shorts, which are knitted by a seamless flat knitting machine having at least a pair of front and rear opposing needle beds, for example, a WHOLEGARMENT^{®} flat knitting machine. In particular, the disclosure relates to pants-type compression garments, such as girdles and shorts, that are configured to be easily donned and improved wearing comfort, and that are effective in preventing and alleviating lymphoedema (and related conditions such as lymph leakage and cellulitis) of the lower abdominal, groin, and genital regions, as well as suppressing exacerbation or worsening of such lymphoedema, thereby providing a therapeutic benefit for such lymphoedema.

### BACKGROUND ART

Lymphedema may occur when lymph flow is impaired due to lymph node dissection or radiation therapy performed in the treatment or surgery of cancer. Lymphedema refers to a condition in which lymph accumulates due to compression, stenosis, or obstruction of lymph nodes or lymphatic vessels caused by various factors, leading to excessive accumulation of fluid, including proteins and lymphocytes, in the interstitial spaces of tissues and cells, thereby causing swelling. For example, when lymph nodes around the abdomen or the inguinal region, such as pelvic lymph nodes or inguinal lymph nodes, are removed in the treatment of gynecological or urological cancers (e.g., uterine cancer, ovarian cancer, prostate cancer), lymphedema may occur in the lower abdominal, groin, or genital regions.

In such lymphedema conditions, there are no pharmacological agents or surgical procedures that are effective for treatment. Currently, treatment relies on physical therapies such as compression therapy, in which pressure is applied to the affected area to promote lymph flow, and treatment methods involving wearing compression garments that apply pressure to the affected area are known.

For example, as medical compression garments, there are pantyhose-type medical compression stockings having a length extending up to the navel. As one example thereof, Patent Document 1: Japanese Unexamined Patent Application Publication No. 2011-115571 discloses a medical stocking in which at least a leg portion is formed of an elastic knitted fabric knitted using a ground yarn and an inlaid yarn having a stronger compressive force than that of the ground yarn. In this medical stocking, the ground yarn is a covering yarn having a total fineness of 75 to 135 decitex, while the inlaid yarn is an elastic yarn including a polyurethane yarn having a recovery stress of 13 to 28 cN at 200% elongation. A compression pressure is set to 45 to 61 hPa in an ankle region extending from the ankle to a lower end of the calf, 23 to 44 hPa in a lower leg region extending from the lower end of the calf to the knee, and 8 to 25 hPa in a thigh region extending from the knee to an upper thigh. Further, the medical stocking is configured such that the compression pressure is gradually reduced in the order of the ankle region, the lower leg region, and the thigh region.

### DISCLOSURE OF THE INVENTION

### TECHNICAL PROBLEM

Unfortunately, conventional pantyhose-type medical compression stockings, including those disclosed in Patent Document 1, are formed by cutting and sewing a thin, high-gauge knitted fabric knitted on a circular knitting machine using relatively fine yarns. Such compression stockings have sewn portions in a panty portion and a crotch portion covering the buttock and the abdominal region, and such sewn portions are stiff and lack stretchability, thereby causing discomfort and impairing skin contact and wearing comfort, during wear. Moreover, such knitted fabric is thin and thus prone to wrinkling, when the stockings are pulled up during wear in order to prevent wrinkling, the knitted fabric corresponding to the affected areas, such as the lower abdominal, groin, and genital regions, is further stretched and thinned due to the poor stretchability of the sewn portions, thereby generating excessive tension. Consequently, the knitted fabric in these areas tends to lift away from the affected areas and fails to contact the affected areas, making it difficult to apply an appropriate pressure thereto. In particular, circular-knitted elastic stockings are knitted by varying dimensions of stitches (or loops) to adjust circumferential dimensions and are knitted by using an elastic yarn continuously inserted into the stitches. Accordingly, such stockings are flexible and easily stretchable, resulting in low stiffness, which makes it difficult to suppress the swelling of the affected area. Conversely, when the stockings are worn with looseness, the stockings get wrinkled and thus bite into the skin, thereby obstructing lymph flow and potentially causing edema or skin inflammation. Therefore, it is difficult for such stockings to apply pressure to the lower abdominal, groin, and genital regions, and to obtain preventive or alleviating effects against lymphoedema in the lower abdominal, groin, and genital regions.

Currently, compression for lymphedema of the lower abdominal, groin, and genital regions is performed by placing a pad, for example, a pad made of urethane, on the lower abdominal, groin, and genital regions and by wearing an elastic stocking over the pad. Unfortunately, wearing a thin elastic stocking of small thickness over a placed pad requires time and effort for putting on and taking off. Moreover, where a wearer moves while wearing such a combination, the elastic stocking tends to bite into the skin and the pad is prone to shift, making it difficult to maintain appropriate compression. This causes discomfort and impairs wearing comfort. In particular, lymphedema in the delicate zone with soft skin tends to be exacerbated and accompanied by lymph leakage. Therefore, in order to reduce patient pain or discomfort as much as possible and to obtain benefits through continued wear, there is a need for a garment that is easy to put on, provides improved wearing comfort, and is effective in preventing, alleviating, suppressing worsening of, and treating lymphoedema (and related conditions such as lymph leakage and cellulitis) of the lower abdominal, groin, and genital regions.

It is an object of the present disclosure to provide a pants-type compression garment that can be worn comfortably and effectively promotes lymph flow in the lower abdominal, groin, and genital regions.

### SOLUTION TO PROBLEM

A pants-type compression garment according to a first aspect includes a pair of left and right leg-side knitted fabric portions (hereinafter also referred to simply as "leg-side knitted fabric portions") each having a small-diameter tubular shape, a crotch-side knitted fabric portion connecting the pair of left and right leg-side knitted fabric portions, and a body-side knitted fabric portion having a large-diameter tubular shape and continuously extending from the leg-side knitted fabric portions and the crotch-side knitted fabric portion. The leg-side knitted fabric portions, the crotch-side knitted fabric portion, and the body-side knitted fabric portion are integrally knitted by a seamless flat knitting machine.

Each of the leg-side knitted fabric portions has a tubular shape having a diameter smaller than that of the body-side knitted fabric portion, and is configured to receive and mainly cover a leg of a wearer when worn. The length of each leg-side knitted fabric portion (inseam length) is not particularly limited, and may be a length of a part of or the entirety of the thigh, or a length extending to above or below the knee, to the calf, to above or below the ankle, to the heel, or to the toes.

The crotch-side knitted fabric portion is continuously knitted in a course direction (a transverse direction of the knitted fabric in which loops are arranged in rows in a widthwise direction) between the pair of left and right leg-side knitted fabric portion so as to connect the pair of left and right leg-side knitted fabric portions, and corresponds to the groin and genital regions of the human body when worn.

The body-side knitted fabric portion has a tubular shape having a diameter larger than that of each leg-side knitted fabric portion, and is continuously knitted in a wale direction (a longitudinal direction of the knitted fabric in which loops are arranged in columns in a lengthwise direction) from the leg-side knitted fabric portions and from the crotch-side knitted fabric portion. The body-side knitted fabric portion is configured to cover the abdominal region and the buttock region of the human body when worn. The length of the body-side knitted fabric portion (waist length) is not particularly limited, and may be a length extending to below the navel, to above the navel and below the waist, to above the waist, or to the bust level.

In the pants-type compression garment according to the first aspect, the pair of left and right leg-side knitted fabric portions and the body-side knitted fabric portion are knitted in a textured knitting pattern forming raised portions and recessed portions alternately arranged in a circumferential direction and continuously extending in a wale direction (a longitudinal direction of the knitted fabric). Examples of textured knitting pattern include rib knitting, pique knitting, seed knitting, moss knitting, and waffle knitting. The raised and recessed portions may be formed on only one of the front surface and the rear surface of the knitted fabric, or may be formed on both the front and rear surfaces. When formed on both the front and rear surfaces, the raised and recessed portions are convex and concave portions, in which the raised portions on one surface correspond to the recessed portions on the other surface, and the recessed portions on one surface correspond to the raised portions on the other surface.

In the pants-type compression garment according to the first aspect, the pair of left and right leg-side knitted fabric portions and the body-side knitted fabric portion are continuously knitted such that the number of stitches on the crotch-side knitted fabric portion side is sequentially decreased from each of the leg-side knitted fabric portions toward the body-side knitted fabric portion. The phrase "the pair of left and right leg-side knitted fabric portions and the body-side knitted fabric portion are continuously knitted such that the number of stitches on the crotch-side knitted fabric portion side is sequentially decreased from each of the leg-side knitted fabric portions toward the body-side knitted fabric portion." means that, when the body-side knitted fabric portion is continuously knitted in a wale direction from each of the left and right leg-side knitted fabric portions, the number of stitches decreases stepwise on the crotch-side knitted fabric portion side, that is, at a connection region among the leg-side knitted fabric portions, the body-side knitted fabric portion, and the crotch-side knitted fabric portion, which corresponds to an inner proximal region of the thigh of the human body when worn, so that each of the leg-side knitted fabric portions has an increased area in a region corresponding to the inner proximal region of the thigh when worn.

In the pants-type compression garment according to the first aspect, the body-side knitted fabric portion includes a low-stretch region covering, when worn, the regions extending from the genital and groin regions to the lower abdominal region of the human body and having a lower elongation rate than other regions. The low-stretch region refers to a high-stiffness region having lower stretchability and higher stiffness than other regions. The low-stretch region is mainly formed in a knitted fabric corresponding to the front of the body-side knitted fabric portion and covers, when worn, the regions extending from the genital and groin regions to the lower abdominal region of the human body. The term "stiffness" in a broad sense, means a force required to stretch the knitted fabric or a force resisting stretching of the knitted fabric. The term "stiffness" in a narrow sense, means an increase in compressive pressure generated when the knitted fabric is stretched by 1 cm in the transverse direction. In this specification, the term "stiffness" is used in the broad sense.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by forming knit stitches and miss stitches. The base yarn is a knitting yarn used to knit the leg-side knitted fabric portions and the body-side knitted fabric portion, that is, used to form an overall shape of the leg-side knitted fabric portions and the body-side knitted fabric portion. The elastic yarn is knitted into the loops of the base yarn by intermeshing the loops and by missing the loops, and has higher elasticity than the base yarn.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by forming knit stitches and miss stitches. The body-side knitted fabric portion includes a low-stretch region covering the regions extending from the genital and groin regions to the lower abdominal region of the human body of the human body and having a lower elongation rate than other regions. The low-stretch region has a lower elongation rate by virtue of a reduced proportion of stitch transfer, as compared with other regions, in portions where the elastic yarn is knitted into the loops of the base yarn.

The base yarn is a knitting yarn used to knit the leg-side knitted fabric portions and the body-side knitted fabric portion, that is, used to form an overall shape of the leg-side knitted fabric portions and the body-side knitted fabric portion. The elastic yarn is knitted into the loops of the base yarn by intermeshing the loops and by missing the loops, and has higher elasticity than the base yarn. The low-stretch region refers to a high-stiffness region having lower stretchability and higher stiffness than other regions. The low-stretch region is mainly formed in a knitted fabric corresponding to the front of the body-side knitted fabric portion and covers, when worn, the regions extending from the genital and groin regions to the lower abdominal region of the human body of the human body. The term "stiffness" in a broad sense, means a force required to stretch the knitted fabric or a force resisting stretching of the knitted fabric. The term "stiffness" in a narrow sense, means an increase in compressive pressure generated when the knitted fabric is stretched by 1 cm in the transverse direction. In this specification, the term "stiffness" is used in the broad sense. The low-stretch region may include no transferred stitches, or may include a reduced proportion of transferred stitches as compared with the other regions. The transferred stitches include stitches formed by lace knitting and tuck knitting.

In the pants-type compression garment according to the first aspect, the body-side knitted fabric portion has the front portion having a shorter length than the back portion. The front portion of the body-side knitted fabric portion is configured to cover the abdominal region of the human body, whereas the back portion of the body-side knitted fabric portion is configured to cover the buttock region of the human body, when worn.

In the pants-type compression garment according to the first aspect, the pair of left and right leg-side knitted fabric portions and the body-side knitted fabric portion are knitted base yarn and elastic yarn. The elastic yarn is interknitted with the loops of the base yarn by forming knit stitches and miss stitches. Further, the body-side knitted fabric portion has the front portion shorter in length than the back portion by virtue of a reduced proportion of stitch transfer, as compared with the back, in portions on the front portion where the elastic yarn is knitted into the loops of the base yarn.

The base yarn is a knitting yarn used to knit the leg-side knitted fabric portions and the body-side knitted fabric portion, that is, used to form an overall shape of the leg-side knitted fabric portions and the body-side knitted fabric portion. The elastic yarn is knitted into the loops of the base yarn by intermeshing the loops and by missing the loops, and has higher elasticity than the base yarn. The body-side knitted fabric portion has the front portion having a shorter length than the back portion by virtue of a reduced proportion of stitch transfer in the front as compared with the back. The front portion of the body-side knitted fabric portion may include no transferred stitches, or may include a reduced proportion of transferred stitches as compared with the back portion. The front portion of the body-side knitted fabric portion is configured to cover the abdominal region of the human body, whereas the back portion of the body-side knitted fabric portion corresponds to cover the buttock region of the human body, when worn.

In the pants-type compression garment according to the first aspect, the pair of left and right leg-side knitted fabric portions, the crotch-side knitted fabric portion, and the body-side knitted fabric portion are formed of a base yarn having a fineness of preferably 1000 to 3000 denier, more preferably 1200 to 3000 denier, and further preferably 1500 to 3000 denier. The base yarn is a knitting yarn used to knit the leg-side knitted fabric portions, the crotch-side knitted fabric portion, and the body-side knitted fabric portion, that is, used to form an overall shape of the leg-side knitted fabric portions, the crotch-side knitted fabric portion, and the body-side knitted fabric portion.

In the pants-type compression garment according to the first aspect, the textured knitting pattern is a rib knitting pattern. The rib knitting forms raised and recessed portions on both the front surface and the rear surface, wherein the raised portions on one surface correspond to the recessed portions on the other surface, and the recessed portions on one surface correspond to the raised portions on the other surface.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by forming knit stitches and miss stitches. With respect to the loops of the base yarn that appear as raised portions on an inner surface of the textured knitting and as recessed portions on an outer surface thereof, the elastic yarn is missed, whereas with respect to the loops of the base yarn that appear as recessed portions on the inner surface and as raised portions on the outer surface thereof, the elastic yarn is interknitted. The base yarn is a knitting yarn used to knit the leg-side knitted fabric portions and the body-side knitted fabric portion, that is, used to form an overall shape of the leg-side knitted fabric portions and the body-side knitted fabric portion. The elastic yarn is knitted into the loops of the base yarn by intermeshing the loops and by missing the loops, and has higher elasticity than the base yarn. The inner surface (rear surface) of the textured knitting refers to a surface that comes into contact with skin when worn, and the outer surface (front surface) thereof refers to an opposite surface serving as a design surface.

In the pants-type compression garment according to the first aspect, the crotch-side knitted fabric portion includes a discontinuous region having no continuously formed raised and recessed portions. The discontinuous region is a region having no continuously formed raised and recessed portions formed by rib knitting, that is, a region in which raised and recessed portions are interrupted.

In the pants-type compression garment according to the first aspect, the crotch-side knitted fabric portion is knitted using base yarn and elastic yarn. The elastic yarn is interknitted into the loops of the base yarn by knit stitches. The base yarn is a knitting yarn used to knit the crotch-side knitted fabric portion. The elastic yarn is knitted into the loops of the base yarn by intermeshing the loops.

### ADVANTAGEOUS EFFECTS OF THE INVENTION

A pants-type compression garment (hereinafter also referred to simply as "garment") according to a first aspect includes a pair of left and right leg-side knitted fabric portions (hereinafter also referred to simply as "leg-side knitted fabric portions") each having a small-diameter tubular shape, a crotch-side knitted fabric portion connecting the leg-side knitted fabric portions, and a body-side knitted fabric portion having a large-diameter tubular shape and continuously extending from the leg-side knitted fabric portions and the crotch-side knitted fabric portion. The leg-side knitted fabric portions, the crotch-side knitted fabric portion, and the body-side knitted fabric portion are integrally knitted by a seamless flat knitting machine. Therefore, the pants-type compression garment has no sewn portions (sewn seams) and thus provides improved skin contact feel. Further, since the pants-type compression garment has no sewn portions, the knitted fabric of the pants-type compression garment is less likely to become overly tensioned and lift away from the body, when worn, thereby allowing appropriate pressure to be readily applied even to the wearer's lower abdominal, groin, and genital regions. In addition, flat-knitting enables formation of a thick and stiff knitted fabric having high stiffness using a relatively thick yarn, thereby allowing generation of a muscle pump effect and a massage effect in response to body movement. Moreover, the thick and stiff knitted fabric is less likely to wrinkle, thereby preventing obstruction of lymph flow caused by localized wrinkling cutting into skin, and improving wearing comfort. Thus, the garment enables comfortable wear and effectively promotes lymph flow in the lower abdominal, groin, and genital regions of the human body.

In the pants-type compression garment according to the first aspect, the pair of left and right leg-side knitted fabric portions and the body-side knitted fabric portion are knitted in a textured knitting pattern having raised portions and recessed portions alternately arranged in a circumferential direction and continuously extending in a wale direction. Since the raised portions and the recessed portions are alternately arranged in the transverse direction, that is, the recessed portions (grooves) present between adjacent raised portions can accommodate variation in body shape by varying spacings between adjacent raised portions in response to body shape change and body movement. As described above, the garment is less likely to wrinkle, thereby preventing obstruction of lymph flow caused by localized wrinkling cutting into skin. In addition to the effects described above, the garment allows the raised portions on an inner surface to be kept in close contact with a part of the body to apply pressure thereto. Therefore, the garment allows appropriate pressure to be readily applied to the lower abdominal, groin, and genital regions. Further, alternating arrangement of the raised portions and the recessed portions results in variations in pressure intensity, thereby improving a massage effect and skin flexibility and further improving lymph flow.

In the pants-type compression garment according to the first aspect, the pair of left and right leg-side knitted fabric portions and the body-side knitted fabric portion are continuously knitted such that the number of stitches on the crotch-side knitted fabric portion side is sequentially decreased from each of the leg-side knitted fabric portions toward the body-side knitted fabric portion. This configuration allows the leg-side knitted fabric portions to have an increased area in a region corresponding to the inner proximal region of the thigh when worn, thereby preventing the knitted fabric from lifting away even along a three-dimensional curved shape in the regions extending from the thigh region to the genital and groin regions of the human body and enabling close contact of the knitted fabric with the regions extending from the thigh region to the genital and groin regions. Therefore, in addition to the effects described above, the garment can enhance promotion of lymph flow in the lower abdominal, groin, and genital regions.

In the pants-type compression garment according to the first aspect, the body-side knitted fabric portion includes a low-stretch region having a lower elongation rate than other regions and covering, when worn, the regions extending from the genital and groin regions to the lower abdominal region. This configuration allows the region covering the regions extending from the genital and groin regions to the lower abdominal region to have an increased stiffness, thereby enhancing a muscle pump effect and a massage effect. Meanwhile, regions other than the regions extending from the genital and groin regions to the lower abdominal region are easily stretchable, thereby facilitating donning and doffing of the garment, preventing an excessively tight feeling during wear, and preventing slippage, looseness, and wrinkling of the garment. Therefore, in addition to the effects described above, the garment can achieve both wearing comfort and an effect of promoting lymph flow in the lower abdominal, groin, and genital regions.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by knit stitches and miss stitches. In portions where the elastic yarn is knitted into the loops of the base yarn, stretchability can be restricted, while in portions where the elastic yarn is missed with respect to the loops of the base yarn, stretchability is readily provided without causing the elastic yarn to appear on an inner surface, thereby reducing contact with the skin. Therefore, in addition to the effects described above, the garment can achieve both comfortable compression and prevention of skin damage caused by the elastic yarn.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by knit stitches and miss stitches. The body-side knitted fabric portion includes a low-stretch region covering, when worn, the regions extending from the genital and groin region to the lower abdominal region and having a lower elongation rate than other regions. The low-stretch region has the lower elongation rate by virtue of a reduced proportion of stitch transfer in portions where the elastic yarn is knitted (not missed) into the loops of the base yarn, as compared with other regions.

Since the regions covering the lower abdominal, groin, and genital regions has a lower elongation rate by virtue of a reduced proportion of stitch transfer in portions where the elastic yarn is knitted into the loops of the base yarn, as compared with other regions, the region has restricted stretch of the elastic yarn, a lower elongation, and higher stiffness, thereby enhancing a muscle pump effect and a massage effect. Meanwhile, since regions other than the region covering the lower abdominal, groin, and genital regions have an increased proportion of stitch transfer in portions where the elastic yarn are knitted into the loops of the base yarn, as compared with the low-stretch region, the regions are more easily stretchable, thereby facilitating donning and doffing of the garment, preventing an excessively tight feeling during wear, and preventing slippage, looseness, and wrinkling of the garment. Therefore, in addition to the effects described above, wearing comfort and an effect of promoting lymph flow in the lower abdominal, groin, and genital regions. Further, in portions where the elastic yarn is knitted into the loops of the base yarn, stretchability can be restricted, while in portions where the elastic yarn is missed with respect to the loops of the base yarn, stretchability is readily provided without causing the elastic yarn to appear on an inner surface. Therefore, the garment can achieve both comfortable compression and prevention of skin damage caused by the elastic yarn. In particular, the low-stretch region is provided with a lower elongation rate by reducing a proportion of stitch transfer in portions where the elastic yarn is knit-stitched to the loops of the base yarn, as compared with other regions, rather than by changing the type of the base yarn. Accordingly, the stitch formation of the low-stretch region is stabilized and manufacturing cost is reduced.

In the pants-type compression garment according to the first aspect, the body-side knitted fabric portion has the front portion having a shorter length than the back portion. Accordingly, when worn, lifting and wrinkling of the knitted fabric are prevented on the front portion covering the abdominal region, thereby allowing the knitted fabric to readily apply pressure to the lower abdominal, groin, and genital regions. Meanwhile, a feeling of tightness caused by body movement is less likely to occur on the back portion covering on the buttock region. Therefore, in addition to the effects described above, the garment can achieve both wearing comfort and an effect of promoting lymph flow in the lower abdominal, groin, and genital regions.

In the pants-type compression garment according to the first aspect, the pair of left and right leg-side knitted fabric portions and the body-side knitted fabric portion are knitted using base yarn and elastic yarn. The elastic yarn is interknitted with the loops of the base yarn by knit stitches and miss stitches. The body-side knitted fabric portion has the front portion having a shorter length than the back portion by virtue of a reduced proportion of stitch transfer in the front portion where the elastic yarn is knitted into the loops of the base yarn, as compared with the back portion. In portions where the elastic yarn is knitted into the loops of the base yarn, stretchability can be restricted, while in portions where the elastic yarn is missed with respect to the loops of the base yarn, stretchability is readily provided without causing the elastic yarn to appear on an inner surface, thereby reducing contact with the skin. Further, since the body-side knitted fabric portion has the front portion having a shorter length than the back portion, lifting and wrinkling of the knitted fabric are prevented on the front portion covering the abdominal region when worn, thereby allowing the knitted fabric to readily apply pressure to the lower abdominal, groin, and genital regions. Meanwhile, a feeling of tightness caused by body movement is less likely to occur on the back portion covering on the buttock region. Therefore, in addition to the effects described above, the garment can achieve wearing comfort, prevention of skin damage caused by the elastic yarn, and an effect of promoting lymph flow in the lower abdominal, groin, and genital regions in combination. In particular, the garment has a difference in length between the front portion and the back portion by reducing a proportion of stitch transfer in portions on the front portion where the elastic yarn is knitted into the loops of the base yarn, as compared with the back portion, rather than by changing the type of the base yarn. This allows stitch formation to be stabilized and enables reduction in manufacturing cost. Accordingly, the stitch formation of the low-stretch region is stabilized and manufacturing cost is reduced.

In the pants-type compression garment according to the first aspect, the pair of left and right leg-side knitted fabric portions, the crotch-side knitted fabric portion, and the body-side knitted fabric portion are knitted using base yarn having a fineness preferably of 1000 to 3000 denier, more preferably 1200 to 3000 denier, and further preferably 1500 to 3000 denier. This configuration allows the knitted fabric to have high stiffness, thereby enabling enhancement of a muscle pump effect and a massage effect in response to body movements. Moreover, such a stiff knitted fabric is less likely to wrinkle and cause obstruction of lymph flow due to localized wrinkling cutting into the skin, thereby providing improved waring comfort. Therefore, in addition to the effects described above, the garment can provide better wearing comfort and can further promote lymph flow in the lower abdominal, groin, and genital regions.

In the pants-type compression garment according to the first aspect, the textured knitting pattern is rib knitting pattern. This configuration allows spacing between adjacent raised portions to easily vary. Accordingly, even when the body moves, the raised portions on an inner surface can remain in close contact with a part of the body to apply pressure thereto. Therefore, in addition to the effects described above, the garment can further promote lymph flow.

In the pants-type compression garment according to the first aspect, the pair of left and right leg-side knitted fabric portions and the body-side knitted fabric portion are knitted using base yarn and elastic yarn. The elastic yarn is interknitted into the loops of the base yarn by knit stitches and miss stitches. The elastic yarn is knitted into the loops of the base yarn that appear as recessed portions on the inner surface and as raised portions on the outer surface, while the elastic yarn is missed with respect to the loops of the base yarn that appear as raised portions on an inner surface of the textured knitting and as recessed portions on an outer surface thereof. In portions where the elastic yarn is knitted into the loops of the base yarn, stretchability can be restricted, whereas in portions where the elastic yarn is missed with respect to the loops of the base yarn, stretchability is readily provided without causing the elastic yarn to appear on an inner surface, thereby reducing contact with the skin and preventing skin damage caused by the elastic yarn. Therefore, in addition to the effects described above, the garment does not cause irritation to a wearer due to the material, stretchability, friction, or the like of the elastic yarn and does not cause skin inflammation due to the elastic yarn even during long-term wear, thereby enabling comfortable wear. Further, the garment achieves appropriate stretchability and high stiffness in combination, thereby enabling both comfortable wear and further promotion of lymph flow through a muscle pump effect and a massage effect.

In the pants-type compression garment according to the first aspect, the crotch-side knitted fabric portion includes a discontinuous region having no continuously formed raised and recessed portions. Accordingly, even when the lower limbs move, the discontinuous region having no continuously formed raised and recessed portions can deform in response to the movement, thereby allowing the knitted fabric to be less likely to experience excessive tension or pulling and to cause abrasion, and facilitating contact of the knitted fabric surrounding the discontinuous region with the genital and groin regions. Therefore, in addition to the effects described above, the garment is less likely to cause discomfort and foreign-body sensation in the genital and the groin regions and can enhance an effect of promoting lymph flow in the lower abdominal, groin, and genital regions.

In the pants-type compression garment according to the first aspect, the crotch-side knitted fabric portion is knitted using base yarn and elastic yarn. The elastic yarn is interknitted with the loops of the base yarn by knit stitches, thereby increasing stretchability. Accordingly, even when the lower limbs move, the discontinuous region can deform in response to the movement, thereby facilitating contact between the knitted fabric surrounding the discontinuous region and the genital and groin regions. Therefore, in addition to the effects described above, the garment can further promote lymph flow lymph flow in the lower abdominal, groin, and genital regions.

### BRIEF DESCRIPTION OF DRAWINGS

[FIG 1] FIG. 1 is a front view of an outer surface of a pants-type compression garment according to an embodiment of the present disclosure.
[FIG 2] FIG. 2 is a back view of an outer surface side of the pants-type compression garment according to the embodiment of the present disclosure.
[FIG 3] FIG. 3 is a front view of an inner surface of the pants-type compression garment according to the embodiment of the present disclosure.
[FIG 4] FIG. 4 is a back view of an inner surface side of the pants-type compression garment according to the embodiment of the present disclosure.
[FIG 5] FIG. 5 illustrates the pants-type compression garment according to the embodiment of the present disclosure when worn, as viewed from the front.
[FIG 6] FIG. 6 illustrates the pants-type compression garment according to the embodiment of the present disclosure when worn, as viewed from the back.
[FIG 7] FIG. 7 illustrates the pants-type compression garment according to the embodiment of the present disclosure when worn, as viewed from the left side.
[FIGS. 8A and 8B] FIG. 8A shows an enlarged view of a portion (I) shown in FIG. 1 and a photograph of an implementation. FIG. 8B shows an enlarged view of a portion (II) shown in FIG. 1 and a photograph of the implementation.
[FIGS. 9A and 9B] FIG. 9A shows an enlarged view of a portion (III) shown in FIG. 1 and a photograph of the implementation. FIG. 9B shows an enlarged view of a portion (IV) shown in FIG. 1 and a photograph of the implementation.
[FIGS. 10A and 10B] FIG. 10A shows an enlarged view of a portion (V) shown in FIG. 1. FIG. 10B shows an enlarged view of a portion (VI) shown in FIG. 2 and a photograph of the implementation.
[FIGS. 11A and 11B] FIG. 11A shows an enlarged view of a portion (VII) shown in FIG. 2 and a photograph of the implementation. FIG. 11B shows an enlarged view of a portion (VIII) shown in FIG. 2 and a photograph of the implementation.
[FIGS. 12A and 12B] FIG. 12A shows an enlarged view of a portion (IX) shown in FIG. 3 and a photograph of the implementation. FIG. 12B shows an enlarged view of a portion (X) shown in FIG. 3 and a photograph of the implementation.
[FIGS. 13A and 13B] FIG. 13A shows an enlarged view of a portion (XI) shown in FIG. 5 and a photograph of the implementation. FIG. 13B shows an enlarged view of a portion (XII) shown in FIG. 5 and a photograph of the implementation.
[FIGS. 14A and 14B] FIG. 14A shows an enlarged view of a portion (XIII) shown in FIG. 5 and a photograph of the implementation. FIG. 14B shows an enlarged view of a portion (XIV) shown in FIG. 6 and a photograph of the implementation.
[FIG. 15] FIG. 15 shows an enlarged view illustrating an outer surface of a crotch-side knitted fabric and its peripheral portions in the unfolded state of the pants-type compression garment according to the embodiment of the present disclosure and a photograph of the implementation.
[FIG. 16] FIG. 16 shows an enlarged view illustrating an inner surface of a crotch-side knitted fabric and its peripheral portions in the unfolded state of the pants-type compression garment according to the embodiment of the present disclosure and a photograph of the implementation.
[FIGS. 17A and 17B] FIG. 17A illustrates recess and raised portions of a body-side knitted fabric portion of the pants-type compression garment according to the embodiment of the present disclosure when worn. FIG. 17B illustrates recess and raised portions of leg-side knitted fabric portions of the pants-type compression garment according to the embodiment of the present disclosure when worn.
[FIGS. 18A and 18B] FIG. 18A illustrates a base yarn and an elastic yarn in rib knitting of the leg-side knitted fabric portions and the body-side knitted fabric portion of the pants-type compression garment according to the embodiment of the present disclosure. FIG. 18B illustrates a schematic diagram of knitting courses for rib knitting of the leg-side knitted fabric portions and the body-side knitted fabric portion of the pants-type compression garment according to the embodiment of the present disclosure.

### DESCRIPTION OF EMBODIMENT

An embodiment of the present disclosure is described hereafter based on drawings.
In the embodiment of the present disclosure, the same marks and the same codes shown in the drawings mean the same or equivalent function parts. Thus, overlapped description thereof has been omitted here.

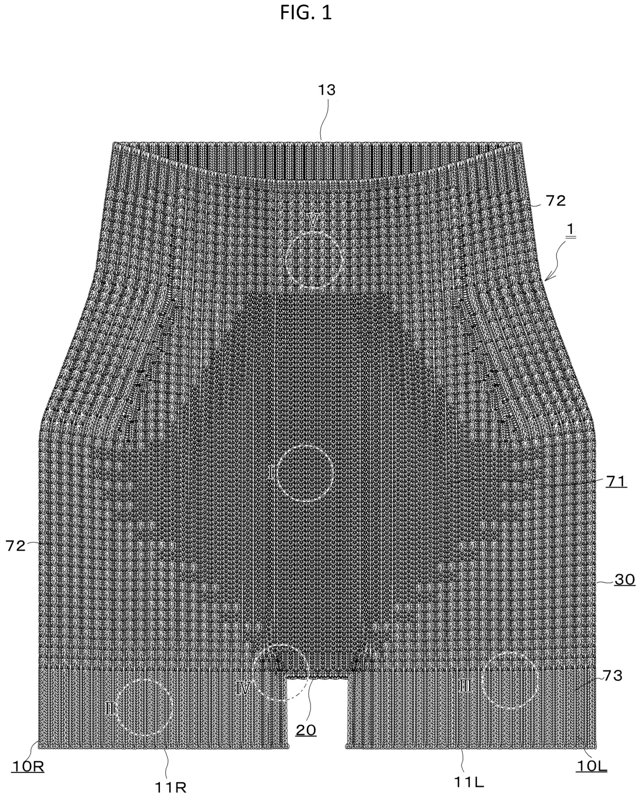

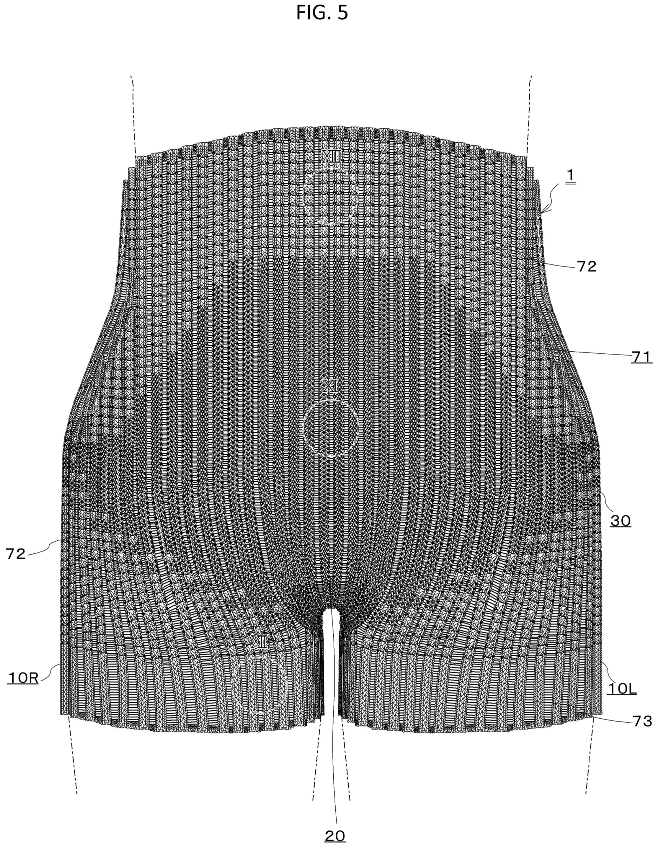

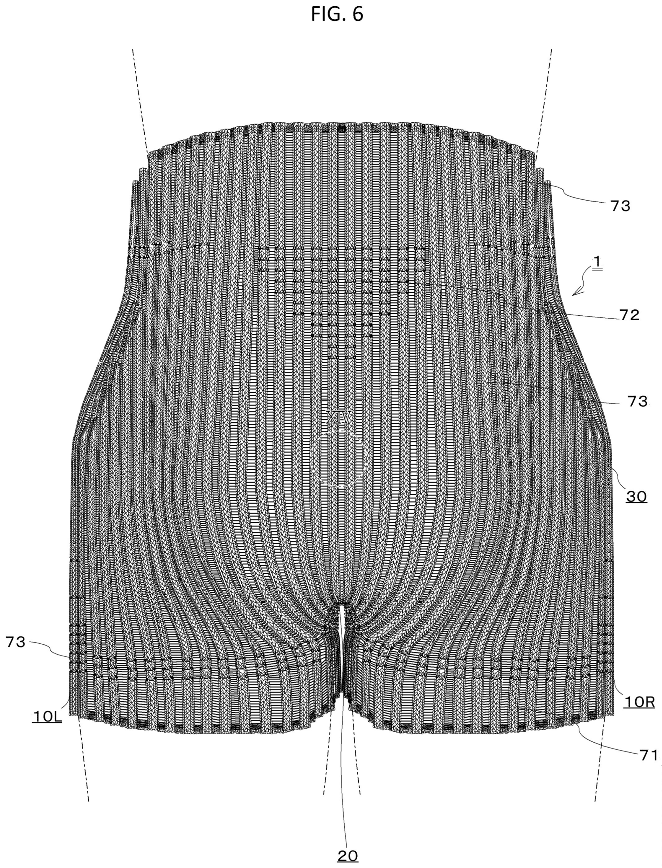

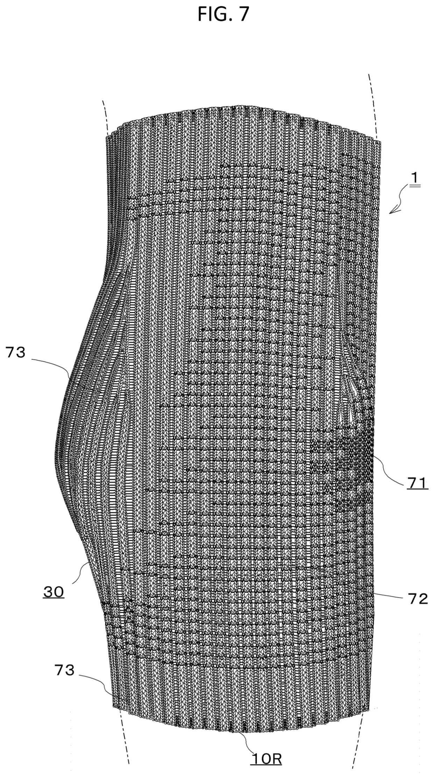

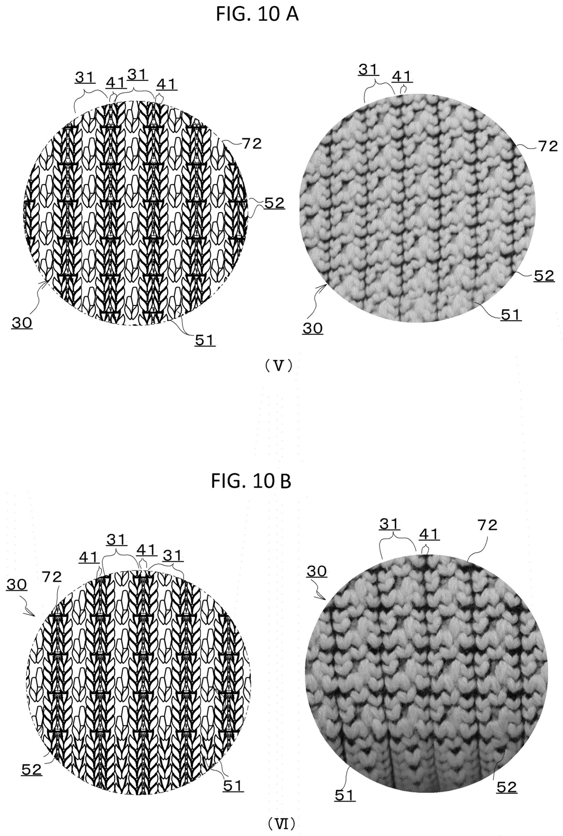

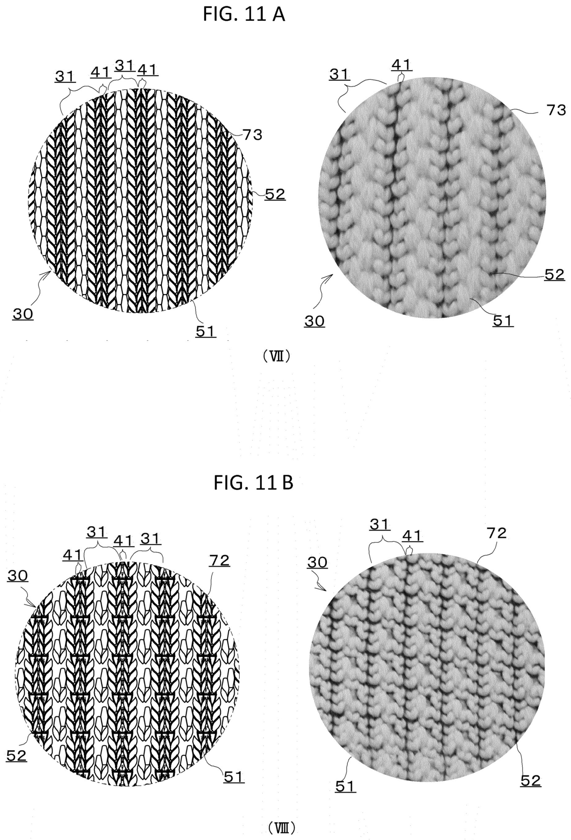

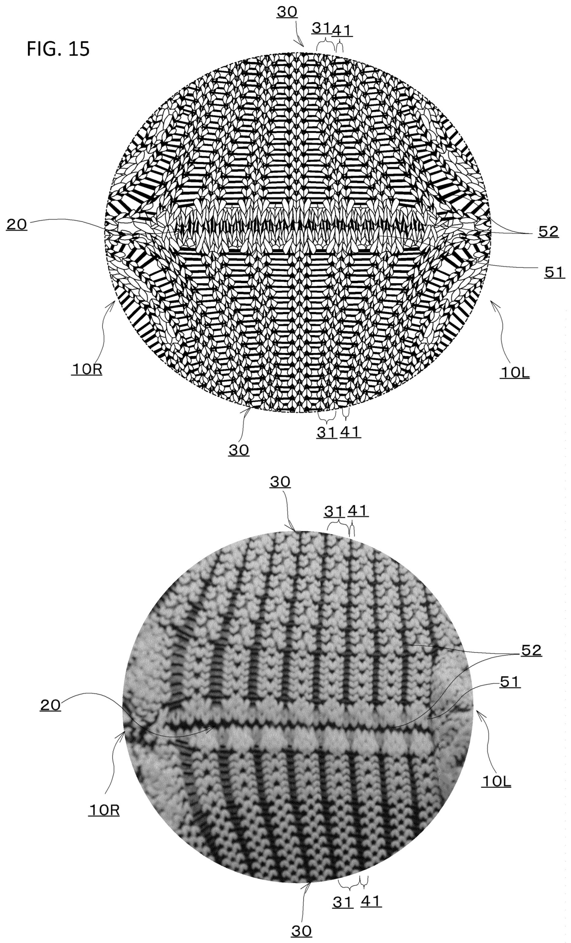

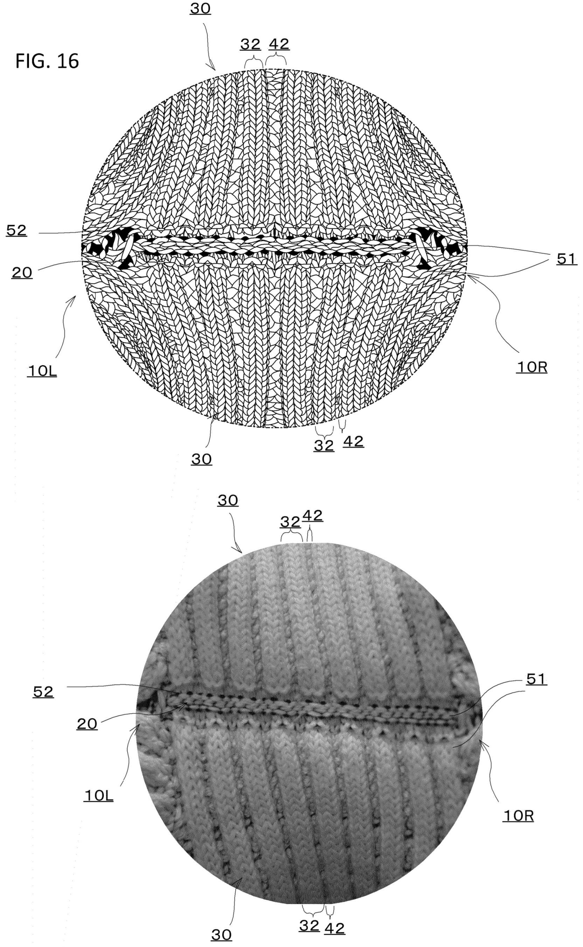

[EMBODIMENT] A pants-type compression garment according to an embodiment of the present disclosure is described with reference to FIGS. 1 to 18. The embodiment of the present disclosure exemplifies a girdle-type compression garment as a pants-type compression garment.

As shown in FIGS. 1 to 7, the pants-type compression garment 1 (hereinafter also referred to simply as "garment 1")according to the embodiment of the present disclosure includes a pair of left and right leg-side knitted fabric portions 10R and 10L (a right leg-side knitted fabric portion 10R and a left leg-side knitted fabric portion 10L), each having a small-diameter tubular shape; a crotch-side knitted fabric portion 20 connecting the pair of left and right leg-side knitted fabric portions 10R and 10L (that is, between the right leg-side knitted fabric portion 10R and the left leg-side knitted fabric portion 10L); and a body-side knitted fabric portion 30 having a large-diameter tubular shape and continuously extending from the pair of left and right leg-side knitted fabric portions 10R and 10L and the crotch-side knitted fabric portion 20. The pair of left and right leg-side knitted fabric portions 10R and 10L (hereinafter also referred to simply as "leg-side knitted fabric portions 10R and 10L "), the crotch-side knitted fabric portion 20, and the body-side knitted fabric portion 30 are integrally and continuously flat-knitted without seams. The garment 1 is worn on the lower half of the human body by inserting the feet through an upper opening 31 of the body-side knitted fabric portion 30 toward lower openings 11R and 11L of the leg-side knitted fabric portions 10R and 10L, and, when worn, covers at least the abdominal region, the buttock region, the groin region, the genital region, and the thigh region of the human body.

The pants-type compression garment 1 according to the present embodiment is integrally and seamlessly knitted by a seamless flat knitting machine, which is a weft knitting machine that continuously knits yarn in a transverse direction. Knitting is started from lower ends of the leg-side knitted fabric portions 10R and 10L, and after knitting to a predetermined length, the crotch-side knitted fabric portion 20 is continuously knitted in a transverse direction between the leg-side knitted fabric portions 10R and 10L, thereby connecting the leg-side knitted fabric portions 10R and 10L. Thereafter, the body-side knitted fabric portion 30 is continuously knitted in a longitudinal direction from the leg-side knitted fabric portions 10R and 10L and the crotch-side knitted fabric portion 20. Thus, the pants-type compression garment 1 includes the leg-side knitted fabric portions 10R and 10L each having a small-diameter tubular shape and being configured to cover the thigh regions of the human body; the crotch-side knitted fabric portion 20 connecting the leg-side knitted fabric portions 10R and 10L and being configured to cover the groin and genital regions of the human body; and the body-side knitted fabric portion 30 having a large-diameter tubular shape, continuously extending in the longitudinal direction from the leg-side knitted fabric portions 10R and 10L and the crotch-side knitted fabric portion 20 and being configured to cover the regions extending from the groin and genital regions to the abdominal region and buttock region of the human body. The leg-side knitted fabric portions 10R and 10L, the crotch-side knitted fabric portion 20, and the body-side knitted fabric portion 30 are continuously knitted as a single piece and integrally formed in a seamless manner.

In the pants-type compression garment 1 according to the present embodiment, the body-side knitted fabric portion 30 has a larger-diameter tubular shape with an upper end opened (the upper opening 31). The pair of left and right leg-side knitted fabric portions 10R and 10L, which are branched downward from the body-side knitted fabric portion 30, have a smaller-diameter tubular shape than the body-side knitted fabric portion 30, with lower ends opened (the lower openings 11R and 11L). The crotch-side knitted fabric portion 20 having a predetermined width is formed at a lower central portion where the body-side knitted fabric portion 30 branches into the pair of left and right leg-side knitted fabric portions 10R and 10L, that is, between the right leg-side knitted fabric portion 10R and the left leg-side knitted fabric portion 10L. The garment 1 is worn on the lower half of the human body by inserting feet through a the upper opening 31 of the body-side knitted fabric portion 30, passing through the interiors of the tubular body-side knitted fabric portion 30 and the leg-side knitted fabric portions 10R and 10L, and exiting from the lower openings 11R and 11L of the leg-side knitted fabric portions 10R and 10L. The garment 1 has a girdle-type configuration capable of covering the regions extending from thigh region to the genital region, the lower abdominal region, the groin region, and the buttock region. That is, the body-side knitted fabric portion 30 is configured to cover the regions extending from the genital and groin regions to the lower abdominal region and the buttock region; the crotch-side knitted fabric portion 20 is configured to cover the genital and the groin regions; and the leg-side knitted fabric portions 10R and 10L branched from the body-side knitted fabric portion 30 are configured to cover the thigh region.

The pants-type compression garment 1 according to the present embodiment is formed by continuously knitting the leg-side knitted fabric portions 10R and 10L, the crotch-side knitted fabric portion 20, and the body-side knitted fabric portion 30 using a seamless flat knitting machine, which knits yarns by reciprocating motion in a transverse direction. Specifically, first, the leg-side knitted fabric portions 10R and 10L are each knitted in a tubular shape. Thereafter, the crotch-side knitted fabric portion 20 is knitted between the leg-side knitted fabric portions 10R and 10L, thereby connecting the leg-side knitted fabric portions 10R and 10L. Subsequently, the body-side knitted fabric portion 30 is continuously knitted in a tubular shape from the leg-side knitted fabric portions 10R and 10L and the crotch-side knitted fabric portion 20. Thus, the body-side knitted fabric portion 30 is formed in a tubular shape and configured to cover the regions extending from the genital and groin regions to the lower abdominal region and the buttock region, and the leg-side knitted fabric portions 10R and 10L are each formed in a tubular shape and configured to cover the left and right thigh regions. The tubular diameter of the body-side knitted fabric portion 30 is larger than that of the leg-side knitted fabric portions 10R and 10L; in other words, the tubular diameter of the leg-side knitted fabric portions 10R and 10L is smaller than that of the body-side knitted fabric portion 30. The crotch-side knitted fabric portion 20 is formed at a lower portion of the body-side knitted fabric portion 30 and between the leg-side knitted fabric portions 10R and 10L.

The pants-type compression garment 1 according to the present embodiment is a knitted fabric in which the leg-side knitted fabric portions 10R and 10L, the crotch-side knitted fabric portion 20, and the body-side knitted fabric portion 30 are continuously and integrally knitted. A base yarn 51 and an elastic yarn 52 are continuously supplied to integrally knit the leg-side knitted fabric portions 10R and 10L, the crotch-side knitted fabric portion 20, and the body-side knitted fabric portion 30 as a single piece. In the leg-side knitted fabric portions 10R and 10L, the crotch-side knitted fabric portion 20, and the body-side knitted fabric portion 30, stitches (loops) arranged in a transverse direction constitute courses, and stitches (loops) arranged in a longitudinal direction constitute wales. That is, a course is a row of stitches extending along a yarn feeding direction of the base yarn 51, and a wale is a column of stitches extending in a direction perpendicular to the course. The number of rows of stitches (loops) counted in a longitudinal direction of the knitted fabric (the Y-axis direction of an X-Y coordinate system) corresponds to a course count or the number of courses, and the number of columns of stitches (loops) counted in a transverse direction of the knitted fabric (the X-axis direction of the X-Y coordinate system) corresponds to a wale (gauge) count or the number of wales.

In the pants-type compression garment 1 according to the present embodiment, the leg-side knitted fabric portions 10R and 10L and the body-side knitted fabric portion 30 are rib-knitted as textured knitting (also referred to as rib knitting or rib stitch) such that both front and rear surfaces are formed in a rib-like configuration. On the front and rear surfaces (that is, inner and outer surfaces), raised portions 31 and 32 and recessed portions 41 and 42 extending continuously in a wale direction (that is, a longitudinal direction) are alternately formed in a circumferential direction. That is, the leg-side knitted fabric portions 10R and 10L and the body-side knitted fabric portion 30 according to the present embodiment have alternating raised and recessed portions along a course direction (that is, a transverse direction or a widthwise direction) by rib knitting of the base yarn 51. Further, the raised portions 31 and 32 and the recessed portions 41 and 42 extend continuously in the wale direction.

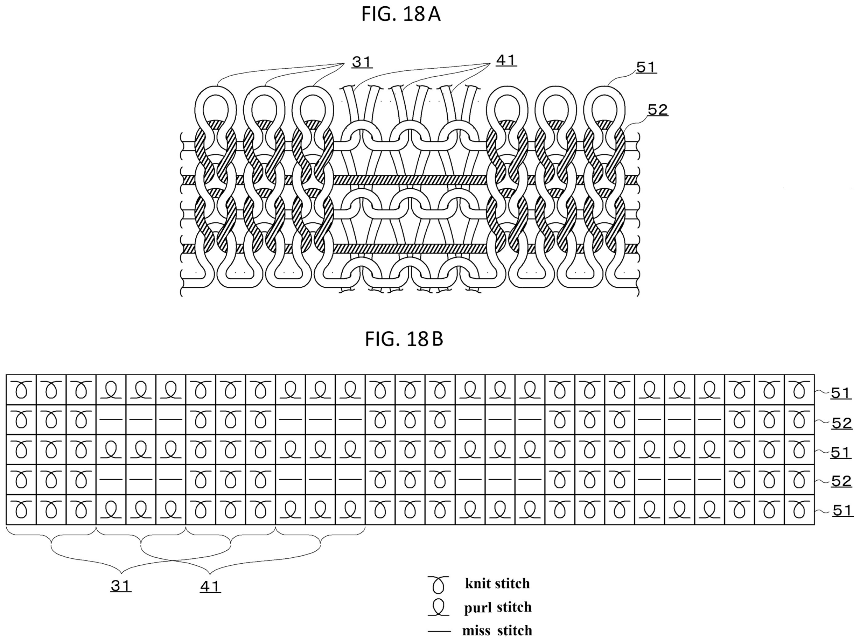

The rib knitting is a knitting method in which knit stitches and purl stitches are alternately knitted at a predetermined cycle in the course direction (the transverse direction) (see Fig. 18). Alternating knit stitches and purl stitches in the course direction aligned with the yarn-feeding direction forms the raised portions 31 and 32 and recessed portions 41 and 42 on the front and rear surfaces of the knitted fabric. In the raised portions 31 and 32 and the recessed portions 41 and 42 formed on the front and rear surfaces (the inner and outer surfaces) by rib knitting of the base yarn 51, stitches appearing as raised portions on one surface of the knitted fabric appear as recessed portions on the opposite surface, whereas stitches appearing as recessed portions on one surface appear as raised portions on the opposite surface. In the leg-side knitted fabric portions 10R and 10L and the body-side knitted fabric portion 30 according to the present embodiment, each of the knit stitches and the purl stitches are intermeshed continuously in the wale direction (the longitudinal direction), which is perpendicular to the yarn-feeding direction. Accordingly, each of the raised portions 31 and 32 and the recessed portions 41 and 42 extend continuously in the wale direction on the front and rear surfaces of the knitted fabric.

Thus, the leg-side knitted fabric portions 10R and 10L and the body-side knitted fabric portion 30 according to the present embodiment have the raised portions 31 and 32 and the recessed portions 41 and 42 that are alternately formed in the circumferential direction and continuously extend in parallel with each other in the wale direction (the longitudinal direction). In the leg-side knitted fabric portions 10R and 10L and the body-side knitted fabric portion 30 according to the present embodiment, the raised portions 31 and 32 and the recessed portions 41 and 42 on the front and rear surfaces (the inner and outer surfaces) are formed such that the knit stitches and the purl stitches are alternately formed in the course direction (the transverse direction) and each continuously intermeshed in the wale direction (the longitudinal direction). As a result, the raised portions 31 and 32 and the recessed portions 41 and 42 of the leg-side knitted fabric portions 10R and 10L and the body-side knitted fabric portion 30 are alternately formed over the entire circumference and each extend continuously in the wale direction.

In the pants-type compression garment 1 according to the present embodiment, the raised portions 31 and 32 and the recessed portions 41 and 42 each extend continuously in the wale direction from the lower ends (the lower opening portions 11R and 11L) of the leg-side knitted fabric portions 10R and 10L and from the lower portion of the body-side knitted fabric portion 30 up to the upper end (the upper opening portion 31)of the body-side knitted fabric portion 30. In the present embodiment, by varying the number of wales, specifically, by decreasing the number of stitches, some of the raised portions 31 and 32 and some of the recessed portions 41 and 42 in the body-side knitted fabric portion 30 terminate partway toward the upper end of the body-side knitted fabric portion 30. The majority of the raised portions 31 and 32 and the recessed portions 41 and 42 formed by rib knitting extend continuously from the lower opening portions 11R and 11L toward the upper opening portion 31 along a direction substantially perpendicular to the knitting width, and also extend continuously from the lower portion of the body-side knitted fabric portion 30 on the crotch-side knitted fabric portion 20 toward the upper opening portion 31.By varying the number of stitches, some of the raised portions 31 and 32 and some of the recessed portions 41 and 42 terminate partway toward the upper opening portion 31 at both left and right sides of the front and back of the knitted fabric.

Thus, in the pants-type compression garment 1 according to the present embodiment, the leg-side knitted fabric portions 10R and 10L and the body-side knitted fabric portion 30 are formed by rib knitting as textured knitting of the base yarn 51 such that the raised portions 31 and 32 and the recessed portions 41 and 42 are alternately formed in the course direction (the transverse direction) on the front and rear surfaces thereof. That is, the leg-side knitted fabric portions 10R and 10L and the body-side knitted fabric portion 30 are formed in a rib configuration such that the raised portions 31 and 32 and the recessed portions 41 and 42 are alternately arranged in the circumferential direction. Further, the raised portions 31 and 32 and the recessed portions 41 and 42 alternately formed in the course direction each extend continuously and in parallel along the wale direction (the longitudinal direction).

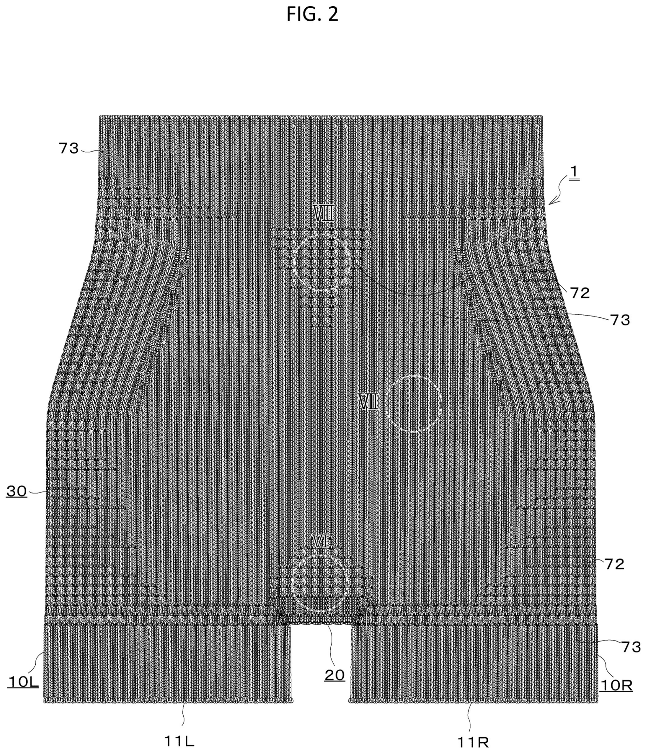

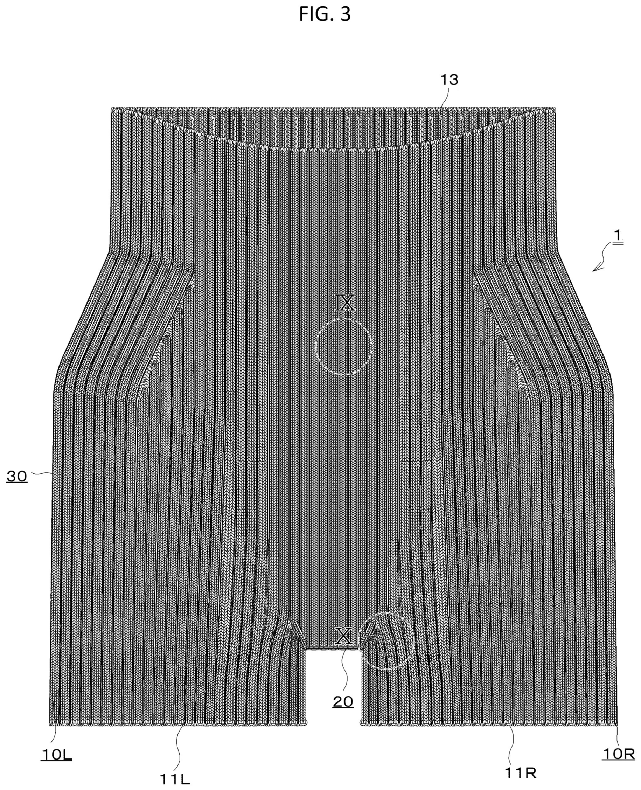

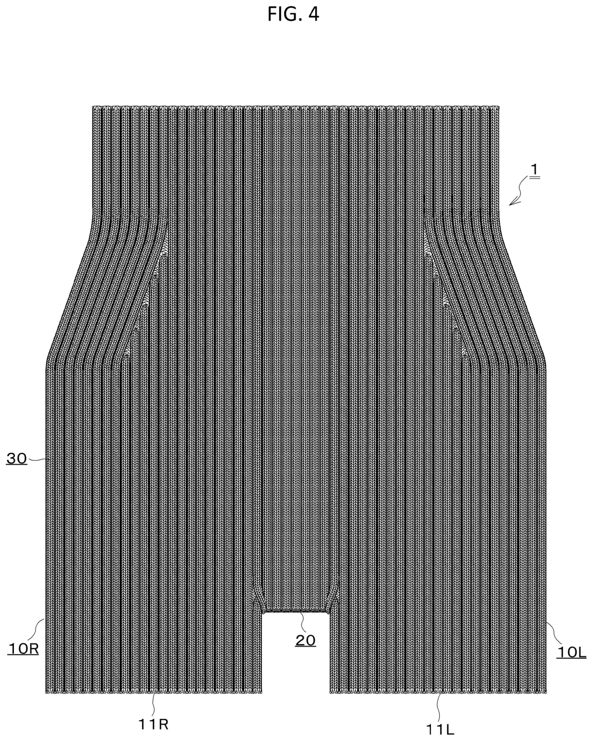

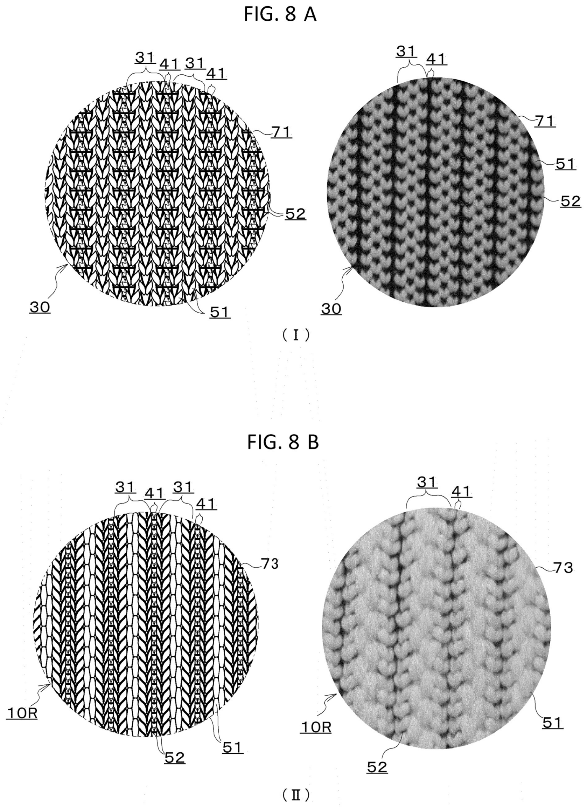

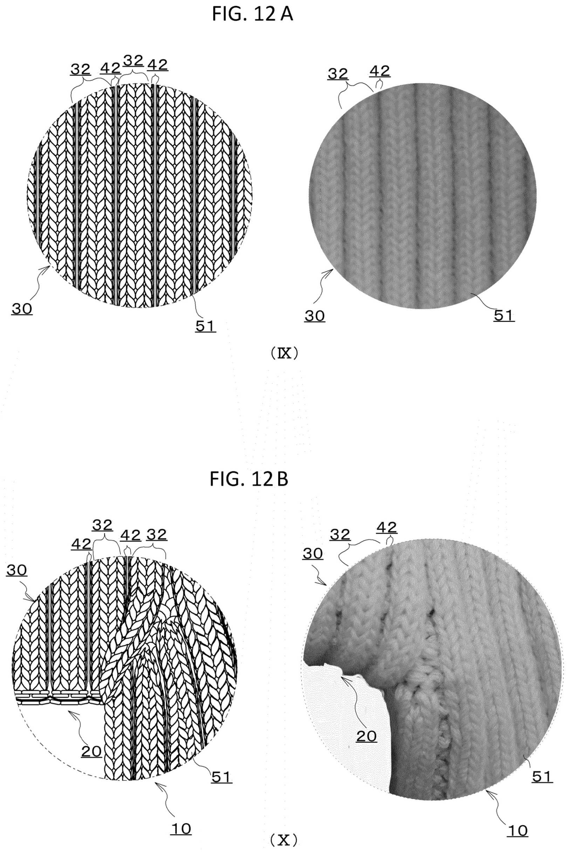

FIGS. 1 and 2 illustrate the outer surface of the pants-type compression garment 1, and FIGS. 3 and 4 illustrate the inner surface thereof. the stitches of the base yarn 51 that appear as raised portions on the outer surface of the pants-type compression garment 1 shown in FIGS. 1 and 2 and appear as recessed portions on the inner surface shown in FIGS. 3 are referred to as outer raised portions 31 on the outer surface and inner recessed portions 42 on the inner surface (see FIGS. 8 to 11). Conversely, the stitches of the base yarn 51 that appear as recessed portions on the outer surface shown in FIGS. 1 and 2 and appear as raised portions on the inner surface shown in FIGS. 3 and 4 are referred to as outer recessed portions 41 on the outer surface and inner raised portions 32 on the inner surface (see FIG. 12).

On the outer surface of the leg-side knitted fabric portions 10R and 10L and the body-side knitted fabric portion 30 of the pants-type compression garment 1, the outer raised portions 31 and the outer recessed portions 41 are alternately formed over the entire circumference in the course direction (the transverse direction), and each extend continuously in a wale direction (the longitudinal direction). On the opposite inner surface, the inner raised portions 32 and inner recessed portions 42 are alternately formed over the entire circumference in the course direction, and each extend continuously in the wale direction. Thus, in the pants-type compression garment 1 according to the present embodiment, the leg-side knitted fabric portions 10R and 10L and the body-side knitted fabric portion 30 are rib knitted using the base yarn 51, such that both the outer surface and the inner surface have a rib configuration in which the raised portions and the recessed portions are alternately and repeatedly formed over the entire circumference and each extend continuously in the wale direction.

In the leg-side knitted fabric portions 10R and 10L and the body-side knitted fabric portion 30 of the pants-type compression garment 1 according to the present embodiment, the raised portions 31 and 32 and the recessed portions 41 and 42 are alternately arranged in the transverse direction on both the outer periphery and the inner periphery (that is, the inner and outer surfaces). That is, the recessed portions (grooves) 41 and 42 are present between adjacent raised portions 31 and between adjacent raised portions 32. When the garment 1 is worn with the inner surface of the knitted fabric facing the human body, the inner raised portions 32 come into close contact with the body and apply pressure thereto. In particular, since the recessed portions 42 are present between adjacent raised portions 32, the recessed portions 42 can deform so as to conform to body shape and movement. That is, the spacing between the raised portions 32 can vary in accordance with the body shape and movement. This configuration allows cutting-in due to wrinkles to be less likely to occur even at bent or curved regions on the genital and groin regions of the human body, and allows obstruction of lymph flow due to wrinkles cutting into the skin to be less likely to occur. Further, since the inner raised portions 32 remain in close contact with the body and continuously apply pressure, the garment 1 can readily applied pressure to the lower abdominal, groin, and genital regions. In addition, since the raised portions 31 and 32 and the recessed portions 41 and 42 extending in the wale direction are alternately arranged in the circumferential direction, differences in pressure are generated between the inner raised portions 32 that contact the body and the inner recessed portions 42 that are less likely to contact the body. As a result, the inner raised portions 32 locally press the skin, thereby producing a massage effect and further improving lymph flow. In particular, since the raised portions 31 and 32 and the recessed portions 41 and 42 extend continuously in the wale direction, they are aligned along the direction of the lymph flow from the genital and groin regions toward an upper direction (specifically, toward a clavicular region) of the human body. Accordingly, the garment 1 does not obstruct lymph flow. Rather, the garment 1, when worn, is likely to generate a tensile force in the longitudinal direction from the genital and groin regions toward an upper direction (specifically, toward the clavicular region), thereby further promoting lymph flow.

In the pants-type compression garment 1 according to the present embodiment, the leg-side knitted fabric portions 10R and 10L, the crotch-side knitted fabric portion 20, and the body-side knitted fabric portion 30 are formed by interknitting the base yarn 51 and the elastic yarn 52. That is, in the present embodiment, the leg-side knitted fabric portions 10R and 10L, the crotch-side knitted fabric portion 20, and the body-side knitted fabric portion 30 are knitted using the base yarn 51 and the elastic yarn 52 having higher elasticity than the base yarn 51.

As the base yarn (also referred to as a ground yarn) 51 forming the base knitted structure, various types of yarns may be used, including, for example, cotton yarns, nylon yarns, polyester yarns, polyurethane yarns, rayon yarns, silk-acrylic yarns, silk yarns, linen yarns, cotton-acrylic blended yarns, linen-acrylic blended yarns, wool yarns, wool-blended yarns such as wool-acrylic blended yarns, moisture-absorbing yarns including block-copolymerizing hydrophobic synthetic fibers with hydrophilic groups or having porous surfaces thereon, and ceramic yarns capable of emitting far-infrared radiation. The base yarn 51 may be non-elastic or elastic. Various types of yarn materials may be used, and two or more types may be combined and bundled together. In particular, the use of a cotton yarn as the base yarn 51 allows the garment 1 to have excellent moisture absorbency and air permeability, high cushioning properties, and a soft texture and comfort against the skin, thereby making it less likely to cause skin irritation. For example, a cotton yarn produced by winding cotton around a core yarn (single covering yarn or double covering yarn) is preferably used. As raw material for the yarn, chemical materials, metals, plants may be used, and twisted yarn formed from combinations of two or more thereof may be used. Typically, spun yarn is used as the base yarn 51; however, filament yarns, monofilament yarns, bulky yarns, hollow yarns, covered yarns, core yarns, composite yarns, flat yarns, non-circular cross-section yarns, split yarns, lacquer-coated yarns, and the like may also be used. The base yarn 51 is not limited to a single yarn, and a twisted yarn formed by twisting two or more single yarns together may be used. Further, a plurality of single yarn and/or twisted yarn may be combined and used as one base yarn 51.

The number of single yarns constituting the base yarn 51 and the thickness of the base yarn 51 are not particularly limited; however, preferably, a yarn having a fineness of 1,000 to 3,000 denier are used, more preferably 1,200 to 3,000 denier, and still more preferably 1,500 to 3,000 denier. The use of the base yarn 51 having a denier within these ranges allows the knitted fabric to have a thickness, as measured with a micrometer, of preferably 2.0 mm to 10.0 mm, more preferably 2.0 mm to 8.0 mm, and still more preferably 2.5 mm to 5.0 mm, thereby making the knitted fabric thick and stiff. As a result, the garment 1 exhibits increased stiffness, thereby enhancing a muscle pump effect and a massage effect caused by body movements. Further, the knitted fabric is less likely to wrinkle during wear. That is, the garment 1 is less likely to wrinkle at portions covering even delicate regions such as the genital and groin regions with bending or curved portions, thereby preventing inhibition of lymph flow caused by localized wrinkle and thus improving wearing comfort. The above thickness refers to the thickness of the knitted fabric formed of the base yarn 51.

The elastic yarn 52 has higher elasticity than the base yarn 51, and examples thereof include yarns made of polyurethane (including spandex) and rubber. Yarns may also be used in which polyamide or cotton is wound around an elastic core such as latex or elastane, or so-called covered yarns or covering yarns in which a highly elastic yarn such as polyurethane is used as a core yarn and other yarns (for example, stretchable yarns such as woolly nylon yarns) are wound around (covered on) the core yarn. The elastic yarn 52 is not limited to a single yarn, and a twisted yarn formed by twisting two or more single yarns together may be used. Further, a plurality of single yarn and/or twisted yarn may be combined and used as one elastic yarn 52. Preferably, the elastic yarn 52 has a fineness of 100 to 2,000 denier, more preferably 200 to 1,800 denier, and still more preferably 300 to 1,600 denier. The use of the elastic yarn 52 with a denier within these ranges can provide a predetermined elasticity even though the yarn is relatively fine. Further, the use of the elastic yarn 52 finer than the base yarn 51 allows the elastic yarn 52 on the inner surface of the leg-side knitted fabric portions 10R and 10L, the crotch-side knitted fabric portion 20, and the body-side knitted fabric portion 30 to be less likely to come into contact with the skin, even in portions where the elastic yarn 52 is knitted together with the base yarn 51. Accordingly, friction between the elastic yarn 52 and the skin due to the material or stretch of the elastic yarn 52 is suppressed, thereby preventing the occurrence of allergies, eczema, and the like due to contact or rubbing of the elastic yarn 52.

In particular, in the leg-side knitted fabric portions 10R and 10L and the body-side knitted fabric portion 30 of the pants-type elastic garment 1 according to the present embodiment, the elastic yarn 52 interknitted with the base yarn 51 is knitted into the stitches forming the outer raised portions 31 and the inner recessed portions 42 of the rib-knitted base structure formed of the base yarn 51, that is, is intermeshed with the loops of the base yarn 51, whereas the elastic yarn 52 is missed with respect to the stitches forming the outer recessed portions 41 and the inner raised portions 32 to forms a float on the outer surface. Thus, the elastic yarn 52 is alternately knitted and missed in the course direction with respect to the rib-knitted base structure formed of the base yarn 51, thereby being interknitted therewith.

Thus, with respect to the stitches forming the outer recessed portions 41 and the inner raised portions 32 of the rib-knitted base structure having the raised and recessed portions formed of the base yarn 51, the elastic yarn 52 is missed and forms a float on the outer surface of the knitted fabric. As a result, the elastic yarn 52 does not appear (is not exposed) at the inner raised portions 32 on the inner surface of the knitted fabric. That is, on the outer surface of the knitted fabric of the leg-side knitted fabric portions 10R and 10L and the body-side knitted fabric portion 30, a float formed by a miss stitch of the elastic yarn 52 is present on stitches forming the outer recessed portions 41 of the rib-knitted base structure formed of the base yarn 51, and the elastic yarn 52 does not appear at the inner raised portions 32 on the inner surface. Accordingly, when the knitted fabric is worn with the inner surface facing the wearer's skin, even though the inner raised portions 32 in the leg-side knitted fabric portions 10R and 10L, the crotch-side knitted fabric portion 20, and the body-side knitted fabric portion 30 come into contact with the wearer's skin, the elastic yarn 52 is not exposed at the inner raised portions 32. Therefore, friction between the elastic yarn 52 and the skin due to the material or stretch of the elastic yarn 52 can be reduced, thereby making it less likely to cause skin irritation such as allergies or eczema due to contact or rubbing of the elastic yarn 52.

As described above, even though the elastic yarn 52 is knitted into the stitches of the outer raised portions 31 and inner recessed portions 42 of the rib-knitted base structure formed of the base yarn 51, the use of the elastic yarn 52 finer than the base yarn 51 reduces contact between the elastic yarn 52 and the skin at the inner recessed portions 42 on the inner surface of the knitted fabric. Similarly, in the crotch-side knitted fabric portion 20, even though the elastic yarn 52 is knitted into the stitches of the plain-knitted (jersey-knitted) base structure formed of the base yarn 51, the use of the elastic yarn 52 finer than the base yarn 51 reduces contact between the elastic yarn 52 and the skin on the inner surface of the knitted fabric.

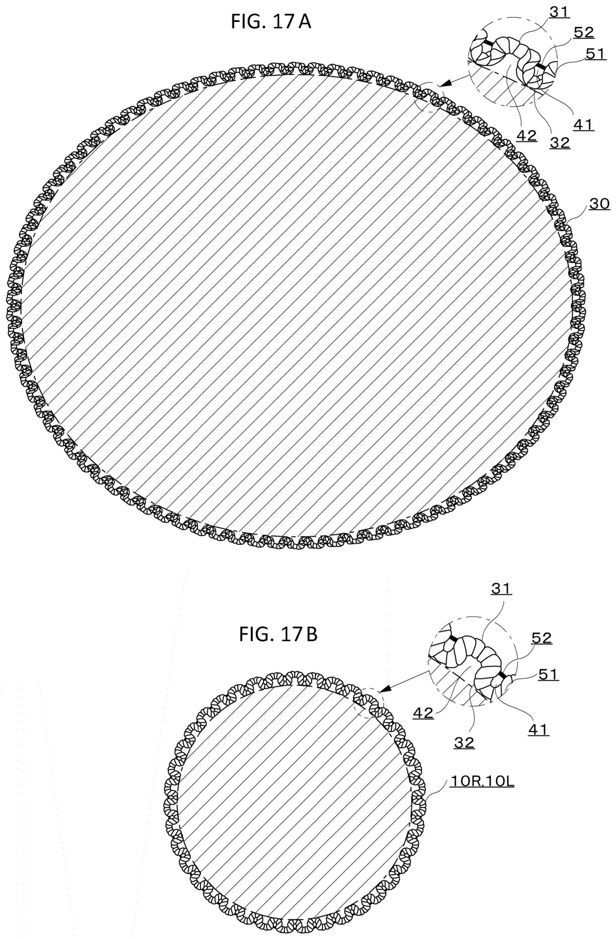

Thus, the elastic yarn 52 is knitted into the stitches forming the outer raised portions 31 and the inner recessed portions 42 of the rib-knitted base structure formed of the base yarn 51, whereas the elastic yarn 52 performs a miss stitch with respect to stitches forming the outer recessed portions 41 and the inner raised portions 32 of the rib-knitted base structure formed of the base yarn 51. As a result, the knitted fabric has a wave-like shape in the circumferential direction (see FIG. 17). The tension of the elastic yarn 52 increases the raised volume (height) of the inner raised portions 32, thereby allowing the inner raised portions 32 to more readily come into close contact with the skin.

In the pants-type compression garment 1 according to the present embodiment, the elastic yarn 52 is knitted into the stitches forming the outer raised portions 31 and the inner recessed portions 42 of the rib-knitted base structure with the recessed and raised portions formed of the base yarn 51. That is, the loops of the elastic yarn 52 are intermeshed with the loops of the base yarn 51 to form stitches of the elastic yarn 52. Accordingly, the outer raised portions 31 and the inner recessed portions 42 are formed of both the base yarn 51 and the elastic yarn 52. Meanwhile, the elastic yarn 52 is missed with respect to the stitches forming the outer recessed portions 41 and the inner raised portions 32. Accordingly, the outer recessed portions 41 and the inner raised portions 32 are formed solely of the base yarn 51, and the elastic yarn 52 forms a float on the outer recessed portion 41.

The elastic yarn 52 is knitted into the stitches forming the outer raised portions 31 and the inner recessed portions 42 of the rib-knitted base structure with the recessed and raised portions formed of the base yarn 51, whereas the elastic yarn 52 performs a miss stitch with respect to the stitches forming the outer recessed portions 41 and the inner raised portions 32. Accordingly, in the outer raised portions 31 and the inner recessed portions 42, the stitches of the base yarn 51 and the stitches of the elastic yarn 52 are alternately formed in the wale direction. As a result, these portions exhibit reduced stretchability in the course direction (the transverse direction) and in the in-plane direction, and increased stiffness, as compared with the outer recessed portions 41 and the inner raised portions 32. Meanwhile, in the outer recessed portions 41 and the inner raised portions 32 formed between adjacent outer raised portions 31 and between adjacent inner recessed portions 42, the elastic yarn 52 performs a miss stitch. As a result, these portions are more readily stretchable in the course direction. Further, when worn, circumferential tension of the elastic yarn 52 generates inward compressive pressure toward the center of the circumference. In addition, the inner raised portions 32 are not stiffer than the outer raised portions 31, thereby providing cushioning properties and improving comfort against the skin.

Thus, the portions where the elastic yarn 52 is knitted into the stitches forming the outer raised portions 31 and the inner recessed portions 42 of the rib-knitted base structure with the recessed and raised portions formed of the base yarn 51 exhibit reduced stretchability in the course direction (the transverse direction) and in the in-plane direction, as compared with the portions where the elastic yarn 52 performs a miss stitch and forms a float on the stitches forming the outer recessed portions 41 and the inner raised portions 32. In the pants-type compression garment 1 according to the present embodiment, the portions where the elastic yarn 52 is knitted into the loops of the base yarn 51 exhibit lower stretchability in the course direction and in the in-plane direction than the portions where the elastic yarn 52 is missed. Meanwhile, the portions where the elastic yarn 52 is missed with respect to the loops of the base yarn 51 exhibit increased stretchability in the course direction and in the in-plane direction. Further, in such portions, while allowing increased stretchability, the elastic yarn 52 is not exposed on the inner surface, thereby improving comfort against the skin. Therefore, by virtue of the outer recessed portions 41 and the inner raised portions 32 in which the elastic yarn 52 is missed with respect to the base yarn 51, the knitted fabric is moderately stretchable in the course direction, thereby facilitating wearing of the garment 1. In addition, spacing between adjacent raised portions 31 and between adjacent raised portions 32 is allowed to vary, and relative movement between adjacent raised portions 31 and between adjacent raised portions 32 is allowed. That is, the inner raised portions 32 are capable of shifting, and the inner recessed portions 42 between adjacent inner raised portions 32 are capable of deforming in response to body movement, thereby accommodating shape changes associated with body movement. As a result, even when the body moves or at bent or curved portions of the body, such as the genital and groin regions and surrounding regions thereof, the inner raised portions 32 can be kept in close contact with the body and can readily apply appropriate pressure thereto, thereby further promoting lymph flow. Meanwhile, by virtue of the outer raised portions 31 and the inner recessed portions 42 in which the elastic yarn 52 is knitted into the base yarn 51, stretchability in the course direction and in the in-plane direction of the knitted fabric is moderately suppressed. This makes it less likely to cause a feeling of tightness, while facilitating formation of a stiff wall of the knitted fabric, and readily producing a muscle pump effect and a massage effect, thereby further promoting lymph flow. Thus, the garment 1 is easy to wear and provides comfortable compression without causing an excessive feeling of tightness during wear, while facilitating formation of the stiff wall of the knitted fabric to readily produce a muscle pump effect and a massage effect, thereby allowing further promotion of lymph flow. Further, since the elastic yarn 52 is missed in the outer recessed portions 41 and the inner raised portions 32 and are not exposed on the inner surface of the knitted fabric, damage to the skin caused by the elastic yarn 52 is also less likely to occur.

In the pants-type compression garment 1 according to the present embodiment, as described later, by varying the knitting structure of the base yarn 51 in selected courses, for example, by locally transferring the stitches of the base yarn 51 in portions where the base yarn 51 forms the outer raised portions 31 and the inner recessed portions 42, elasticity (stretchability) and stiffness are locally varied, and the length of the body-side knitted fabric portion 30 is also adjusted. In addition, by forming openwork stitches through partial stitch transfer, breathability is improved.

Hereinafter, the knitting process of the pants-type compression garment 1 according to the present embodiment will be described in detail. The pants-type compression garment 1 according to the present embodiment is knitted using a seamless flat knitting machine having at least a pair of opposing front and rear needle beds. For example, by using a WHOLEGARMENT^{®} flat knitting machine manufactured by Shima Seiki Mfg., Ltd. (WHOLEGARMENT is a registered trademark of Shima Seiki Mfg., Ltd.), such as flat knitting machines of the WHOLEGARMENT MACH2 series (e.g., X, XS, VS, SWG), the leg-side knitted fabric portions 10R and 10L, the crotch-side knitted fabric portion 20, and the body-side knitted fabric portion 30 are seamlessly and integrally knitted in a continuous manner. Hereinafter, an example will be described in which the pants-type compression garment 1 is seamlessly knitted using a WHOLEGARMENT^{®} flat knitting machine having a pair of opposing front and rear needle beds.

In the present embodiment, a WHOLEGARMENT^{®} flat knitting machine is used, which has at least a pair of front and rear needle beds and is configured such that the front and rear needle beds are capable of relative racking. Specifically, the WHOLEGARMENT^{®} flat knitting machine includes needle beds in which two knitting needles per gauge are slidably mounted and the front and rear needle beds are configured to be movable relative to each other by racking, and a carriage that travels over upper surfaces of the respective needle beds for advancing and retracting the knitting needles. The knitted fabric is formed using the base yarn 51 and the elastic yarn 52 supplied in the transverse direction by a yarn feeder. That is, by using a WHOLEGARMENT^{®} flat knitting machine with at least a pair of front and rear needle beds, the base yarn 51 is supplied in a circular manner across the front and rear needle beds, whereby the front portion of the garment 1 is knitted on one needle beds and the back portion is knitted on the other needle bed. In this manner, the leg-side knitted fabric portions 10R and 10L having a small-diameter tubular shape, the crotch-side knitted fabric portion 20 disposed between and connecting the leg-side knitted fabric portions 10R and 10L, and the body-side knitted fabric portion 30 having a large-diameter tubular shape are continuously knitted as a single piece in a girdle-like form.
As the base yarn 51, for example, 20-count two-ply cotton yarn used in one to ten strands may be employed. As the elastic yarn 52, for example, two double-covered yarn may be employed, each including a polyurethane core yarn (260 denier) covered with polyester yarn (75 denier).

In knitting the pants-type compression garment 1 using a flat knitting machine having a pair of opposing front and rear needle beds, the base yarn 51 and the elastic yarn 52 are fed in a circumferential manner to knitting needles of the front and rear needle beds to form a tubular knitted fabric. Specifically, knitting is first started from the lower ends of the leg-side knitted fabric portions 10R and 10L. After the leg-side knitted fabric portions 10R and 10L, which are spaced apart from each other by a predetermined distance, are knitted to a predetermined length using the base yarn 51 and the elastic yarn 52, the crotch-side knitted fabric portion 20 is continuously knitted in the transverse direction (the course direction) between the leg-side knitted fabric portions 10R and 10L using the base yarn 51 and the elastic yarn 52, thereby connecting the leg-side knitted fabric portions 10R and 10L. Subsequently, the body-side knitted fabric portion 30 is continuously knitted in the wale direction from the leg-side knitted fabric portions 10R and 10L and the crotch-side knitted fabric portion 20 using the base yarn 51 and the elastic yarn 52. The knitting is completed at the upper end side of the body-side knitted fabric portion 30. Thus, the leg-side knitted fabric portions 10R and 10L each having a small-diameter tubular shape, the crotch-side knitted fabric portion 20 connecting the leg-side knitted fabric portions 10R and 10L, and the body-side knitted fabric portion 30 having a large-diameter tubular shape are integrally knitted. The yarn feeding position at which knitting is started for each of the leg-side knitted fabric portions 10R and 10L is, for example, a side corresponding to an inner thigh of the leg-side knitted fabric portions 10R and 10L. The upper opening side (upper opening 31) of the body-side knitted fabric portion 30, that is, the knitting end side, is finished by a bind-off knitting process (bind-off treatment) to prevent unraveling.

The pants-type compression garment 1 according to the present embodiment, in which the leg-side knitted fabric portions 10R and 10L each having a small-diameter tubular shape, the crotch-side knitted fabric portion 20 connecting the leg-side knitted fabric portions 10R and 10L, and the body-side knitted fabric portion 30 having a large-diameter tubular shape are integrally and seamlessly knitted using a seamless flat knitting machine, has no sewn portions and therefore provides excellent skin contact and wearing comfort. Further, since the garment 1 free of sewn portions, the knitted fabric is less likely to lift due to excessive tension of the knitted fabric during wear, thereby allowing appropriate pressure to be readily applied to the lower abdominal, groin, and genital regions, and enabling improvement of microcirculation and promotion of lymph flow. Moreover, a flat-knitted fabric produced by using such a seamless flat knitting machine can be formed using a thicker yarn and with the smaller number of needles per inch than a circular-knitted fabric produced by a circular knitting machine. This allows the stitch density of the knitted fabric to be reduced, thereby improving air permeability. In addition, the use of a thicker yarn provides a thick and stiff knitted fabric, thereby making it less likely to form wrinkles at bent or curved portions of the body, such as the genital and groin regions and its surrounding regions during wear, and preventing obstruction of lymph flow caused by wrinkles cutting into the skin. Furthermore, such a thick and stiff knitted fabric exhibits reduced stretchability and increased stiffness, thereby further promoting lymph flow through a muscle pump effect and a massage effect.

In the pants-type compression garment 1 knitted using a seamless flat knitting machine, the front portion and the back portion have the same number of wales. This garment 1 includes the leg-side knitted fabric portions 10R and 10L each having a small-diameter tubular shape, the crotch-side knitted fabric portion 20 connecting the leg-side knitted fabric portions 10R and 10L, and the body-side knitted fabric portion 30 having a large-diameter tubular shape and continuously formed from the leg-side knitted fabric portions 10R and 10L and the crotch-side knitted fabric portion 20 in the wale direction. The front portion of the garment 1 has a left-right symmetrical shape about a center line in the width direction and the back portion also has a left-right symmetrical shape about a center line in the width direction. In the garment 1 knitted using the seamless flat knitting machine, the front portion and the back portion are knitted in an overlapped state. In the crotch-side knitted fabric portion 20 formed between the leg-side knitted fabric portions 10R and 10L, the front portion (corresponding to a front body panel) and the back portion (corresponding to a back body panel) are integrally and seamlessly formed into a closed knitted structure. In such seamless flat knitting, linear stitch lines formed at locations where the yarn feeding direction is reversed (at left and right end portions of the front and rear needle beds) correspond to left and right sides where the front portion is continuous with the back portion without seams.

An example of knitting using a seamless flat knitting machine will be described below. First, at the start of knitting the leg-side knitted fabric portions 10R and 10L, when a yarn feeder carried by a carriage moves from left to right, the base yarn 51 is supplied in a zigzag manner across knitting needles of front and rear needle beds while the front and rear needle beds are in a racked position, thereby forming stitches. Thereafter, stitches formed on the knitting needles of the rear needle bed are transferred to opposing knitting needles of the front needle bed, whereby one of the front portion and the back portion of the knitted fabric is set into a state capable of rib knitting. Next, when the carriage is reversed and the yarn feeder carried thereby moves from right to left, the base yarn 51 is supplied in a zigzag manner across the knitting needles of the rear needle bed that were previously unselected and the knitting needles of the front needle bed, thereby forming stitches. Thereafter, stitches formed on the knitting needles of the front needle bed are transferred to opposing knitting needles of the rear needle bed, whereby the other of the front and back of knitted fabric is set into a state capable of rib knitting.

To prevent the rib knitting at the cast-on portion from unraveling, the stitches transferred to the front needle bed are transferred back to the knitting needles of the rear needle bed. Thereafter, a circular knitting operation is performed by supplying the yarn to the knitting needles located on corresponding sides of the front and rear needle beds. Further, the stitches formed on the knitting needles of the rear needle bed are transferred to the knitting needles of the front needle bed. Next, similarly, a circular knitting operation is performed by supplying the base yarn 51 to knitting needles located on corresponding sides of the front and rear needle beds. Through these operations, a stitch-locking operation for preventing unraveling is performed.

After the stitch-locking operation has been completed, the base yarn 51 is supplied in a zigzag manner to the knitting needles of the front needle bed and the rear needle bed, with the front and rear needle beds being in a racked position, to form stitches of the base yarn 51. Thereafter, the stitches formed on the knitting needles of the rear needle bed are transferred to the opposing knitting needles of the front needle bed. Subsequently, the base yarn 51 is supplied to the knitting needles of the rear needle bed and the front needle bed. Next, the stitches formed on the knitting needles of the front needle bed are transferred to the opposing knitting needles of the rear needle bed. By repeating these knitting courses, a rib-knitted structure of the base yarn 51 is formed.

In rib knitting, the stitches appearing as the knit stitches of the outer raised portions 31 on the outer surface of the knitted fabric are held on knitting needles of the front needle bed, and the stitches appearing as the purl stitches of the outer recessed portions 41 are transferred to knitting needles of the opposing rear needle bed. Thereafter, the base yarn 51 is supplied in a zigzag manner across the knitting needles of the front and rear needle beds to knit one of the front and the back of the knitted fabric in a transverse direction (the course direction). After the transverse knitting, the stitches that have been transferred to the rear needle bed for forming purl stitches are transferred back to the original knitting needles of the front needle bed, thereby holding all stitches of one knitted fabric on the front needle bed. Subsequently, when knitting the other of the front and back of the knitted fabric, the stitches appearing as the knit stitches of the outer raised portions 31 on the outer surface of the knitted fabric are held on knitting needles of the rear needle bed, and the stitches appearing as the purl stitches of the outer recessed portions 41 are transferred to the opposing front needle bed. Thereafter, the base yarn 51 is supplied in a zigzag manner across the knitting needles of the front and rear needle beds to knit the other knitted fabric. After this knitting, the stitches transferred to the front needle bed for forming the purl stitches are transferred back to the original knitting needles of the rear needle bed, thereby holding all stitches of the other knitted fabric on the rear needle bed. Through circumferential feeding of the base yarn 51, the front and back of the knitted fabric is formed into a tubular shape. Thus, a rib-knitted structure formed of the base yarn 51, in which the knit stitches and the purl stitches are alternately arranged, is formed.

The knitting pattern of the leg-side knitted fabric portions 10R and 10L and the body-side knitted fabric portion 30 according to the present embodiment will be described below.
The base structure of a 3×3 rib knit, in which three knit stitches and three purl stitches are alternately arranged, is formed using the base yarn 51. The elastic yarn 52 is interknitted with the 3×3 rib knit base structure (alternating three knit stitches and three purl stitches) of the base yarn 51 in alternate courses (alternate rows). That is, the loops of the base yarn 51 and the loops of the elastic yarn 52 are alternately arranged in the wale direction (see FIG. 18). Specifically, as described above, the elastic yarn 52 is knitted into the stitches of the base yarn 51 that form the outer raised portions 31 and inner recessed portions 42, which appear as convex portions on the outer surface of the knitted fabric and as concave portions on the inner surface. That is, the elastic yarn 52 is knitted into the stitches that are the knit stitches on the outer surface and the purl stitches on the inner surface. Meanwhile, the elastic yarn 52 is missed with respect to the stitches of the base yarn 51 that form the outer recessed portions 41 and inner raised portions 32, which appear as concave portions on the outer surface and as convex portions on the inner surface. That is, the elastic yarn 52 is missed with respect to the stitches that are the purl stitches on the outer surface and the knit stitches on the inner surface. In this manner, the elastic yarn 52 is interknitted with the 3×3 rib-knit base structure formed of the base yarn 51 by alternately repeating knit stitches and miss stitches.

That is, following a rib-knitting step in which a 3×3 rib knit is formed by feeding the base yarn 51, an elastic-yarn knitting step is performed by feeding the elastic yarn 52. In the elastic-yarn knitting step, the elastic yarn 52 is knit-stitched on knitting needles having three knit stitches forming the outer raised portions 31 and the inner recessed portions 42 of the rib-knit base structure formed of the base yarn 51, while the elastic yarn 52 is miss-stitched on knitting needles having three purl stitches forming the outer recessed portions 41 and the inner raised portions 32. Thereafter, by feeding the base yarn 51 again, the base yarn 51 is knit-stitched on the knitting needles on which the elastic yarn 52 was knit-stitched in the preceding elastic-yarn knitting step, and is also knit-stitched on the knitting needles where the elastic yarn 52 was miss-stitched, thereby performing a 3×3 rib-knitting step. Subsequently, the elastic-yarn knitting step is performed again. Thus, the base yarn 51 and the elastic yarn 52 are alternately knitted in each course.

As described above, in the knitted fabric of the leg-side knitted fabric portions 10R and 10L and the body-side knitted fabric portion 30, floating yarn formed by miss stitches of the elastic yarn 52 are overlaid on the stitches of the base yarn 51 forming the outer recessed portions 41 on the outer surface. Accordingly, the elastic yarn 52 is not exposed at the inner raised portions 32 of the inner surface. In addition, since the elastic yarn 52 is finer than the base yarn 51, the elastic yarn 52 is hardly exposed at the inner recessed portions 42 of the inner surface. Thus, the elastic yarn 52 is not exposed at the inner raised portions 32 that come into close contact with the wearer's body, and is hardly exposed at the inner recessed portions 42. As a result, when the knitted fabric is worn with the inner surface facing and contacting the human body, contact between the elastic yarn 52 and the wearer's skin is avoided, irritation to the skin caused by the material, stretchability, friction, or the like of the elastic yarn 52 is prevented, and even though the elastic yarn 52 is used, the feel against the skin is not impaired, thereby making it possible to prevent skin troubles such as allergies, eczema, and the like caused by the elastic yarn 52.

In the knitted fabric of the leg-side knitted fabric portions 10R and 10L and the body-side knitted fabric portion 30 according to the present embodiment, the 3×3 rib knit formed of the base yarn 51 is circumferentially knitted such that three knit stitches are continuously formed following three knit stitches of a preceding course, and three purl stitches are continuously formed following three purl stitches of the preceding course. As a result, the raised portions 31 and 32 and recessed portions 41 and 42 formed on the front and rear surfaces by the rib knitting extend continuously in the wale direction (the longitudinal direction). The elastic yarn 52 is knitted into the stitches forming the outer raised portions 31 and the inner recessed portions 42 by the base yarn 51. Accordingly, the outer raised portions 31 and the inner recessed portions 42 are formed of both the base yarn 51 and the elastic yarn 52.

Thus, in the knitted fabric of the leg-side knitted fabric portions 10R and 10L and the body-side knitted fabric portion 30 according to the present embodiment, rib knitting is employed to form a raised-and-recessed structure in which the raised portions 31 and 32 and the recessed portions 41 and 42 are alternately arranged in the circumferential direction and extend continuously in the wale direction. In this configuration, the elastic yarn 52 is knitted into the stitches of the base yarn 51 that form the outer raised portions 31 and the inner recessed portions 42, whereas the elastic yarn 52 is missed with respect to the stitches of the base yarn 51 that form the outer recessed portions 41 and the inner raised portions 32.

In the crotch-side knitted fabric portion 20 according to the present embodiment, at the start of knitting, when the yarn feeder carried by the carriage moves from left to right, the base yarn 51 is supplied in a zigzag manner across selected knitting needles of the front and rear needle beds in a racked position. Next, when the carriage is reversed and the yarn feeder carried thereby moves from right to left, the base yarn 51 is supplied in a zigzag manner across the knitting needles of the front and rear needle beds that have been left as empty needles, thereby forming plain knitting (also referred to as jersey knitting or stockinette knitting). In addition, the elastic yarn 52 is knitted into the base yarn 51. In the crotch-side knitted fabric portion 20 according to the present embodiment, the knitted fabric is formed only of knit stitches when viewed from the outer surface and only of purl stitches when viewed from the inner surface. Accordingly, the crotch-side knitted fabric portion 20 has a non-rib region in which alternating raised and recessed portions are not formed in the course direction, unlike rib knitting.

In the crotch-side knitted fabric portion 20, the elastic yarn 52 is knitted into the stitches of the base yarn 51 that are formed only of knit stitches on the outer surface of the knitted fabric and only of purl stitches on the inner surface. Thus, the crotch-side knitted fabric portion 20 is composed of the course sequences of the stitches of the base yarn 51, and the course sequences of the stitches of the elastic yarn 52, each of which is formed only of knit stitches on the outer surface and only of purl stitches on the inner surface. That is, the base yarn 51 and the elastic yarn 52 are interknitted with each other to form the knitted fabric that is composed of only knit stitches on the front surface and only purl stitches on the rear surface.

Thereafter, the stitches formed on the knitting needles of the rear needle bed are transferred to the opposing knitting needles of the front needle bed, whereby one of the front and the back of the knitted fabric of the body-side knitted fabric portion 30 is set into a state capable of rib knitting. Similarly, the stitches formed on the knitting needles of the front needle bed are transferred to the opposing knitting needles of the rear needle bed, whereby the other of the front and back of the knitted fabric of the body-side knitted fabric portion 30 is set into a state capable of rib knitting.

Hereinafter, the knitting pattern of the leg-side knitted fabric portions 10R and 10L, the crotch-side knitted fabric portion 20, and the body-side knitted fabric portion 30 according to the present embodiment will be described in more detail. In the knitting pattern of the leg-side knitted fabric portions 10R and 10L and the body-side knitted fabric portion 30 according to the present embodiment, the elastic yarn 52 is interknitted with the 3×3 rib-knit base structure formed of the base yarn 51. Specifically, the elastic yarn 52 is knitted into all three knit stitches forming the outer raised portions 31 that appear convex on the outer surface of the knitted fabric, and is missed with respect to all three purl stitches forming the outer recessed portions 41 that appear concave on the outer surface of the knitted fabric. By alternately repeating knit stitching and miss stitching in units of three stitches within each course, the elastic yarn 52 is interknitted with the 3×3 rib-knit base structure formed of the base yarn 51 (see FIG. 18).

Further, in selected portions, the middle stitch of three knit stitches forming the outer raised portion 31 appearing convex on the outer surface of the knitted fabric is transferred (overlapped) onto an adjacent left or right stitch. That is, the middle stitch of the three knit stitches forming the outer raised portion 31, the middle stitch of three knit stitches forming the outer raised portion 31 appearing convex on the outer surface is transferred to either an adjacent left stitch or an adjacent right stitch. In a subsequent stitch transfer operation, the middle stitch of the three knit stitches forming the outer raised portion 31 is transferred to the opposite side (right or left) relative to the previous stitch transfer. Such stitch transfers are appropriately repeated in the wale direction.

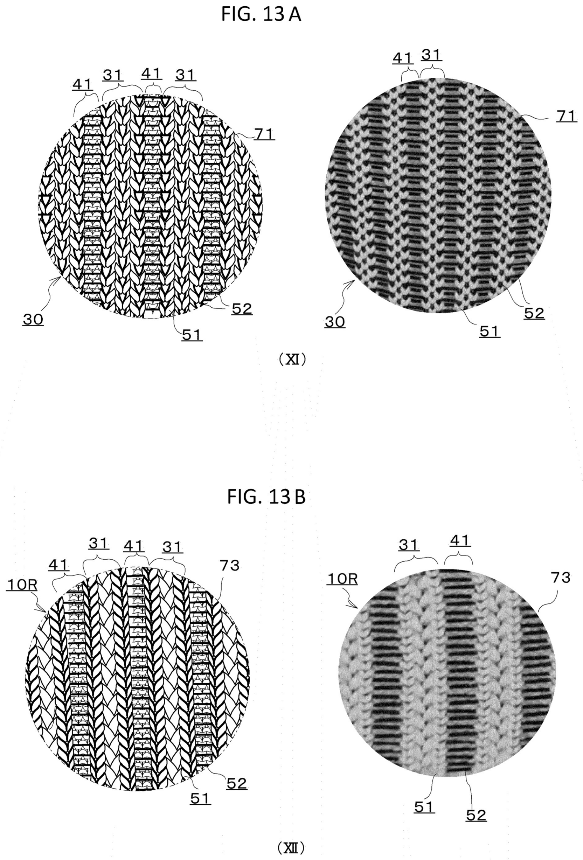

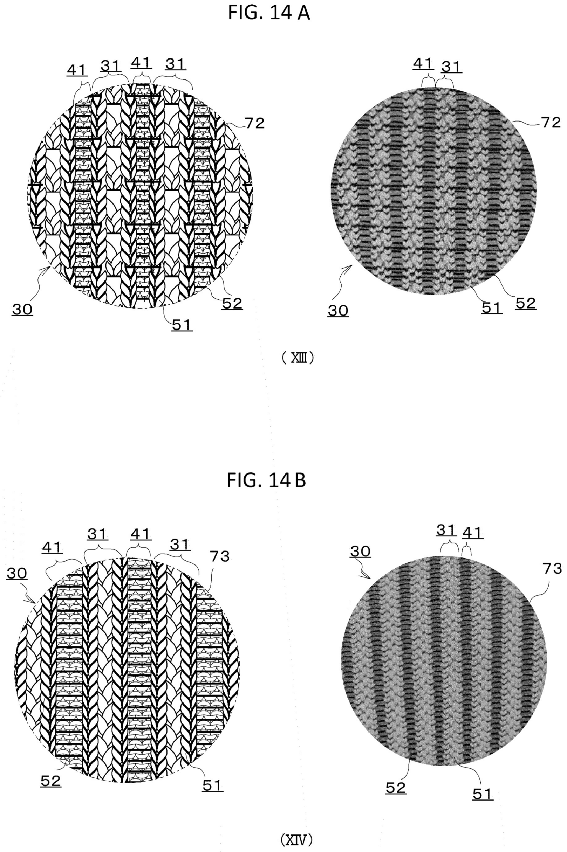

Thus, in the outer raised portions 31 appearing convex on the outer surface and the inner recessed portions 42 appearing concave on the inner surface, by transferring the middle stitch onto the adjacent left stitch or the adjacent right stitch in predetermined courses, a mesh-like knitting structure having open stitches is formed, thereby forming openwork stitches (see FIG. 13(b) and FIGS. 14(a) and 14(b)). As a result, when the knitted fabric is stretched, enlarged stitch gaps (openings) are formed in the outer raised portions 31 appearing convex on the outer surface and the inner recessed portions 42 appearing concave on the inner surface. This allows the knitted fabric to have improved air permeability. In another embodiment, tuck knitting may be employed. Similar effects can also be obtained by tuck knitting or the like.

In the present embodiment, stretchability of the knitted fabric is controlled by varying the stitch-transfer interval. Specifically, in the front portion of the body-side knitted fabric portion 30 that covers the lower abdominal, groin, and genital regions when worn, the middle stitch of the three knit stitches forming the outer raised portion 31 appearing convex on the outer surface and the inner recessed portion 42 appearing concave on the inner surface is not transferred to either the adjacent left stitch or the adjacent right stitch. As a result, the front of the body-side knitted fabric portion 30 has a relatively fine stitch density (see FIG. 13(a)) and a low-stretch region (also referred to as light-stretch region) 71 in which elongation of the elastic yarn 52 interknitted with the base yarn 51 is suppressed.

Thus, in the low-stretch region 71, the middle stitch of the three knit stitches forming the outer raised portion 31 appearing as convex on the outer surface and the inner recessed portions 42 appearing as concave on the inner surface is not transferred to either the adjacent left stitch or the adjacent right stitch, whereby the elongation of the elastic yarn 52 is suppressed. Meanwhile, in a region surrounding the low-stretch region 71, the middle stitch of the three knit stitches forming the outer raised portions 31 appearing as convex on the outer surface and the inner recessed portions 42 appearing as concave on the inner surface is transferred to either the adjacent left stitch or the adjacent right stitch. Further, the middle stitch of the three knit stitches forming the outer raised portions 31 is transferred to the opposite side (right or left) relative to the previous stitch transfer. Such stitch transfer is alternately repeated at predetermined course intervals. As a result, transferred stitches (coarse stitches) are formed at the middle of the three knit stitches forming the outer raised portions 31 and the inner recessed portions 42 at predetermined course intervals (see FIG. 14(a)). Accordingly, the region surrounding the low-stretch region 71 allows the elastic yarn 52 interknitted with the base yarn 51 to be more stretchable than in the low-stretch region 71. This region surrounding the low-stretch region 71 is an intermediate-stretch region (also referred to as moderate-stretch region) 72 that has higher stretchability than the low-stretch region 71 but lower stretchability than a high-stretch region 73 described later. In addition, the back portion of the body-side knitted fabric portion 30 also locally includes the intermediate-stretch region 72 at portions corresponding, when worn, to the regions near the genital and groin regions, left and right sides of the buttock region, and the recessed portions of the buttock region.

Further, in the portions of the body-side knitted fabric portion 30 corresponding, when worn, to the buttock region and the waist region, and in the leg-side knitted fabric portions 10R and 10L, the middle stitch of the three knit stitches forming the outer raised portion 31 appearing convex on the outer surface and the inner recessed portion 42 appearing concave on the inner surface is transferred to either the adjacent left stitch or the adjacent right stitch. In addition, the middle stitch of the three knit stitches forming the outer raised portion 31 and the inner recessed portion 42 is transferred to the opposite side (right or left) relative to the previous stitch transfer. Such stitch transfer is alternately repeated in each course. As a result, transferred stitches (coarse stitches) are formed at the middle of the three knit stitches forming the outer raised portion 31 and the inner recessed portion 42 in each course (see FIG. 13(b) and FIG. 14(b)). This region allows the elastic yarn 52 interknitted with the base yarn 51 to be more stretchable than in the intermediate-stretch region 72 and is a high-stretch region73 (also referred to as highly stretchable region) having higher stretchability than the intermediate-stretch region 72.

In the present embodiment, the lower central portion of the front of the body-side knitted fabric portion 30, which corresponds, when worn, to the region covering the lower abdominal, groin, and genital regions, is the low-stretch region 71. This low-stretch region 71 has no stitch transfer. That is, in this low-stretch region 71, stitch transfer of the middle stitch of the three knit stitches forming the outer raised portion 31 appearing convex on the outer surface and the inner recessed portion 42 appearing concave on the inner surface is not performed. Accordingly, this low-stretch region 71 has relatively fine stitches, whereby elongation of the elastic yarn 52 interknitted with the base yarn 51 is suppressed.

In the lower portions at the left and right end sides and the upper portions at the central part of the front of the body-side knitted fabric portion 30, which surround the low-stretch region 71, and in the lower and upper central portions and the left and right sides of the back of the body-side knitted fabric portion 30, transferred stitches (coarse stitches) are formed at predetermined course intervals by transferring the middle stitch of three knit stitches, which form the outer raised portion 31 appearing convex on the outer surface and the inner recessed portion 42 appearing concave on the inner surface, to either the adjacent left stitch or the adjacent right stitch. As a result, in these regions, the elastic yarn 52 interknitted with the base yarn 51 is relatively more stretchable than in the low-stretch region 71. Each of these regions is the intermediate-stretch region 72 having higher stretchability than the low-stretch region 71 but lower stretchability than the high-stretch region 73.

In the central portions and the upper end portions of the back of the leg-side knitted fabric portions 10R and 10L and the body-side knitted fabric portion 30, transferred stitches (coarse stitches) are formed at alternate courses by transferring the middle stitch of three knit stitches, which form the outer raised portion 31 appearing convex on the outer surface and the inner recessed portion 42 appearing concave on the inner surface, to either the adjacent left stitch or the adjacent right stitch. As a result, the elastic yarn 52 interknitted with the base yarn 51 is more stretchable than in the intermediate-stretch region 72. Each of these regions is the high-stretch region 73 having higher stretchability than the intermediate-stretch region 72.

As described above, the garment 1 according to the present embodiment has the low-stretch region 71 located in the lower central portion of the front of the body-side knitted fabric portion 30, which is configured to cover the lower abdominal, groin, and genital regions, when worn. In the low-stretch region 71, the three knit stitches forming the outer raised portion 31 appearing convex on the outer surface are not subjected to stitch transfer. Accordingly, the low-stretch region 71 is formed of relatively fine stitches of the base yarn 51 and the elastic yarn 52, thereby exhibiting suppressed stretchability of the elastic yarn 52 interknitted with the base yarn 51. Further, the garment 1 according to the present embodiment has the intermediate-stretch region 72 and the high-stretch region 73 located in portions of the front of the body-side knitted fabric portion 30 other than the region covering the lower abdominal, groin, and genital regions when worn. In the intermediate-stretch region 72 and the high-stretch region 73, the middle stitch of three knit stitches forming the outer raised portion 31 appearing convex on the outer surface of the rib knit structure is transferred (overlapped) onto the adjacent left stitch or the adjacent right stitch. Accordingly, the intermediate-stretch region 72 and the high-stretch region 73 have coarse stitches of the base yarn 51 and the elastic yarn 52. In these intermediate-stretch region 72 and the high-stretch region 73, the elastic yarn 52 interknitted with the base yarn 51 exhibits increased stretchability.

In the body-side knitted fabric portion 30, the regions other than the regions covering the lower abdominal, groin, and genital regions when worn, have coarse stitches formed by stitch transfer, thereby allowing the elastic yarn 52 to stretch readily. Meanwhile, the regions covering the lower abdominal, groin, and genital regions, when worn, has no transferred stitches, whereby stretch of the elastic yarn 52 is suppressed.

Thus, in the garment 1 according to the present embodiment, the crotch-side knitted fabric portion 20 has no transferred stitches and relatively fine stitches in the regions covering the lower abdominal, groin, and genital regions, when worn, in which the middle stitch of three knit stitches forming the outer raised portion 31 appearing convex on the outer surface and the inner recessed portion 42 appearing concave on the inner surface is not transferred to the adjacent left stitch or right stitch. This region covering the lower abdominal, groin, and genital regions, when worn, has suppressed stretchability of the elastic yarn 52 interknitted with the base yarn 51 and is the low-stretch region 71 exhibiting a lower elongation rate than other regions. Therefore, the region configured to cover the lower abdominal, groin, and genital regions has a lower elongation rate and higher stiffness than other regions, thereby enhancing a muscle pump effect and a massage effect and facilitating lymph flow from the genital and groin regions toward the lower abdominal region.

The body-side knitted fabric portion 30 has coarse stitches formed by stitch transfer in the regions other than the region configured to cover the lower abdominal, groin, and genital regions, in which the middle stitch of three knit stitches forming the outer raised portion 31 appearing convex on the outer surface is transferred to either the adjacent left stitch or the adjacent right stitch at predetermined course intervals or in each course. These regions are the intermediate-stretch region 72 and the high-stretch region 73, in which the elastic yarn 52 interknitted with the base yarn 51 is more readily stretchable than in the low-stretch region 71. Thus, in the region other than the region covering the lower abdominal, groin, and genital regions when worn, the elastic yarn 52 is readily stretchable. This allows the garment 1 to be easily donning and doffing and prevents a feeling of tightness during wearing, and prevents the garment 1 from slipping, loosening, and wrinkling. That is, the regions corresponding to the thigh region, the buttock region, the flank region, and the waist region of the human body are relatively stretchable, thereby reducing a feeling of tightness and a constrictive feeling even when the wearer is seated or in motion.
Therefore, the garment 1 according to the present embodiment achieves both promotion of lymph flow in the lower abdominal, groin, and genital regions and comfort during wearing.

The region that covers the lower abdominal, groin, and genital regions, when worn, in the body-side knitted fabric portion 30, is the low-stretch region 71, while the other regions in the body-side knitted fabric portion 30 are the intermediate-stretch region 72 and the high-stretch region 73 having higher stretchability than the low-stretch region 71. The stretchability of the intermediate-stretch region 72 and the high-stretch region 73 allows ease of donning and movement of the garment 1 and provides a comfortable fit without causing a feeling of tightness. Further, the low-stretch region 71 exhibits higher stiffness, thereby enhancing a muscle pump effect and a massage effect. Therefore, it is possible to promote lymph flow from the genital and groin regions toward an upper direction while maintaining a comfortable fit without causing a feeling of tightness. Thus, the garment 1 according to the present embodiment has varying stretchability and stiffness, thereby achieving both wearing comfort and promotion of lymph flow by a muscle pump effect and a massage effect.

Further, in the present embodiment, the intermediate-stretch region 72 having a higher elongation rate than the low-stretch region 71 surrounds the low-stretch region 71, and the high-stretch region 73 is adjacent to the intermediate-stretch region 72. This stepwise variation in stretchability reduces the load applied to the base yarn 51 due to differences in elongation rate, thereby making the garment 1 resistant to permanent deformation. Therefore, even after repeated washing, the garment 1 is less likely to deform or excessively stretch and can maintain high durability.

As shown in FIGS. 1 to 7, in the low-stretch region 71 of the front of the body-side knitted fabric portion 30, which is knitted in the wale direction from the crotch-side knitted fabric portion 20 and the leg-side knitted fabric portions 10R and 10L, the number of the outer raised portions 31 in which stitches are not transferred is gradually increased from the central portion in the width direction toward both left and right sides. As a result, the low-stretch region 71 gradually widens laterally from the width (crotch width) of the crotch-side knitted fabric portion 20 toward a longitudinally central portion above the crotch-side knitted fabric portion 20. Thereafter, as knitting proceeds in the wale direction, the number of the outer raised portions 31 in which stitches are not transferred is gradually decreased from both left and right sides toward the central portion in the width direction. As a result, the low-stretch region 71 gradually narrows laterally from the longitudinally central portion toward an upper side. That is, as shown in FIGS. 1 to 7, the low-stretch region 71 in the front of the body-side knitted fabric portion 30, which is a region covering the lower abdominal, groin, and genital regions when worn, has a shape that widens from the lower portion toward a longitudinally central portion in the body-side knitted fabric portion 30, and narrows from the longitudinally central portion toward the upper portion in the body-side knitted fabric portion 30. The low-stretch region 71 has a length of approximately three-quarters of the total length of the body-side knitted fabric portion 30 from the lower end, and has a width ranging from the width of the crotch-side knitted fabric portion 20 to approximately three-fifths of the width of the body-side knitted fabric portion 30.

The low-stretch region 71 covers the region extending from the genital and groin regions to the lower abdominal region of a wearer. For example, the crotch-side knitted fabric portion 20 preferably has a knitting width (crotch width) of 5 to 10 cm, and more preferably of 6 to 10 cm. Accordingly, the low-stretch region 71 has a minimum width of, for example, approximately 5 to 10 cm and a maximum width of, for example, approximately 22 to 27 cm, and has a length of, for example, approximately 10 to 25 cm extending upward from the lower end of the crotch-side knitted fabric portion 20. In the front of the body-side knitted fabric portion 30, the low-stretch region 71, which is a region covering the lower abdominal region (including the groin region), preferably has a longitudinal length corresponding to approximately 1/3 to 4/5, and more preferably approximately 1/3 to 3/4, of the total number of courses. In the front of the body-side knitted fabric portion 30, the low-stretch region 71 preferably has a width not less than a width corresponding to the number of wales (knitting width) of the crotch-side knitted fabric portion 20 and not greater than a width corresponding to the maximum number of wales (knitting width) of the front of the body-side knitted fabric portion 30, and more preferably not less than a width corresponding to the number of wales of the crotch-side knitted fabric portion 20 and not greater than approximately four-fifths of the maximum number of wales of the front of the body-side knitted fabric portion 30.

The garment 1 according to the present embodiment includes the low-stretch region 71 that covers the lower abdominal, groin, and genital regions when worn. The low-stretch region 71 has no transferred stitches and has dense knit stitches formed of the base yarn 51 forming the inner recessed portions 42 and the outer raised portions 31 and the elastic yarn 52 interknitted therewith. The intermediate-stretch region 72 formed around the low-stretch region 71 has transferred stitches and the greater number of coarser knit stitches than the low-stretch region 71. The high-stretch region 73 adjacent to the intermediate-stretch region 72 has a higher ratio of transferred stitches than the intermediate-stretch region 72 and the greater number of coarser knit stitches than the intermediate-stretch region 72. In the garment 1 according to the present embodiment, each of the front and the back of the leg-side knitted fabric portions 10R and 10L is the high-stretch region 73. The front of the body-side knitted fabric portion 30 has a relatively large proportion of the low-stretch region 71 and the intermediate-stretch region 72 in the longitudinal direction, whereas the back of the body-side knitted fabric portion 30 has a relatively large proportion of the high-stretch region 73 in the longitudinal direction.

Thus, the front of the body-side knitted fabric portion 30 has a relatively large proportion of the low-stretch region 71 and the intermediate-stretch region 72 in a longitudinal direction, whereas the back of the body-side knitted fabric portion 30 has a relatively large proportion of the high-stretch region 73 in the longitudinal direction. As a result, the front of the body-side knitted fabric portion 30 has a shorter length than the back thereof. That is, in the body-side knitted fabric portion 30 according to the present embodiment, the front portion covering abdominal region contracts to have a shorter length than the back portion covering the buttock region. Accordingly, when worn, the garment 1 does not cause a feeling of tightness on the buttock region, and prevents lifting of the knitted fabric on the front portion covering the abdominal region, thereby allowing the knitted fabric to more readily contact with the abdominal region and to apply pressure to the lower abdominal, groin, and genital regions. Therefore, the garment 1 achieves both wearing comfort and promotion of lymph flow in the lower abdominal, groin, and genital regions.

In the garment 1 according to the present embodiment, knitting is started from the lower ends of the tubular leg-side knitted fabric portions 10R and 10L. After the leg-side knitted fabric portions 10R and 10L are knitted to a predetermined length, stitches are gradually decreased at mutually opposing inner-side portions (inner thigh sides) of the right leg-side knitted fabric portion 10R and the left leg-side knitted fabric portion 10L. Thereafter, the body-side knitted fabric portion 30 is knitted between the right leg-side knitted fabric portion 10R and the left leg-side knitted fabric portion 10L.

In the present embodiment, during continuous knitting of the crotch-side knitted fabric portion 20 and the body-side knitted fabric portion 30 from the p tubular leg-side knitted fabric portions 10R and 10L, stitches are decreased at the inner thigh sides. The leg-side knitted fabric portions 10R and 10L and the body-side knitted fabric portion 30 are continuously knitted with stitch decreasing near the crotch-side knitted fabric portion 20 from each of the leg-side knitted fabric portions 10R and 10L toward the body-side knitted fabric portion 30.

Accordingly, the knitted fabric has a sufficiently large area at the thigh portion. As a result, lifting and wrinkling of the knitted fabric due to tensile stress are less likely to occur near a junction between the inner thigh region and the crotch region when worn. The knitted fabric is more likely to contact with three-dimensional curved shapes at bent portions extending from the genital and groin regions to the thigh region, that is, at curved or bent inner portions of the genital and groin regions and the thigh region, and can readily apply pressure to the genital region through which lymph fluid flows from the leg side, lower abdominal side, and buttock side, and to a groin region where lymph nodes are concentrated. As shown in FIGS. 1 to 7, in the central portion of the front of the body-side knitted fabric portion 30, the outer recessed portions 41 and the inner raised portions 32 extending in the wale direction extend substantially linearly from the genital and groin region toward the upper side of the lower abdominal region when worn. In the central portion of the back of the body-side knitted fabric portion 30, the outer recessed portions 41 and the inner raised portions 32 extending in the wale direction extends substantially linearly from the anal side toward the genital and groin regions when worn. Meanwhile, in both left and right sides of the front of the body-side knitted fabric portion 30, the outer recessed portions 41 and the inner raised portions 32 extend upward from each inner thigh region on the left and right sides. Accordingly, the outer recessed portions 41 and the inner raised portions 32 can be aligned with lymph flow extending from legs and buttocks toward the lower abdominal, groin, and genital regions, thereby facilitating guidance of lymph flow from the leg sides and the buttock sides toward the genital and groin regions, and further facilitating guidance of lymph flow from the genital and groin region toward the lower abdominal region. That is, lymph is guided from the legs and the buttocks toward the lymph nodes in the genital and groin regions, and is further guided toward left and right pelvic region, thereby inducing lymph flow toward a subclavian lymph return opening. Therefore, lymph flow is guided from the genital and groin regions toward the lower abdominal region, enabling effective promotion of lymph flow and preventing or reducing accumulation of lymph and occurrence of lymphedema (swelling) due to lymph accumulation.

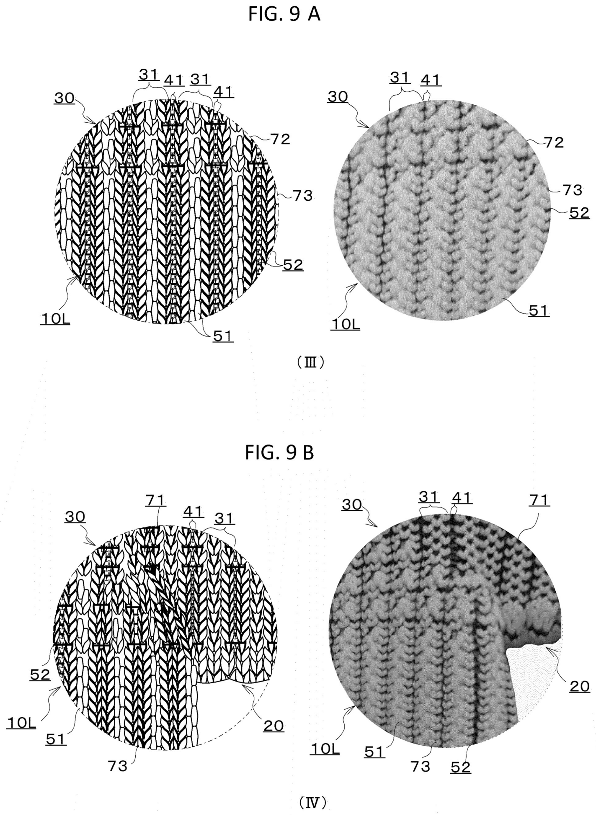

When knitting of the body-side knitted fabric portion 30 is continued using a sum of the number of wales of the crotch-side knitted fabric portion 20 and the constant (unchanged) number of wales from the leg-side knitted fabric portions 10R and 10L without performing stitch decreasing, the front and back of the leg-side knitted fabric portions 10R and 10L, the crotch-side knitted fabric portion 20, and the body-side knitted fabric portion 30 forms a substantially planar configuration. In this case, the upper inner ends of the leg-side knitted fabric portions 10R and 10L do not protrude inward beyond the left and right ends of the crotch-side knitted fabric portion 20. In contrast, when the body-side knitted fabric portion 30 is continuously knitted with the reduced number of wales of the leg-side knitted fabric portions 10R and 10L by stitch decreasing, the front and the back of the leg-side knitted fabric portions 10R and 10L, the crotch-side knitted fabric portion 20, and the body-side knitted fabric portion 30 assumes a more three-dimensionally contoured configuration. In this case, the upper inner ends of the leg-side knitted fabric portions 10R and 10L protrude inward beyond the left and right ends of the crotch-side knitted fabric portion 20 (see FIGS. 9(b) and 12(b)).

In knitting of the crotch-side knitted fabric portion 20, the base yarn 51 and the elastic yarn 52 are also supplied to knitting needles in the intermediate portion on the needle beds that have not been used while the leg-side knitted fabric portions 10R and 10L were being knitted. That is, in addition to the knitting needles at the left and right ends on the front and rear needle beds used for knitting the leg-side knitted fabric portions 10R and 10L, yarns are supplied in a circumferential manner also to knitting needles in the intermediate portion on the front and rear needle beds that had not been used while during knitting of the leg-side knitted fabric portions 10R and 10L. As a result, the pair of left and right leg-side knitted fabric portions 10R and 10L, the crotch-side knitted fabric portion 20, and the body-side knitted fabric portion 30 are continuously knitted. In knitting of the crotch-side knitted fabric portion 20, for example, the base yarn 51 is knitted on the intermediate knitting needles that had not been used on the front and rear needle beds, and the elastic yarn 52 is knitted into the base yarn 51. Thereafter, the rear needle bed is racked to one of the left and right sides, and loops on the knitting needles of the rear needle bed are overlapped and transferred onto the loops on the knitting needles of the front needle bed. Subsequently, the rear needle bed is returned to its original position, and then the rear needle bed is racked to the other of the left and right sides. Next, the loops on the knitting needles of the rear needle bed and the loops on the knitting needles of the front needle bed are overlapped and transferred. Thereafter, the rear needle bed is returned to its original position. Similarly, the loops on the front needle bed are transferred. As a result, the crotch-side knitted fabric portion 20 is knitted such that the front portion and the back portion are integrally and seamlessly formed between the leg-side knitted fabric portions 10R and 10L. Subsequently, the base yarn 51 is supplied in a circumferential manner to the knitting needles at the left and right end portions on the front and rear needle beds used for knitting the leg-side knitted fabric portions 10R and 10L, and to the intermediate knitting needles on the front and rear needle beds used at the beginning of knitting of the crotch-side knitted fabric portion 20. By alternately knitting rib-knit courses of the base yarn 51 and knit courses of the elastic yarn 52, the body-side knitted fabric portion 30 is continuously knitted from the leg-side knitted fabric portions 10R and 10L and the crotch-side knitted fabric portion 20.

In the present embodiment, the crotch-side knitted fabric portion 20 is formed by plain knitting (jersey knitting). That is, the crotch-side knitted fabric portion 20 is formed only of knit stitches on the outer surface of the knitted fabric and only of purl stitches on the inner surface of the knitted fabric. Accordingly, the crotch-side knitted fabric portion 20 has a non-rib region in which alternating raised and recessed portions are not formed in the course direction, unlike rib knitting. The crotch-side knitted fabric portion 20 does not have recessed and raised portions formed by rib knitting and corresponds to the portion in which the raised portions 31 and 32 and the recessed portions 41 and 42 formed in the leg-side knitted fabric portions 10R and 10L and the body-side knitted fabric portion 30 are interrupted. Accordingly, the crotch-side knitted fabric portion 20 is a discontinuous region in which continuity of the raised and recessed portions is interrupted. In particular, the crotch-side knitted fabric portion 20 according to the present embodiment, the base yarn 51 and the elastic yarn 52 are interknitted, is formed only of the knit stitches on the outer surface of the knitted fabric and only of the purl stitches on the inner surface of the knitted fabric.

Thus, in the garment 1 according to the present embodiment, the leg-side knitted fabric portions 10R and 10L and the body-side knitted fabric portion 30 are formed by rib knitting (raised and recess knitting) that forms the raised portions 31 and 32 and the recessed portions 41 and 42 alternately arranged in the circumferential direction and continuously extending in the wale direction. Meanwhile, the crotch-side knitted fabric portion 20 is formed by plain knitting and is the non-rib region having no raised portions 31 and 32 and the recessed portions 41 and 42, that is, the discontinuous region in which continuity of the raised and recessed portions is interrupted. The pair of left and right leg-side knitted fabric portions 10R and 10L and the body-side knitted fabric portion 30 have the raised portions 31 and 32 and the recessed portions 41 and 42 continuously extending in the wale direction, while the crotch-side knitted fabric portion 20, which connects the leg-side knitted fabric portions 10R and 10L and is applied to the genital and groin regions, does not have the raised portions 31 and 32 and the recessed portions 41 and 42 and is the discontinuous region in which continuity of the raised and recessed portions is interrupted.

Accordingly, even when lower limbs of the human body move during wear, the discontinuous region of the crotch-side knitted fabric portion 20, in which continuity of the raised and recessed portions is interrupted, deforms so as to conform to body movement. The presence of this discontinuous region reduces excessive tension and pulling in the knitted fabric, and friction between the knitted fabric and the skin. Further, the presence of the discontinuous region allows movement in the width direction of knitted fabric surrounding the discontinuous region, that is, the knitted fabric of the body-side knitted fabric portion 30 and the leg-side knitted fabric portions 10R and 10L. Even when the lower limbs move, the inner raised portions 32 can be readily brought into close contact with the genital and groin regions. Therefore, the knitted fabric is less likely to become tense or tight, to be pulled, and to cause friction against the skin even in delicate regions such as the genital and groin regions, thereby reducing discomfort and foreign-body sensation. Further, the raised portions 31 and 32 and the recessed portions 41 and 42 of the front and back of the body-side knitted fabric portion 30 enable lymph flow in anterior and posterior regions around the genital and groin regions to be guided to the discontinuous region, thereby further guiding lymph around the genital and groin regions toward the left and right groin regions. Accordingly, promotion of lymph flow in the lower abdominal, groin, and genital regions is enhanced, thereby effectively preventing or reducing lymphedema (and related conditions such as lymph leakage and cellulitis) in the lower abdominal, groin, and genital regions.

In particular, the discontinuous region of the crotch-side knitted fabric portion 20 is formed by plain knitting, and the elastic yarn 52 is interknitted with the loops of the base yarn 51, whereby the region is readily stretchable. Accordingly, even when the lower limbs move, the knitted fabric is less likely to become tense or tight. Further, the inner raised portions 32 of the knitted fabric surrounding the discontinuous region, that is, the knitted fabric of the body-side knitted fabric portion 30 and the leg-side knitted fabric portions 10R and 10L is readily brought into close contact with the genital and groin regions. Therefore, excessive tension and pulling of the knitted fabric are prevented even in delicate regions such as the genital and groin regions, thereby reducing discomfort and foreign-body sensation. In addition, a lymph flow promoting effect in the lower abdominal, groin, and genital regions can be further enhanced.

In the present embodiment, a ratio between the number of wales x corresponding to a maximum knitting width of each of the leg-side knitted fabric portions 10R and 10L and the number of wales y corresponding to a knitting width (crotch width) of the crotch-side knitted fabric portion 20 is preferably set to 2:8 ≤ x:y ≤ 4:6, and more preferably to 2.5:7.5 ≤ x:y ≤ 3.5:5.5. This configuration allows the knitted fabric corresponding to the thigh portion to have a sufficiently large area. Accordingly, the knitted fabric is less likely to be pulled, to lift, and to wrinkle near the junction between the inner thigh region and the genital and groin regions when worn. As a result, the knitted fabric is more likely to contact with three-dimensional curved shapes at bent portions extending from the genital and groin regions to the thigh region, that is, curved or bent inner-side portions of the genital and groin regions and the thigh region, thereby readily applying pressure to the genital and groin regions where lymph nodes are concentrated. Further, the knitted fabric can more readily apply pressure to regions through which lymph flows from the legs, the lower abdomen, and buttocks toward the genital and groin regions. Therefore, lymph flow can be more readily guided from the genital and groin regions toward the lower abdominal region, thereby enabling effective promotion of lymph flow.

In the present embodiment, the body-side knitted fabric portion 30 is knitted such that the number of wales is gradually reduced from the longitudinally central portion thereof toward the upper side (toward the upper opening portion 31). Accordingly, the knitted fabric can readily fit the waist shape of the human body, is prevented from lifting and slipping, and can readily apply appropriate pressure to the lower abdominal, groin, and genital regions. Therefore, promotion of lymph flow can be enhanced.

In the present embodiment, the front and back of the body-side knitted fabric portion 30 is knitted with stitch decreasing toward the upper side. As a result, in the region extending from the longitudinally central portion toward the upper side in the front and back of the body-side knitted fabric portion 30, the outer raised portions 31 and the inner recessed portions 42 on both left and right sides thereof are inclined slightly inward toward the upper side. Some of the raised portions 31 and 32 and the recessed portions 41 and 42 terminate before reaching the upper opening portion 31 while extending upward.

As described above, in the present embodiment, the garment 1 is manufactured by integrally knitting the leg-side knitted fabric portions 10R and 10L each having a small-diameter tubular shape, the crotch-side knitted fabric portion 20 connecting the leg-side knitted fabric portions 10R and 10L, and the body-side knitted fabric portion 30 having a large-diameter tubular shape and continuously extending in the wale direction from the leg-side knitted fabric portions 10R and 10L and the crotch-side knitted fabric portion 20, using a seamless flat knitting machine that continuously knits stitches in the transverse direction.

Thus, the garment 1 according to the present embodiment is formed by integrally knitting, using a seamless flat knitting machine, the leg-side knitted fabric portions 10R and 10L each having a small-diameter tubular shape and configured to cover the thigh region, the crotch-side knitted fabric portion 20 connecting the leg-side knitted fabric portions 10R and 10L and configured to cover the genital and groin regions and the body-side knitted fabric portion 30 having a large-diameter tubular shape and configured to cover the abdominal region and the buttock region from the genital and groin regions. Therefore, since the garment 1 has no sewn portions that are stiff and non-stretchable, the garment 1 provides a comfortable feel against the skin and a comfortable fit when worn. In addition, since the garment 1 has no sewn portions, the garment 1 is prevented from locally lifting away from the skin due to the knitted fabric being excessively stretched and thinned and becoming tense. In particular, as compared with circular knitting, flat knitting can form a thicker and stiffer knitted fabric using a relatively thick knitting yarn. Accordingly, when worn, the knitted fabric that functions as a rigid wall against the skin. As a result, when muscles contract and bulge due to body movement, lymphatic vessels are compressed by the knitted fabric that functions as the rigid wall, thereby facilitating flow of accumulated lymph fluid.

The leg-side knitted fabric portions 10R and 10L, the crotch-side knitted fabric portion 20, and the body-side knitted fabric portion 30 are continuously knitted without switching the type of the base yarn 51 and the elastic yarn 52. In addition, the leg-side knitted fabric portions 10R and 10L and the body-side knitted fabric portion 30 are continuously knitted without switching the rib knitting structure to another knitting structure. Since the knitted fabric is formed without switching the yarns and the knitting structure, the knitted fabric does not bite into the skin at switching portions and thus does not inhibit lymph flow.

The leg-side knitted fabric portions 10R and 10L, the crotch-side knitted fabric portion 20, and the body-side knitted fabric portion 30 are knitted using the base yarn 51 having a fineness of preferably 1000 to 3000 denier, more preferably 1200 to 3000 denier, and still more preferably 1500 to 3000 denier. Use of this base yarn 51 enables the leg-side knitted fabric portions 10R and 10L, the crotch-side knitted fabric portion 20, and the body-side knitted fabric portion 30 to have a thickness of preferably 2.0 mm to 10.0 mm, more preferably 2.0 mm to 8.0 mm, and still more preferably 2.5 mm to 5.0 mm, thereby forming a thick and stiff knitted fabric. This thick and stiff knitted fabric has low stretchability and high stiffness, thereby enhancing a muscle pump effect and a massage effect and further promoting lymph flow.

In the garment 1 according to the present embodiment, the elastic yarn 52 is missed with respect to the knit stitches of the base yarn 51 forming the outer recessed portions 41 and the inner raised portions 32, which are formed by rib knitting as textured knitting and appear convex on the inner surface and concave on the outer surface. Floating yarn of the elastic yarn 52 formed by stitch missing is exposed at the recessed portions (outer recessed portions 41) on the outer surface, and the elastic yarn 52 is not exposed at the raised portions (inner raised portions 32) on the inner surface, thereby eliminating contact of the elastic yarn 52 with the skin and ensuring moderate stretchability in a transverse direction. Meanwhile, the elastic yarn 52 is knitted into the knit stitches of the base yarn 51 forming the outer raised portions 31 and the inner recessed portions 42, which are formed by rib knitting as textured knitting and appear convex on the outer surface and concave on the inner surface. This configuration suppresses stretchability of the knitted fabric in the transverse direction and in the in-plane and increases stretch stiffness. Therefore, the garment 1 achieves both moderate transverse stretchability and high stiffness, thereby enabling compatibility of ease of donning and doffing, comfort during wearing, and promotion of lymph flow through a muscle pump effect and a massage effect.

In the garment 1 according to the present embodiment, the leg-side knitted fabric portions 10R and 10L and the body-side knitted fabric portion 30 are knitted by rib knitting as textured knitting that forms the raised portions 31 and 32 and the recessed portions 41 and 42 alternately arranged in the circumferential direction and extending in the wale direction. Since the raised and recessed portions extending in the wale direction are alternately arranged in the circumferential direction, and the recessed portions 41 and 42 are present between adjacent raised portions 31 and 32, the recessed portions 41 and 42 between the raised portions 31 and 32 can deform so as to conform to body shape and movement through changes in the spacing between the raised portions 31 and 32. Consequently, the inner raised portions 32 remain in close contact with the body and continuously apply pressure, thereby allowing pressure to be more readily applied to the lower abdominal, groin, and genital regions. Further, the alternating arrangement of the raised portions 31 and 32 and the recessed portions 41 and 42 in the circumferential direction yields variations in pressure (strong pressure and weak pressure), thereby generating a massage effect and further promoting lymph flow. In particular, the raised portions 31 and 32 and the recessed portions 41 and 42 extend continuously in the wale direction corresponding to the direction of lymph flow of lymphatic vessels in the lower abdominal, groin, and genital regions during wearing, and thus are less likely to inhibit lymph flow. Rather, when worn, a longitudinal pulling force is more readily generated from the genital and groin regions toward the upper abdominal region, thereby facilitating lymph flow from the groin region toward the subclavian lymph return site.

The garment 1 according to the present embodiment has moderate stretchability due to transverse stretchability provided by rib knitting and further due to stretchability of the elastic yarn 52. This garment 1 allows the inner raised portions 32 to be brought into close contact with the skin in conformity with body shape and movement. Since the inner raised portions 32 have a relatively large surface area, they are readily maintained in close contact with the skin even when the body moves. In particular, since rib knitting is employed as textured knitting, the spacing between adjacent inner raised portions 32 readily changes, thereby allowing the inner raised portions 32 to continuously apply pressure to body portions even when the body moves. Further, since flat knitting enables formation of the thick and stiff knitted fabric, the knitted fabric is less likely to become excessively thin or overly tight due to stretching. Accordingly, even when the body moves, the knitted fabric is less likely to wrinkle or lift away from the skin due to tension, and the inner raised portions 32 can be consistently maintained in close contact with the skin. Therefore, the inner raised portions 32 can be readily brought into close contact with, and stably apply pressure to the lower abdominal, groin, and genital regions, thereby enhancing the effect of promoting lymph flow.
In addition, due to transverse stretchability provided by rib knitting and further due to stretchability of the elastic yarn 52, the knitted fabric has stretchability in the transverse direction and the circumferential direction, thereby facilitating ease of donning and doffing.

Thus, the garment 1 according to the present embodiment is formed of the thick and stiff knitted fabric by flat knitting using the base yarn 51 and the elastic yarn 52 interknitted therewith. In the leg-side knitted fabric portions 10R and 10L and the body-side knitted fabric portion 30, the elastic yarn 52 is interknitted with the loops of the base yarn 51 by a combination of knitting and missing. Transverse stretchability is exhibited at the portions where the elastic yarn 52 is missed, whereas stretchability in the transverse direction and the in-plane direction is suppressed at the portions where the elastic yarn 52 is knitted. As a result, moderate transverse stretchability facilitates ease of donning and doffing, while suppression of in-plane stretchability increases stiffness, thereby enabling promotion of lymph flow through enhancement of a muscle pump effect and a massage effect. Therefore, lymph flow can be promoted without applying high compressive force that would otherwise cause a feeling of tightness or discomfort, and enhancement of a muscle pump effect and a massage effect can be achieved with a comfortable compressive force. Further, since the garment 1 can be worn comfortably for a long period of time, the garment 1 enables prevention and reduction of lymphedema (and related conditions such as lymph leakage and cellulitis), as well as suppression of exacerbation thereof, and inhibition of worsening thereof in the lower abdominal, groin, and genital regions.

Since the garment 1 according to the present embodiment is formed of the thick and stiff knitted fabric by flat knitting, the knitted fabric is less likely to wrinkle even at bent portions of the body during wearing. In addition, since the garment 1 is knitted by rib knitting as textured knitting to form the raised portions 31 and 32 and the recessed portions 41 and 42 extending in the wale direction, the knitted fabric is configured to have tension in the direction in which the raised and recessed portions extend during wearing, thereby allowing the knitted fabric to be less likely to wrinkle. Accordingly, the knitted fabric does not bite into the skin due to wrinkles, thereby not inhibiting lymph flow. Rather, since the raised portions 31 and 32 and the recessed portions 41 and 42 extend in the wale direction, the inner raised portions 32 that apply pressure to the lower abdominal, groin, and genital regions during wearing are aligned along the flow direction of lymphatic vessels. Therefore, lymph flow is not inhibited and can be guided in its natural flow direction, thereby promoting lymph flow.

In the garment 1 according to the present embodiment, the leg-side knitted fabric portions 10R and 10L and the body-side knitted fabric portion 30 are continuously knitted with stitch decreases from each of the leg-side knitted fabric portions 10R and 10L toward the body-side knitted fabric portion 30 near the crotch-side knitted fabric portion 20. This allows each leg-side knitted fabric portions 10R and 10L to have a larger area at portions corresponding to the inner proximal portion of the thigh region when worn. As a result, the knitted fabric is prevented from lifting even along three-dimensional curved shapes extending from the thigh regions to the genital and groin regions, and the inner raised portions 32 of the knitted fabric can readily press the regions extending from the thigh region to the genital and groin regions where inguinal lymph nodes are located. Further, when worn, in the central portion on the front of the body-side knitted fabric portion 30, the outer recessed portions 41 and the inner raised portions 32 extending in the wale direction extend substantially linearly upward from the genital and groin regions toward the upper portion of the lower abdominal region. Meanwhile, on the left and right sides of the front of the body-side knitted fabric portion 30, the outer recessed portions 41 and the inner raised portions 32 extend upward from the inner thigh regions. Accordingly, the outer recessed portions 41 and the inner raised portions 32 are arranged so as to follow the direction of lymph flow toward the groin region. As a result, lymph flow from the leg and genital regions toward the inguinal region, and further from the inguinal region toward the subclavian lymph return site, can be more readily guided. Consequently, lymph flow can be promoted in a two-dimensional manner, thereby enhancing the effects of preventing and reducing lymphedema in the genital and groin regions.

The garment 1 according to the present embodiment promotes lymph flow in the lower abdominal, groin, and genital regions simply by being worn, thereby eliminating the need to separately wear pads. Accordingly, skin irritation caused by moisture accumulation resulting from wearing such pads does not occur. In particular, since the thick and stiff knitted fabric is formed by flat knitting and rib knitting is employed as textured knitting, the knitted fabric is less likely to wrinkle and bite into the skin. Further, coarse stitches are locally formed by performing stitch transfer at the portions where the elastic yarn 52 is knitted into the base yarn 51, thereby improving air permeability and preventing moisture accumulation. In addition, the use of a cotton material for the base yarn 51 further prevents moisture accumulation. Moreover, the elastic yarn 52 is missed with respect to knit stitches of the base yarn 51 forming the outer recessed portions 41 and the inner raised portions 32, which are formed by rib knitting as textured knitting and appear convex on the inner surface and concave on the outer surface. Floating yarn of the elastic yarn 52 formed by stitch missing is exposed at the recessed portions (outer recessed portions 41) on the outer surface, and the elastic yarn 52 is not exposed at the raised portions (inner raised portions 32) on the inner surface, thereby eliminating contact of the elastic yarn 52 with the skin. As a result, the garment 1 is less likely to cause skin irritation and damage. That is, the garment 1 exhibits high preventive and protective effects against pain, skin damage, skin lacerations, and skin tears.

Thus, the garment 1 according to the present embodiment includes the body-side knitted fabric portion 30 that has a large-diameter tubular shape and configured to cover the abdominal region, the buttock region, and the genital and groin regions; the leg-side knitted fabric portions 10R and 10L that branch from the body-side knitted fabric portion 30, has a small-diameter tubular shape, and configured to cover thigh regions; and the crotch-side knitted fabric portion 20 that connects the leg-side knitted fabric portions 10R and 10L configured to cover the genital and groin regions. The leg-side knitted fabric portions 10R and 10L, the crotch-side knitted fabric portion 20, and the body-side knitted fabric portion 30 are integrally knitted by a seamless flat knitting machine. Since the garment 1 has no sewn portions, it provides an improved feel against the skin. In addition, even at bent portions of the body such as the genital and groin regions and inner thigh region during wearing, the knitted fabric is prevented from lifting due to excessive tension, and can be readily brought into close contact with the body. As a result, the knitted fabric can readily apply pressure to the portions where suppression of edema is required, such as the lower abdominal, groin, and genital regions. In particular, since flat knitting by a seamless flat knitting machine enables formation of the thick and stiff knitted fabric, the knitted fabric is less likely to wrinkle, and biting of the knitted fabric into the skin due to wrinkles can be prevented even at delicate portions such as the genital and groin regions and the inner thigh region, thereby improving wearing comfort. Accordingly, appropriate compressive pressure can be readily applied even to curved or bent portions such as the genital and groin regions and the inner thigh regions. Further, since flat knitting by a seamless flat knitting machine enables formation of the thick and stiff knitted fabric, the knitted fabric that functions as the rigid wall against the skin, thereby facilitating generation of a muscle pump effect and a massage effect and enabling promotion of lymph flow.

Accordingly, lymph flow can be promoted with a moderate compressive pressure without requiring a high compressive pressure. As a result, the garment 1 provides improved comfort against the skin, and since excessive compressive pressure is not required, comfortable wearing is achieved. Further, such comfortable wearing enables long-term continuous wearing of the garment 1, thereby providing effects of preventing and reducing lymphedema (and related conditions such as lymph leakage and cellulitis), as well as exacerbation and progression thereof in the lower abdominal, groin, and genital regions. In particular, the garment 1 enables prevention and reduction of lymphedema (and related conditions such as lymph leakage and cellulitis) in the genital and groin regions (including the pubic region), which causes significant physical and psychological distress, and can significantly alleviate patient suffering.

Further, even after repeated washing, the garment 1 is less likely to lose its shape. In addition, the use of a cotton material for the base yarn 51 prevents moisture accumulation and improves comfort against the skin. As a result, the garment 1 exhibits enhanced preventive and protective effects against pain, skin damage, skin lacerations, and skin tears.

In the garment 1 according to the present embodiment, the leg-side knitted fabric portions 10R and 10L and the body-side knitted fabric portion 30 are formed by rib knitting as textured knitting, and have the raised portions 31 and 32 and the recessed portions 41 and 42 that are alternately formed over the entire circumference. In other words, the knitted fabric is formed in a ribbed or wave shape in which the raised and recessed portions are alternately and continuously arranged in the circumferential direction of the tubular structure, and each of the raised and recessed portions continuously extends in the longitudinal direction (along the length of the human body), that is, in the wale direction. Thus, the leg-side knitted fabric portions 10R and 10L and the body-side knitted fabric portion 30 each have the raised and recessed portions (the raised portions 31 and 32 and the recessed portions 41 and 42) that extend in the wale direction and are alternately arranged over the entire circumference.

In the garment 1 according to the present embodiment, the leg-side knitted fabric portions 10R and 10L and the body-side knitted fabric portion 30 are knitted by rib knitting as textured knitting, in which the raised portions 31 and 32 and the recessed portions 41 and 42 are alternately arranged in the circumferential direction and extend in the wale direction. Accordingly, the raised portions 31 and 32 and the recessed portions 41 and 42 are alternately arranged in the transverse direction (the circumferential direction) on both the outer circumferential surface and the inner circumferential surface, and the recessed portions (grooves) 41 and 42 are present between adjacent raised portions 31 and 32. As a result, when worn, the recessed portions 41 and 42 between the raised portions 31 and 32 can deform so as to conform to body shape through changes in spacing between the raised portions 31 and 32. Consequently, the knitted fabric is less likely to bite into the skin due to wrinkles and to inhibit lymph flow. Moreover, the inner raised portions 32 are maintained in close contact with the body and continuously apply pressure, thereby allowing pressure to be more readily applied to the lower abdominal, groin, and genital regions. In addition, variation in compressive pressure due to the alternating arrangement of the raised portions 31 and 32 and the recessed portions 41 and 42 yields a massage effect, thereby further improving lymph flow. In particular, the raised portions 31 and 32 and the recessed portions 41 and 42, which continuously extend in the wale direction, are oriented along the direction of lymph flow extending upward (toward the clavicular side) from the genital and groin regions. Accordingly, the raised portions 31 and 32 and the recessed portions 41 and 42 do not inhibit lymph flow. Rather, during wearing, they can provide a vertical tensile force tending to pull upward from the genital and groin regions toward an upper abdominal region, thereby facilitating flow of lymph from the genital and groin regions toward the subclavian lymph return site. Further, rib knitting allows high stretchability in the transverse direction and the circumferential direction of the knitted fabric, thereby facilitating donning and doffing.

The crotch-side knitted fabric portion 20, which connects the leg-side knitted fabric portions 10R and 10L configured to be applied to the genital and groin regions, does not have raised portions 31 and 32 and recessed portions 41 and 42, and is the discontinuous region in which continuity of the raised portions and recessed portions is interrupted. Accordingly, even when the lower limbs move during wearing, the discontinuous region of the crotch-side knitted fabric portion 20 can deform so as to conform to changes in body shape. As a result, excessive tension and pulling in the knitted fabric, and friction between the knitted fabric and the skin is less likely to occur. Consequently, the knitted fabric surrounding the crotch-side knitted fabric portion 20, in particular, the inner raised portions 32, be more readily brought into contact with the genital and groin regions.

Accordingly, even with respect to delicate portions such as the genital and groin regions, the knitted fabric is less likely to experience excessive tension, pulling, and friction against the skin, and is therefore less likely to cause discomfort or foreign-body sensation. Further, the raised portions 31 and 32 and the recessed portions 41 and 42 of the front and back of the body-side knitted fabric portion 30 allow lymph flow at anterior and posterior sides around the genital and groin region to be guided to the discontinuous region, thereby enabling lymph flow around the genital region to be guided toward the left and right inguinal regions. Accordingly, lymph flow in the lower abdominal, groin, and genital regions is promoted, and lymphedema (and related conditions such as lymph leakage and cellulitis) in the lower abdominal, groin, and genital regions can be effectively prevented and reduced.

In particular, in the discontinuous region of the crotch-side knitted fabric portion 20, which is plain-knitted and has no raised or recessed portions, the elastic yarn 52 is knitted into the loops of the base yarn 51. Accordingly, this region is readily stretchable, so that even when the lower limbs move, the knitted fabric is less likely to experience excessive tension, pulling, and friction against the skin. As a result, the knitted fabric surrounding the crotch-side knitted fabric portion 20, in particular, the inner raised portions 32, can more be readily brought into contact with the genital and groin regions. Therefore, even with respect to delicate portions such as the genital and groin regions, the knitted fabric is less likely to be subjected to excessive tension and pulling, thereby further reducing discomfort and foreign-body sensation. In addition, this configuration can enhance the effect of promoting in the lower abdominal, groin, and genital regions.

In the garment 1 according to the present embodiment, the leg-side knitted fabric portions 10, the crotch-side knitted fabric portion 20, and the body-side knitted fabric portion 30 are formed of a knitted structure in which the base yarn 51 and the elastic yarn 52 are interknitted. With respect to the base yarn 51, the portions in which the elastic yarn 52 is knitted and the portions in which the elastic yarn 52 is missed are alternately arranged in the course direction, thereby achieving both moderate stretchability and moderate stiffness of the knitted fabric. Accordingly, the garment 1 is easy to don and doff and provides moderate compression without causing a feeling of tightness. Further, the garment 1 exhibits increased stiffness, thereby enhancing a muscle pump effect and a massage effect, and further enhancing the effect of promoting lymph flow.

In particular, in the garment 1 according to the present embodiment, the elastic yarn 52 is knitted into the stitches of the base yarn 51 forming the outer raised portions 31 and the inner recessed portions 42, which appear convex on the outer surface and concave on the inner surface of the rib-knitted fabric, thereby forming the knit stitches of the elastic yarn 52. Meanwhile, the elastic yarn 52 is missed with respect to the knit stitches of the base yarn 51 forming the inner raised portions 32 and the outer recessed portions 41, which appear concave on the outer surface and convex on the inner surface, thereby forming floating yarn of the elastic yarn 52 on the outer recessed portions 41. Accordingly, when the garment 1 is worn with the inner surface facing the human body, the floating yarn of the elastic yarn 52 is exposed on the outer surface, while the elastic yarn 52 is not exposed on the inner surface of the inner raised portions 32. As a result, even when the inner raised portions 32 press against the skin, the elastic yarn 52 does not come into contact with the skin. Therefore, even in the delicate genital and groin regions, the elastic yarn 52 is unlikely to cause skin irritation, and irritation to the skin due to the material, elasticity, friction, and the like of the elastic yarn 52 can be effectively prevented.

In the garment 1 according to the present embodiment, the knit stitches forming the outer raised portions 31 and the inner recessed portions 42, in which the elastic yarn 52 is knitted into the knit stitches of the base yarn 51, include stitch-transfer portions and non-stitch-transfer portions for every course or at predetermined course intervals, which are formed by varying the knitting pattern. This configuration locally varies the elongation rate and stiffness of the knitted fabric.

In the garment 1 according to the present embodiment, the body-side knitted fabric portion 30 includes the low-stretch region 71 having a lower elongation rate than other regions and covering the lower abdominal, groin, and genital regions when worn. Accordingly, the region covering the genital and the lower abdominal regions (including the groin region) during wearing exhibits increased stiffness, thereby enhancing a muscle pump effect and a massage effect. Meanwhile, regions other than the lower abdominal, groin, and genital regions are more stretchable, thereby facilitating donning and doffing and enabling comfortable wearing without causing a feeling of tightness. As a result, compatibility between wearing comfort and a lymph flow promoting effect in the lower abdominal, groin, and genital regions can be achieved.

Further, the garment 1 according to the present embodiment includes the intermediate-stretch region 72 surrounding the low-stretch region 71 and the high-stretch region 73 adjacent to the intermediate-stretch region 72. The intermediate-stretch region 72 has a higher elongation rate than the low-stretch region 71, and the high-stretch region 73 has a higher elongation rate than the intermediate-stretch region 72. Varying the stretchability in a stepwise manner reduces a load applied to the base yarn 51 due to differences in elongation rate, thereby making the garment 1 resistant to permanent deformation. Accordingly, even after repeated washing, the garment 1 is less likely to lose its shape or stretch out, thereby exhibiting high durability.

In the garment 1 according to the present embodiment, the left and right leg-side knitted fabric portions 10R and 10L are the high-stretch region 73 having a high proportion of transferred stitches and low stitch density. Further, the front of the body-side knitted fabric portion 30, along the longitudinal direction, has a high proportion of the low-stretch region 71 having no transferred stitches and high stitch density, and the intermediate-stretch region 72 having a lower proportion of transferred stitches than the high-stretch region 73. Meanwhile, the back of the body-side knitted fabric portion 30, along the longitudinal direction, has a high proportion of the high-stretch region 73 having a high proportion of transferred stitches and a low stitch density. As a result, the front portion of the body-side knitted fabric portion 30 is shorter in length than the back portion. Accordingly, when worn, the garment 1 does not cause a feeling of tightness on the buttock region, while preventing lifting of the knitted fabric away from the body on the front covering the abdominal region and allowing the knitted fabric to more readily contact with the abdominal region, thereby facilitating application of pressure to the lower abdominal, groin, and genital regions. Therefore, the garment 1 can achieve both wearing comfort and a lymph flow promoting effect in the lower abdominal, groin, and genital regions.

In the garment 1 according to the present embodiment, the leg-side knitted fabric portions 10R and 10L and the body-side knitted fabric portion 30 are continuously knitted with stitch decreases from each of the leg-side knitted fabric portions 10R and 10L toward the body-side knitted fabric portion 30 near the crotch-side knitted fabric portion 20. This configuration allows the leg-side knitted fabric portions 10R and 10L to have increased areas. Accordingly, when worn, the knitted fabric does not lift away from the body even near inner proximal portions of the thigh region, that is, along three-dimensional curved shapes extending from the thigh regions to the genital and groin regions, and can readily apply pressure to regions extending from the thigh region to the genital and groin regions where inguinal lymph nodes are present. Therefore, a lymph flow promoting effect in the lower abdominal, groin, and genital regions can be further enhanced.

Thus, the garment 1 according to the present embodiment can readily apply pressure even to the lower abdominal, groin, and genital regions and has increased stiffness, thereby enhancing a muscle pump effect and a massage effect. As a result, the garment 1 enables promotion of lymph flow in the lower abdominal, groin, and genital regions without the need to separately wear pads. That is, the labor of separately wearing pads can be eliminated, and simply by wearing the garment 1 without the need to wear separate pads, the knitted fabric can closely contact the lower abdominal, groin, and genital regions to apply pressure thereto. In addition, the thick and stiff knitted fabric can enhance a muscle pump effect and a massage effect, thereby promoting lymph flow.

In the garment 1 according to the present embodiment, with respect to the lower abdominal, groin, and genital regions, excessive tension in the knitted fabric and lifting of the knitted fabric from the body surface are prevented, thereby facilitating close contact of the knitted fabric with the body, and facilitating application of pressure by the inner protrusions 32. Further, since the garment 1 has increased stiffness, the garment 1 can enhance a muscle pump effect and a massage effect and thus promote lymph flow, even without increasing compressive pressure. By avoiding excessively high compressive pressure, the garment 1 can provide wearing comfort. Favorable fit and comfortable wearability enable long-term continuous wearing of the garment 1. As a result, the garment 1 achieves prevention and reduction of lymphedema (and related conditions such as lymph leakage and cellulitis) in the lower abdominal, groin, and genital regions, as well as suppression of exacerbation and progression thereof. For example, therapeutic and preventive effects can be provided for localized edema in the lower abdominal regions (including the groin region) due to lymphatic causes (such as developmental malformation, lymph node dissection, and lymphangitis), as well as for localized edema in the lower abdominal region due to venous causes (such as varicose veins, deep vein thrombosis, post-thrombotic syndrome, and pulmonary thromboembolism). In addition, therapeutic and preventive effects can be provided for localized edema in the lower abdominal regions (including the groin region) associated with inflammatory causes (various inflammations), allergic causes (due to drugs, plants, insect bites, etc.), vasoneurotic causes (such as Quincke's edema), disuse causes (such as prolonged bedridden states and paralysis), and sequelae of trauma or general surgery.

The garment 1 according to the present embodiment is a compression garment having elasticity throughout and used in compression therapy for applying pressure to a part of the human body, and may be treated as a medical device for the treatment of lymphedema. The compressive pressure can be set to any desired value, for example, within a range of 3 to 70 mmHg, by adjusting parameters such as the number of the stitches of the base yarn 51 and the elastic yarn 52, the elasticity of the elastic yarn 52, the manner in which the elastic yarn 52 is interknitted with the base yarn 51, and the loop diameter of the base yarn 51 and the elastic yarn 52, and the like. Accordingly, a desired compressive pressure can be easily designed by adjusting the number of stitches or the stitch density.

In particular, when a high compressive pressure is applied to delicate portions such as the genital and groin regions, there is a risk of causing skin damage, skin lacerations, and skin tears. Excessively high compressive pressure may conversely inhibit lymphatic return. However, the garment 1 of the present embodiment is formed of the thick and stiff knitted fabric, thereby enhancing a muscle pump effect and a massage effect and thus achieving a lymph flow promoting effect without increasing compressive pressure. Furthermore, the thick and stiff knitted fabric is less likely to bite into the skin, thereby reducing the risk of skin damage, skin lacerations, and skin tears. Therefore, the garment 1 achieves a lymph flow promoting effect while maintaining excellent wearing comfort.

The garment 1 according to the present embodiment is formed of the knitted fabric integrally knitted by a seamless flat knitting machine and has elasticity. That is, the leg-side knitted fabric portions 10R and 10L, the crotch-side knitted fabric portion 20, and the body-side knitted fabric portion 30, which are integrally knitted by a seamless flat knitting machine, are stretchable by virtue of being knitted fabric. In particular, elasticity provided by the elastic yarn 52 and stretchability provided by rib knitting facilitate donning of the garment 1. When donning the garment 1, it is only necessary to put it on against elasticity in the tubular diameter direction perpendicular to the longitudinal direction of the garment 1. Therefore, even a person who is not skilled can easily don and doff the garment 1 without excessive effort by applying predetermined force in the tubular diameter direction and the longitudinal direction. Thus, the garment 1 can be smoothly donned and doffed, is easy to handle, and is not subjected to excessive force during donning and doffing, thereby providing excellent durability. Even elderly persons or women with relatively low physical strength can easily and smoothly don and doff the garment 1 with a weak force by applying force in the longitudinal direction of the garment 1 and in the direction perpendicular thereto. Meanwhile, when worn, the wearer can receive an appropriate compressive pressure as the garment 1 conforms to the body shape of the wearer.

In particular, the region covering the lower abdominal, groin, and genital regions is configured as the low-stretch region 71 having high stiffness, while its surrounding region is configured to have high stretchability. Accordingly, the garment 1 can be donned without requiring a strong force, thereby facilitating donning even for elderly persons or women having relatively low physical strength. After donning, the region covering the region extending from the genital and groin regions to the lower abdominal region can provide a muscle pump effect and a massage effect by virtue of the high stiffness resulting from the low stretchability of the low-stretch region 71, thereby enabling promotion of lymph flow in the lower abdominal, groin, and genital regions.

In the leg-side knitted fabric portions 10R and 10L and the body-side knitted fabric portion 30 of the garment 1 according to the present embodiment, the base yarn 51 and the elastic yarn 52 are knitted in alternating courses. In the inner raised portions 32 and the outer recessed portions 41 formed by rib knitting, the elastic yarn 52 is formed as a miss stitch with respect to the loops of the base yarn 51, thereby ensuring stretchability in the transverse direction. Meanwhile, in the inner recessed portions 42 and the outer raised portions 31, the elastic yarn 52 is knitted into the loops of the base yarn 51, thereby suppressing stretchability in the longitudinal direction and the in-plane direction relative to the transverse direction. As a result, the garment 1 can be easily donned and doffed, and during wearing, exhibits appropriate stiffness to provide a muscle pump effect and a massage effect. In addition, the garment 1 is less likely to slip even when the wearer moves, and can maintain a stable and comfortable fit.

The garment 1 according to the present embodiment has no sewn portions, thereby providing wearing comfort. Specifically, in the garment 1 of the present embodiment, each of the leg-side knitted fabric portions 10R and 10L is first knitted into a seamless tubular shape by a seamless flat knitting machine. After the leg-side knitted fabric portions 10R and 10L are knitted to a predetermined length in the wale direction, the crotch-side knitted fabric portion 20 is continuously knitted in the course direction between the leg-side knitted fabric portions 10R and 10L by the seamless flat knitting machine. Further, the body-side knitted fabric portion 30 is knitted into a seamless tubular shape to a predetermined length in the wale direction from the leg-side knitted fabric portions 10R and 10L and the crotch-side knitted fabric portion 20 by the seamless flat knitting machine. As a result, the pants-type compression garment 1 has no seams and therefore does not cause stress on the skin due to seams, and is less likely to lose its shape due to washing. In addition, since the garment 1 has no sewn seams, it is not subjected to stress on seams caused by stretching, whereby tensile forces are dispersed and excellent durability can be achieved. Such a seamless structure facilitates overall management of yarn ends.

The garment 1 of the present embodiment is flat-knitted, allowing the use of thicker yarns and a lower stitch density than circular knitting, thereby improving air permeability. The garment 1 of the present embodiment, which is knitted by a seamless flat knitting machine, has transferred stitches formed at predetermined portions by transferring (overlapping) the loops of the base yarn 51 and the elastic yarn 52 to adjacent needles. As a result, when the knitted fabric is stretched, openings (openwork eyelets), in which stitches open widely, are formed, thereby improving air permeability. Thus, the garment 1 has transferred stitches formed by stitch transfer of the loops of the base yarn 51 and the elastic yarn 52, and therefore exhibits improved air permeability. Accordingly, the garment 1 is resistant to moisture accumulation. Preferably, the use of a thick cotton yarn, which is resistant to wrinkling, as the base yarn 51 further prevents moisture accumulation and improves wearing comfort.

Garments knitted by conventional circular knitting machines have fine stitches and are prone to wrinkling, and therefore exhibit poor air permeability in regions having a high stitch density. Further, knitted fabrics made of nylon are likely to cause allergic reactions on the skin.
In contrast, the garment 1 of the present embodiment is flat-knitted using a relatively thick yarn, thereby having a low stitch density and improved air permeability. In addition, since the garment 1 is flat-knitted using relatively a thick yarn, the garment 1 is thick and stiff, thereby being less likely to wrinkle, and preventing inhibition of lymph flow due to biting of wrinkles into the skin. Preferably, the use of cotton materials as the base yarn 51 improves moisture absorbency, suppresses moisture accumulation, and provides more comfortable wearability.
Accordingly, the garment 1 of the present embodiment can achieve both promotion of lymph flow and improvement of air permeability.

The garment 1 according to the present embodiment is formed by flat knitting. The base yarn 51 has a fineness of preferably 1000 to 3000, more preferably 1200 to 3000, and still more preferably 1500 to 3000. The knitted fabric of the leg-side knitted fabric portions 10R and 10L, the crotch-side knitted fabric portion 20, and the body-side knitted fabric portion 30 have a thickness of preferably 2.0 mm to 10.0 mm, more preferably 2.0 mm to 8.0 mm, and still more preferably 2.5 mm to 5.0 mm. The elastic yarn 52 is interknitted with the base yarn 51. Consequently, as compared with garments formed by circular knitting, the garment 1 is thicker and stiffer, exhibits reduced stretchability and has high stiffness. The elastic yarn 52 is knitted into the stitches of the base yarn 51 forming the outer raised portions 31 and the inner recessed portions 42, whereas the elastic yarn 52 is formed as a miss stitch with respect to knit stitches of the base yarn 51 forming the outer recessed portions 41 and the inner raised portions 32. With this knitting configuration, the portions where the elastic yarn 52 is missed have relatively high stretchability, and the portions where the elastic yarn 52 is knitted have relatively low stretchability, thereby ensuring both ease of donning and doffing and increased stiffness.

When the garment 1 according to the present embodiment is worn, the circumference and volume (containing muscle volume) of a body portion on which the garment 1 is worn changes in response to movements of the wearer, such as walking, exercising, and changes in posture including sitting, standing, and lying. The garment 1 of the present embodiment is thicker and stiffer than circular-knitted garments and has suppressed elongation of the elastic yarn 52 at the portions where the elastic yarn 52 is interknitted. As a result, the garment 1 exhibits high stiffness (force resisting elongation of the knitted fabric). The knitted fabric with higher stiffness produces a larger increase in compressive pressure when stretched, and has relatively low compliance with changes in the circumference (muscle volume) of the applied body portion, and does not sufficiently stretch in accordance with such changes. As a result, when the circumference of the body is large (i.e., when muscles are contracted), the compressive pressure increases significantly, whereas when the circumference is small (i.e., when muscles are relaxed), the compressive pressure decreases, thereby generating a difference in compressive pressure between contraction and relaxation phases. This difference between increases and decreases in compressive pressure (a pressure difference between contraction and relaxation phases) produces a milking effect on lymph fluid, thereby enhancing a muscle pump effect and a massage effect. Thus, the knitted fabric having higher stiffness generates a larger increase in compressive pressure when muscle volume or circumference increases due to muscle bulging, whereas during muscle relaxation, the knitted fabric does not sufficiently follow the decrease in muscle volume and the compressive pressure decreases. Consequently, a large difference in compressive pressure between muscle contraction and relaxation is generated, and repeated increases and decreases in compressive pressure caused by body movement enhance a muscle pump effect and a massage effect. As a result, lymph flow can be promoted.
The garment 1 having higher stiffness allows the compressive pressure to decrease when the wearer is in a recumbent position (i.e., when the muscles are relaxed). Accordingly, even during nighttime sleep, the garment 1 does not cause an excessive feeling of tightness due to high compressive pressure, thereby improving wearing comfort and enabling safe use. That is, even when the muscles are relaxed, the garment 1 does not apply excessively high compressive pressure and can be used safely.

In the garment 1 according to the present embodiment, the front of the body-side knitted fabric portion 30 has the low-stretch region 71 covering the lower abdominal, groin, and genital regions when worn, and has the intermediate-stretch region 72 and the high-stretch region 73 that are regions other than the low-stretch region 71. As a result, the region covering the lower abdominal, groin, and genital regions when worn has relatively high stiffness, thereby relatively enhancing a muscle pump effect and a massage effect, as well as preventing wrinkling and suppressing biting into the skin. The other regions have relatively low stiffness and appropriate elasticity, thereby improving wearing comfort, and further preventing slippage and facilitating donning and doffing of the garment 1.

Thus, the garment 1 according to the present embodiment provides a comfortable compressive feeling without excessively increasing the compression pressure, and has relatively high stiffness, thereby enhancing a muscle pump effect and a massage effect and promoting lymph flow and thus enhancing the effect of alleviating lymphedema. Specifically, compression by the garment 1 prevents leakage and stagnation of lymph from lymphatic capillaries, and pressure by the garment 1 is applied in the direction in which lymph is to be guided, thereby directing lymph toward lymph nodes. Furthermore, the garment 1 has relatively high stiffness, thereby enhancing a muscle pump effect or a massage effect and further promoting lymph flow.

In the garment 1 according to the present embodiment, the elastic yarn 52 is knitted into the stitches of the base yarn 51 forming the outer raised portions 31 and the inner recessed portions 42 by rib knitting, whereas the elastic yarn 52 is formed as a miss stitch with respect to the stitches of the base yarn 51 forming the outer recessed portions 41 and the inner raised portions 32. Thus, the base yarn 51 and the elastic yarn 52 are interknitted. With this configuration, stretchability in the tubular diameter direction is enhanced at the portions where the elastic yarn 52 is formed as a miss stitch, while stretchability in the longitudinal direction and in the in-plane direction is suppressed at the portions where the elastic yarn 52 is formed as a knit stitch. As a result, stiffness is increased to enhance a muscle pump effect and a massage effect, thereby enhancing promotion of lymph flow, while sufficient stretchability is ensured to facilitate donning and doffing. In addition, even if the elastic yarn 52 is partially cut, the influence of a reduction in tension due to cutting of the elastic yarn 52 is minimized.

In the compression garment 1 according to the present embodiment, the Young's modulus in the transverse direction (E) is set to be greater than the Young's modulus in the longitudinal direction (E₀) perpendicular thereto, that is, E > E₀. If the Young's modulus in the transverse direction (E) and the Young's modulus in the longitudinal direction (E₀) are equal, elongation in both directions becomes identical. If E < E₀, the garment 1 is readily pulled in the direction in which it is worn on the human body (that is, a body-length direction), but is difficult to be pulled in the direction in which the tubular diameter expands for insertion of a leg of the human body. Conversely, when E > E₀, the garment 1 is readily stretched in the direction in which the tubular diameter expands for insertion of a leg and such stretching reduces resistance during insertion of the body, thereby improving ease of donning. Accordingly, it is preferable that the Young's modulus in the longitudinal direction (E₀) be smaller than the Young's modulus in the transverse direction (E). This configuration, when the garment 1 is put on or taken off, enables reduction of elastic deformation in the direction in which the garment 1 is worn on the human body (the body-length direction), and allows the garment 1 to be readily pulled and moved in the longitudinal direction along the body as desired.
That is, when an external force is applied to put on or taken off the garment 1, the garment 1 is less likely to elastically deform and stretch in the wearing direction (that is, the body-length direction), and can be moved by pulling force in the wearing direction. Further, since resistance to insertion of a leg is determined by the Young's modulus in the transverse direction (E), even an unskilled wearer can easily don the garment 1. After being worn, the garment 1 is resistant to slippage and to stretching in the longitudinal direction and the in-plane direction, and thus exhibits high stiffness.

In the garment 1 according to the present embodiment, the elastic yarn 52 is knitted into the loops of the base yarn 51 forming the outer raised portions 31 and the inner recessed portions 42, which appear convex on the outer surface and concave on the inner surface in rib knitting as textured knitting. Meanwhile, the elastic yarn 52 is formed as a miss stitch with respect to the loops of the base yarn 51 forming the inner raised portions 32 and the outer recessed portions 41, which appear concave on the inner surface and convex on the outer surface in the rib knitting. Accordingly, the floating yarn formed by miss stitches of the elastic yarn 52 is exposed at the outer recessed portions 41 on the outer surface, that is, the floating yarn of the elastic yarn 52 is overlaid on the outer recessed portions 41, whereas the elastic yarn 52 is not exposed at the inner raised portions 32 on the opposite inner surface of. Therefore, when the garment 1 is worn with the inner surface facing the skin, the floating yarn of the elastic yarn 52 formed by the miss stitches is exposed on the outer surface, and the elastic yarn 52 is not exposed on the inner surface of the inner raised portions 32 that come in contact with the skin. As a result, contact between the elastic yarn 52 and the skin is avoided. Further, the use of a thinner elastic yarn 52 than the base yarn 51 allows exposure of the elastic yarn 52 to be reduced even on the inner recessed portions 42. Accordingly, irritation to the wearer due to the material, elasticity, friction, and the like of the elastic yarn 52 can be suppressed, thereby improving wearing comfort. Preferably, the use of the base yarn 51 made of a cotton material improves water absorbency and feel against the skin, and can prevent moisture accumulation.

In the garment 1 according to the present embodiment, the region covering the lower abdominal, groin, and genital regions is configured as the low-stretch region 71 having higher stiffness than other regions. This configuration enables enhancement of a muscle pump effect and a massage effect in the region extending from the genital and groin regions to the lower abdominal region and promotion of lymph flow.

In the garment 1 according to the present embodiment, the body-side knitted fabric portion 30 has the raised and recessed portions (the raised portions 31, 32 and the recessed portions 41, 42) extending in the longitudinal direction, whereas the crotch-side knitted fabric portion 20 does not have the raised portions 31, 32 and the recessed portions 41, 42 and thus is configured as the discontinuous region in which continuity of the raised and recessed portions is interrupted. Accordingly, even when the lower limbs of the wearer move, the discontinuous region in the crotch-side knitted fabric portion 20, can deform so as to conform to the body shape and movement, and tightening, pulling, and friction of the knitted fabric are unlikely to occur. Further, the presence of the discontinuous region allows shifting in the width direction of the knitted fabric around the crotch-side knitted fabric portion 20 configured as the discontinuous region, namely, the knitted fabric of the body-side knitted fabric portion 30 and the leg-side knitted fabric portions 10R and 10L. As a result, even when the lower limbs of the wearer move, the inner raised portion 32 can be easily brought into close contact with the genital and groin regions.
Therefore, even in delicate regions such as the genital and groin regions, the garment 1 is less likely to be tightened and pulled, thereby being less likely to cause discomfort or foreign-body sensation.

Further, the anterior and posterior lymph flow around the genital and groin regions is guided to the non-raised and recessed region through the raised portions 31, 32 and the recessed portions 41, 42 of the front and back of the body-side knitted fabric portion 30, thereby facilitating guidance of lymph fluid around the genital region toward the left and right inguinal regions and enabling promotion of lymph flow into the normal lymph nodes. Accordingly, the effect of promoting lymph flow in the lower abdominal, groin, and genital regions is enhanced, and lymph edema (associated lymph leakage and cellulitis) in the lower abdominal, groin, and genital regions can be effectively prevented and alleviated. Further, in the front and back of the body-side knitted fabric portion 30, the portion that covers, during wearing, the regions extending from the lower abdominal regions toward the genital and groin regions is configured as the low-stretch region 71. The low-stretch region 71 is configured to cover the regions extending from the genital region to the left and right groin regions and the pelvic region. This configuration enables lymph fluid to be easily guided from the genital region toward the left and right groin regions and the pelvic region. Accordingly, lymph flow can be effectively promoted even in patients with severe deformation caused by lymph edema in the lower abdominal, groin, and genital regions.

The discontinuous region of the crotch-side knitted fabric portion 20 is formed by plain knitting, in which the elastic yarn 52 and the base yarn 51 is interknitted. Accordingly, this discontinuous region is easy to stretch, whereby excessive tension and pulling in the knitted fabric and friction between the knitted fabric and the skin are less likely to occur. As a result, the inner raised portion 32 of the knitted fabric around the discontinuous region, namely, the knitted fabric of the body-side knitted fabric portion 30 and the leg-side knitted fabric portions 10R and 10L can be easily brought into close contact with the genital and groin regions. Therefore, even in the delicate the genital and groin regions, excessive tension and pulling are less likely to occur, thereby being less likely to cause discomfort or foreign-body sensation, and enhancing promotion of lymph flow in the lower abdominal, groin, and genital regions. Accordingly, even when the lower limbs of the wearer move, the discontinuous region of the crotch-side knitted fabric portion 20, in which continuity of the raised and recessed portions is interrupted, can deform so as to conform to the body shape and movement, thereby facilitating close contact of the knitted fabric with the genital and groin regions.

Also in the low-stretch region 71 of the body-side knitted fabric portion 30, the elastic yarn 52 is knitted into the loops of the base yarn 51 forming the outer raised portion 31 and the inner recessed portion 42, which appear convex on the front surface and concave on the rear surface in rib knitting, so that the stitches forming the outer raised portion 31 and inner recessed portion 42 are formed of the stitches of the base yarn 51 and the elastic yarn 52. Meanwhile, the elastic yarn 52 is formed as a miss stitch with respect to the loops of the base yarn 51 forming the inner raised portion 32 and the outer recessed portion 41, which appear concave on the rear surface and convex on the front surface in rib knitting. The knit-stitches of the base yarn 51 and the elastic yarn 52, and the miss-stitches of the elastic yarn 52 with respect to the stitches of the base yarn 51 are repeated along the course direction. This configuration achieves moderate stiffness and stretchability in combination. As a result, the garment 1 has increased stiffness without causing a feeling of tightness, thereby enabling promotion of lymph flow through a muscle pump effect and a massage effect. Therefore, while ensuring wearing comfort, the garment 1 enables further promotion of lymph flow from the genital and groin regions to the lower abdominal region.

In the garment 1 according to the above embodiment, the region surrounding the low-stretch region 71 is configured as the intermediate-stretch region 72 having a higher elongation rate than the low-stretch region 71, and the high-stretch region 73 having a higher elongation rate than the intermediate-stretch region 72 is adjacent to the intermediate-stretch region 72. This stepwise variation in stretchability reduces loads applied to the base yarn 51 and the elastic yarn 52 due to differences in elongation rate, and suppresses deterioration of the knitted fabric.

The intermediate-stretch region 72 and the high-stretch region 73 have transferred stitches in the outer raised portions 31 and the inner recessed portions 42, thereby allowing the elastic yarn 52 to more readily exhibit its stretchability, making it less likely to cause a feeling of tightness, and also improving air permeability. Further, in the garment 1 according to the present embodiment, stretchability of the elastic yarn 52 is varied by varying the knitting structure. Since stretchability is not varied by changing the yarns, costs can be reduced and productivity can be improved.

The garment 1 according to the above embodiment is formed by flat knitting, which enables shape variation by varying the number of stitches and allows for greater design flexibility. By varying the number of wales in the longitudinal direction of the garment 1 in accordance with changes in the cross-sectional dimensions of the human body at the wearing portion, the garment 1 can accommodate differences in cross-sectional dimensions relative to the length of the human body. Further, varying the number of stitches in accordance with changes in the cross-sectional dimensions of the human body increases in design flexibility and can be set to any circumferential length, thereby facilitating design of an optimal diameter corresponding to the cross-sectional dimensions of the human body.

Thus, the garment 1 according to the present embodiment is integrally knitted by a seamless flat knitting machine and has no sewn portions, and is provided with the raised and recessed portions (the raised portions 31, 32 and the recessed portions 41, 42) that are alternately arranged in the circumferential direction and extend in the wale direction. With this configuration, the knitted fabric can be easily brought into close contact with the lower abdominal, groin, and genital regions, and can readily apply pressure to the lower abdominal, groin, and genital regions. As a result, lymph flow from capillaries to lymphatic vessels is guided, and lymph is guided from the region anterior to the anus of the buttocks, the thigh region, and the genital region toward the inguinal region, and further guided from there toward the pelvic region, thereby assisting lymph to flow into normally functioning lymph nodes. In addition, by forming the knitted fabric to be thick and stiff to increase stiffness, a muscle pump effect and a massage effect are enhanced, thereby promoting lymph flow. Therefore, without excessively increasing compression pressure, comfortable wear that allows continuous wearing is achieved, and long-term wearing prevents and alleviates lymphedema.

As described above, the pants-type compression garment 1 according to the above embodiment includes the pair of left and right leg-side knitted fabric portions 10R and 10L each having a small-diameter tubular shape, the crotch-side knitted fabric portion 20 connecting the pair of left and right leg-side knitted fabric portions 10R and 10L, and the body-side knitted fabric portion 30 having a large-diameter tubular shape and continuously extending from the pair of left and right leg-side knitted fabric portions 10R and 10L and the crotch-side knitted fabric portion 20. The pair of left and right leg-side knitted fabric portions 10R and 10L, the crotch-side knitted fabric portion 20, and the body-side knitted fabric portion 30 are integrally knitted by a seamless flat knitting machine.

According to the garment 1 of the above embodiment, the leg-side knitted fabric portions10R and 10L each having a small-diameter tubular shape, the crotch-side knitted fabric portion 20 connecting the leg-side knitted fabric portions 10R and 10L, and the body-side knitted fabric portion 30 having a large-diameter tubular shape and continuously extending from the leg-side knitted fabric portions 10R and 10L and the crotch-side knitted fabric portion 20, are integrally knitted by a seamless flat knitting machine. Accordingly, the garment 1 has no sewn portions and thus provides improved skin contact feel. Further, since the garment 1 has no sewn portions, which would cause tightening of the knitted fabric, the knitted fabric can be easily brought into close contact even with the genital and groin regions and its surrounding bent or curved portions of the body, and allows pressure to be readily applied thereto. Moreover, flat knitting, in which stitches are continuously knitted in the transverse direction, allows thicker yarns to be used as compared with circular knitting, thereby enabling formation of a thicker and stiffer knitted fabric. As a result, even at the genital and groin regions and its surrounding bent or curved portions of the body, the thick and stiff knitted fabric is less likely to bite into the skin due to wrinkles, thereby suppressing localized inhibition of lymph flow and improving wearing comfort. That is, the absence of sewn portions in the garment 1 reduces lifting of the knitted fabric due to due to tension during wearing, thereby facilitating application of appropriate pressure even to the lower abdominal, groin, and genital regions, which have bent or curved portions of the body. In addition, flat knitting allows a thicker and stiffer knitted fabric to be formed using a thicker yarn, thereby increasing stiffness of the knitted fabric. As a result, when worn, the knitted fabric overlaps the skin to function as a wall-like structure, thereby enabling generation of a muscle pump effect and a massage effect caused by movement of the body. Accordingly, the garment 1 can be comfortably worn and can facilitate lymph flow in the lower abdominal, groin, and genital regions. In particular, since the garment 1 is seamless, it provides a favorable feel against the skin. In addition, the garment 1, which is formed by flat knitting, allows a thicker knitted fabric having high stiffness to be formed using a thicker yarn. As a result, even without applying excessively high compression pressure, the garment 1 can promote lymph flow through a muscle pump effect and a massage effect caused by movement of the body. Therefore, the garment 1 reduces discomfort during wearing and provides comfortable compression, thereby enabling continuous comfortable wearing for a long period of time. Consequently, the garment 1 prevents and alleviates lymphedema (and related conditions such as lymph leakage and cellulitis) in the lower abdominal, groin, and genital regions, and suppresses worsening thereof.

Further, the garment 1 of the above embodiment enables lymph flow in the lower abdominal, groin, and genital regions to be promoted simply by wearing the garment 1, without the need to separately attach pads. Moreover, since the garment 1 is a knitted fabric integrally formed by a seamless flat knitting machine and has elasticity, the garment 1 can be easily donned and doffed.

In the garment 1 according to the above embodiment, the use of the base yarn 51, which forms the leg-side knitted fabric portions 10R and 10L, the crotch-side knitted fabric portion 20, and the body-side knitted fabric portion 30, having a fineness of preferably 1000 to 3000 denier, more preferably 1200 to 3000 denier, and still more preferably 1500 to 3000 denier allows the knitted fabric to be stiff and exhibit higher stiffness. This configuration enables enhancement of a muscle pump effect and a massage effect caused by movement of the body. In addition, this configuration enables the knitted fabric to be less likely to wrinkle and thus prevents localized inhibition of lymph flow caused by biting due to wrinkles, and further improve wearing comfort. Thus, this configuration improves wearing comfort and enhances promotion of lymph flow in t the lower abdominal, groin, and genital regions.

In the garment 1 according to the above embodiment, the leg-side knitted fabric portions 10R and 10L, the crotch-side knitted fabric portion 20, and the body-side knitted fabric portion 30 have a thickness preferably in a range of 2 mm to 15 mm, more preferably 2.5 mm to 14 mm, and still more preferably 2.5 mm to 13 mm. This configuration allows the knitted fabric to exhibit higher stiffness, thereby enabling enhancement of a muscle pump effect and a massage effect caused by movement of the body. In addition, this configuration enables the knitted fabric to be less likely to wrinkle and thus prevents localized inhibition of lymph flow caused by biting due to wrinkles, and further improve wearing comfort. Thus, this configuration improves wearing comfort and enhances promotion of lymph flow in the lower abdominal, groin, and genital regions. The above thickness refers to the thickness of the knitted fabric formed of the base yarn 51 used to knit the leg-side knitted fabric portions 10R and 10L, the crotch-side knitted fabric portion 20, and the body-side knitted fabric portion 30.

According to the garment 1 of the above embodiment, the leg-side knitted fabric portions 10R and 10L and the body-side knitted fabric portion 30 are formed by rib knitting as textured knitting, in which the raised portions 31, 32 and the recessed portions 41, 42 are alternately arranged in the circumferential direction and extend continuously in the wale direction. Accordingly, the raised and recessed portions are alternately arranged in the transverse direction on both the outer circumferential surface and the inner circumferential surface, and the inner recessed portions (grooves) 42 are present between adjacent inner raised portions 32. As a result, during wearing, the inner recessed portions42 between adjacent inner raised portions 32, that is, the spacing between the inner raised portions 32, can accommodate shape changes corresponding to the body shape. Accordingly, biting-in due to wrinkles and resultant inhibition of lymph flow are less likely to occur. In addition, since the inner raised portions 32 remain in constant close contact with the body and apply pressure thereto, pressure can be more readily applied to the lower abdominal, groin, and genital regions. Further, alternating arrangement of the raised portions 31 and 32 and the recessed portions 41 and 42 generates variations in pressure, thereby generating a massage effect and thus improving lymph flow. In particular, the recessed portions 41 and 42, which extend continuously in the wale direction between the raised portions 31 and 32, are oriented in the direction corresponding to the direction of lymph flow from the genital and groin regions toward the clavicular region of the human body, thereby not obstructing the lymph flow. Rather, during wearing, a longitudinal pulling force directed upward from the genital and groin sides is readily generated, thereby facilitating lymph flow more effectively.

According to the pants-type compression garment 1 of the above embodiment, the textured knitting is rib knitting. This rib knitting allows the spacing between the raised portions (inner raised portions 32) to be easily varied. Accordingly, even when the body moves, pressure can be continuously applied to body portions by the raised portions on the inner surface (the inner raised portions 32), thereby further enhancing promotion of lymph flow. In addition, rib knitting increases stretchability in the transverse direction and the circumferential direction, thereby facilitating donning and doffing of the garment 1.

According to the garment 1 of the above embodiment, the leg-side knitted fabric portions 10R and 10L and the body-side knitted fabric portion 30 are continuously knitted with a gradual decrease in the number of stitches from each leg-side knitted fabric portion 10R, 10L toward the body-side knitted fabric portion 30 at the side of the crotch-side knitted fabric portion 20. Accordingly, each leg-side knitted fabric portion 10R, 10L is configured to secure a relatively large area corresponding to the inner proximal thigh region of the human body when worn. Therefore, even with respect to the three-dimensional curved shape extending from the thigh region to the genital and groin regions, the knitted fabric is less likely to lift and can be more easily brought into close contact with and apply pressure to the regions extending from the thigh region to the genital and groin regions. As a result, the effect of promoting lymph flow in the lower abdominal, groin, and genital regions can be further enhanced.

In the garment 1 according to the above embodiment, the ratio of the number of wales of the crotch-side knitted fabric portion 20 to the maximum number of wales of each of the leg-side knitted fabric portions 10R and 10L preferably satisfies 2:8 ≤ (the number of wales of the crotch-side knitted fabric portion 20) : (the maximum number of wales of each leg-side knitted fabric portion 10R, 10L) ≤ 4:6, and more preferably satisfies 2.5:7.5 ≤ (the number of wales of the crotch-side knitted fabric portion 20) : (the maximum number of wales of each leg-side knitted fabric portion 10R, 10L) ≤ 3.5:5.5. Such a configuration prevents lifting of the knitted fabric even along the three-dimensional curved shape extending from the thigh region to the genital and groin regions, thereby allowing the knitted fabric to be more easily apply pressure to the regions extending from the thigh region to the genital and groin regions. That is, lifting caused by tightness of the knitted fabric on the genital and groin regions is prevented, and the inner raised portions 32 of the knitted fabric can be brought into close contact with the genital and groin regions and its surrounding bent or flexed areas. Accordingly, the effect of promoting lymph flow in the genital and groin regions can be further enhanced. The term "the number of wales" refers to the number of stitches (stitches connected in the longitudinal direction) within a predetermined interval in the transverse direction.

According to the garment 1 of the above embodiment, the leg-side knitted fabric portions 10R and 10L and the body-side knitted fabric portion 30 are configured such that the elastic yarn 52 is interknitted with the loops of the base yarn 51 by knit stitches and miss stitches. Accordingly, the portions where the elastic yarn 52 is knitted into the loops of the base yarn 51 are less likely to stretch, while the portions where the elastic yarn 52 is missed are readily stretchable without causing the elastic yarn 52 to be exposed on the inner surface. As a result, both comfortable compression and prevention of skin damage caused by the elastic yarn 52 can be achieved. Further, the use of the elastic yarn 52 increases stretchability, thereby facilitating donning and doffing, improving fit to the body, and enabling stable pressure to be applied during wear. In addition, varying the manner in which the elastic yarn 52 is interknitted with the loops of the base yarn 51 enables local variation in stretchability and stiffness.

According to the garment 1 of the above embodiment, the elastic yarn 52 is knitted into the loops of the base yarn 51 forming the stitches of the outer raised portions 31 and the inner recessed portions 42, which appear concave on the inner surface and convex on the outer surface in rib knitting as textured knitting. Meanwhile, the elastic yarn 52 is missed with respect to the loops of the base yarn 51 forming stitches of the outer recessed portions 41 and the inner raised portions 32, which appear convex on the inner surface and concave on the outer surface. Accordingly, the portions where the elastic yarn 52 is knitted into the loops of the base yarn 51 are less likely to stretch, while the portions where the elastic yarn 52 is missed are readily stretchable without causing the elastic yarn 52 to be exposed on the inner raised portions 32. As a result, the elastic yarn 52 is less likely to come into contact with the skin and cause skin damage. The wearer is less likely to feel irritation caused by the material, stretch, friction of the elastic yarn 52 and to experience skin inflammation caused by the elastic yarn 52 even during long-term wear. Accordingly, comfortable wearing is achieved. Furthermore, achieving both appropriate stretchability and stiffness enables both wearing comfort and enhanced promotion of lymph flow through a muscle pump effect and a massage effect.

According to the garment 1 of the above embodiment, the leg-side knitted fabric portions 10R and 10L and the body-side knitted fabric portion 30 are formed by textured knitting that forms the raised portions 31 and 32 and the recessed portions 41 and 42 alternately arranged in the course direction and extending continuously in the wale direction. Meanwhile, the crotch-side knitted fabric portion 20 is configured as the discontinuous region in which continuity of the raised portions 31 and 32 and the recessed portions 41 and 42 alternately arranged in the course direction is interrupted. Accordingly, even when the lower limbs move, the discontinuous region can accommodate shape changes of the body, thereby preventing excessive tension, pulling, and rubbing of the knitted fabric. Further, the knitted fabric around the discontinuous region, particularly the inner raised portions 32 of the body-side knitted fabric portion 30 and the leg-side knitted fabric portions 10R and 10L, can easily brought into close contact with the genital and groin regions. As a result, the garment 1 is less likely to cause discomfort or foreign-body sensation even in the delicate genital and groin regions. Further, the raised portions 31 and 32 and the recessed portions 41 and 42 of the front and back of the body-side knitted fabric portion 30 enable lymph flow around the anterior and posterior peripheries of the genital region to be guided into the discontinuous region, thereby directing the lymph flow toward the left and right inguinal regions. Accordingly, promotion of lymph flow in the lower abdominal, groin, and genital regions can be further enhanced, thereby effectively preventing and alleviating lymphedema (and related conditions such as lymph leakage or cellulitis) in the lower abdominal, groin, and genital regions.

In the crotch-side knitted fabric portion 20, the elastic yarn 52 is knitted into the loops of the base yarn 51 forming the crotch-side knitted fabric portion 20. This configuration allows the crotch-side knitted fabric portion 20 to be readily stretchable when the lower limbs move. Accordingly, even when the lower limbs move, the crotch-side knitted fabric portion 20 can effectively accommodate shape changes, thereby preventing excessive tension, pulling, and rubbing of the knitted fabric. Consequently, the body-side knitted fabric portion 30 and the pair of left and right leg-side knitted fabric portions 10R and 10L around the discontinuous region, particularly the inner raised portions 32, can be more easily brought into close contact with the genital and groin regions. As a result, promotion of lymph flow in the lower abdominal, groin, and genital regions can be further enhanced.

In the above embodiment, the discontinuous region of the crotch-side knitted fabric portion 20, in which continuity of the raised and recessed portions is interrupted, is formed by plain knitting. However, the knitting pattern is not limited to plain knitting as long as the raised and recessed portions are not continuous. For example, a knitting method in which plain knitting and rib knitting are formed in alternated courses may be employed.

According to the garment 1 of the above embodiment, the front of the body-side knitted fabric portion 30 has the low-stretch region 71 having a lower elongation rate than other regions in the portion that covers the lower abdominal, groin, and genital regions. This configuration increases stiffness in the region covering the lower abdominal, groin, and genital regions, thereby enabling enhancement of a muscle pump effect and a massage effect. Meanwhile, the portions other than the portion covering the lower abdominal, groin, and genital regions are more stretchable, thereby facilitating donning and doffing, preventing a feeling of tightness during wear, and further preventing slippage, looseness, and wrinkling. Accordingly, both wearing comfort and enhanced promotion of lymph flow in the lower abdominal, groin, and genital regions can be achieved.

In particular, according to the garment 1 of the above embodiment, the low-stretch region 71 is configured to have a lower elongation rate than other regions by reducing the proportion of transferred stitches in the portions where the elastic yarn 52 is knitted into the loops of the base yarn 51. Since the elongation rate is varied by varying the proportion of transferred stitches, not by changing the base yarn 51 or the elastic yarn 52, the stitch structure is readily stabilized, productivity is improved, and costs can be reduced.

According to the garment 1 of the above embodiment, the low-stretch region 71 is surrounded by the intermediate-stretch region 72 having a higher elongation rate than the low-stretch region 71, and the high-stretch region 73 having a higher elongation rate than the intermediate-stretch region 72 is adjacent to the intermediate-stretch region 72. This gradual variation in stretchability reduces loads applied to the base yarn 51 and the elastic yarn 52 due to differences in elongation rate and allows the garment 1 to hold its shape.

In another embodiment, the knitting pattern may be kept constant without imparting variations in stretchability of the knitted fabric. Alternatively, the stretchability of the knitted fabric may be varied by changing the types of the base yarn 51 or the elastic yarn 52, or by changing the knitting structure in ways other than stitch transfer.

According to the garment 1 of the above embodiment, the body-side knitted fabric portion 30 has a shorter length on the front portion than on the back portion. This configuration prevents lifting and wrinkling of the front of the knitted fabric that covers the abdominal region during wearing and thus allows the knitted fabric to facilitate application of pressure to the lower abdominal, groin, and genital regions. Further, this configuration does not cause a feeling of tightness on the buttock region. Thus, both wearing comfort and enhanced promotion of lymph flow in the lower abdominal, groin, and genital regions can be achieved.

According to the garment 1 of the above embodiment, the body-side knitted fabric portion 30 has a shorter length on the front portion than on the back portion by reducing the proportion of transferred stitches in the portions where the elastic yarn 52 is knitted into the loops of the base yarn 51, as compared with other portions. Since the length is adjusted by varying the proportion of transferred stitches rather than by changing the base yarn 51 or the elastic yarn 52, the stitch structure is readily stabilized, productivity is improved, and costs can be reduced.
Further, according to the garment 1 of the above embodiment, the formation of transferred stitches enhances air permeability, thereby reducing moisture accumulation during wearing.

In another embodiment, the knitting pattern on the front and the back of the body-side knitted fabric portion 30 may be kept the same. The length of the knitted fabric between the front portion and the back portion may be same. Alternatively, the length of the knitted fabric may be different between the front portion and the back portion by changing the type of the base yarn 51 or the elastic yarn 52, or by varying the knitting structure in ways other than stitch transfer.

Further, in the garment 1 according to the above embodiment, the body-side knitted fabric portion 30 is knitted such that the number of wales is gradually reduced from the central portion of its length toward the upper side. Accordingly, the body-side knitted fabric portion 30 narrows in width toward the upper side to conform to the waistline shape of the human body. As a result, the garment 1 is prevented from downward slippage and enables pressure to be readily applied to the lower abdominal, groin, and genital regions. In the above embodiment, the body-side knitted fabric portion 30 is knitted such that the number of wales decreases toward the upper side at corresponding front portion and the back portion. As a result, on both the front and the back of the body-side knitted fabric portion 30, the outer raised portions 31 and the inner recessed portions 42 on both left and right sides thereof are inclined slightly inward toward the upper side from a substantially intermediate portion in the longitudinal direction, and some of the raised portions 31and 32 and the recessed portions 41 and 42 terminate midway as they extend upward. In another embodiment, the body-side knitted fabric portion 30 may be knitted such that the number of wales decreases at the lateral edges of the front and the back of the knitted fabric. Further, in another embodiment, the body-side knitted fabric portion 30 may have the constant number of wales toward the upper side without decreasing the number of wales, or may have variation in the circumferential dimension by changing the knitting structure or the type of knitting yarn.

According to the garment 1 of the above embodiment, the raised and recessed portions formed by rib knitting as textured knitting in the leg-side knitted fabric portions 10R and 10L and the body-side knitted fabric portion 30 are linear raised portions 31and 32 and recessed portions 41 and 42 extending in the wale direction, that is, in the longitudinal direction perpendicular to the knitting width. In the body-side knitted fabric portion 30 and the leg-side knitted fabric portions 10R and 10L of the above embodiment, the raised portions and recessed portions (raised portions 31 and 32 and recessed portions 41 and 42) are alternately arranged over the entire circumference and extend in the direction perpendicular to the knitting width. That is, the body-side knitted fabric portion 30 and the leg-side knitted fabric portions 10R and 10L have ridge-like structure in which the raised portions and the recessed portions are alternately continuous along the circumference. Each of the raised portions and recessed portions (the raised portions 31 and 32 and the recessed portions 41 and 42) extends straight along the direction perpendicular to the knitting width. The ends of the raised portions and recessed portions are located at the upper opening of the body-side knitted fabric portion 30, at the lower openings 11R and 11L of the leg-side knitted fabric portions 10R and 10L, at the closed side of the crotch-side knitted fabric portion 20, and at the above-described decreased stitch portions. Accordingly, during wearing, the raised portions 31 and 32 and the recessed portions 41 and 42 extend along the direction of lymph flow and are less likely to obstruct lymph flow, thereby facilitating promotion of lymph flow from the thigh region and the genital and groin regions toward the lymph return site below the clavicle.

In the above embodiment, the leg-side knitted fabric portions 10R and 10L and the body-side knitted fabric portion 30 are knitted with a 3×3 rib configuration, in which three knit stitches and three purl stitches are alternately repeated along the wale direction. In another embodiment, the stitch cycle of the knit stitches and the purl stitches is not particularly limited. For example, the numbers of the knit stitches and purl stitches may be the same, that is, the number of the stitches forming the outer raised portions 31 and the inner recessed portions 42 may be the same as the number of the stitches forming the outer recessed portions 41 and the inner raised portions 32, and rib knitting such as 1 (knit) × 1 (purl), 2 (knit) × 2 (purl), 4 (knit) × 4 (purl), 5 (knit) × 5 (purl), 6 (knit) × 6 (purl), 7 (knit) × 7 (purl), 8 (knit) × 8 (purl), or the like may be employed. Alternatively, the numbers of the knit stitches and the purl stitches may be different, such as 2 (knit) × 3 (purl) or 2 (knit) × 4 (purl), or the numbers of the knit stitches and the purl stitches may be varied periodically or irregularly in the circumferential direction.

In the above embodiment, the raised and recessed portions (raised portions 31 and 32 and recessed portions 41 and 42) are formed on both the outer surface and the inner surface by rib knitting as textured knitting. In another embodiment, the textured knitting is not limited to rib knitting, as long as the raised and recessed portions can be formed on at least one of the outer surface and the inner surface. For example, the raised and recessed portions may be formed by other knitting structures such as seed stitch knitting, pique knitting, or waffle knitting.

In the leg-side knitted fabric portions 10R and 10L and the body-side knitted fabric portion 30 of the above embodiment, the base yarn 51 and the elastic yarn 52 are knitted in alternating courses. In another embodiment, the knitting pattern of the base yarn 51 and the elastic yarn 52 are not limited thereto. For example, the base yarn 51 and the elastic yarn 52 may be alternately knitted at intervals of every two courses, every three courses, or the like, or may be alternately knitted at irregular intervals in the wale direction. In the above embodiment, in order to reduce contact of the elastic yarn 52 with the wearer's skin, the elastic yarn 52 is knitted into the loops forming the outer raised portions 31 and the inner recessed portions 42 of the rib knitting formed by the base yarn 51, while being missed with respect to the loops forming the inner raised portions 32 and the outer recessed portions 41. In another embodiment, the manner in which the elastic yarn 52 is incorporated into the base yarn 51 forming the rib knitting is not limited thereto. For example, the elastic yarn 52 may be entirely interknitted by knit stitches or may be incorporated by inlay knitting.

The leg-side knitted fabric portions 10R and 10L and the body-side knitted fabric portion 30 are configured such that the elastic yarn 52 is interknitted with the loops of the base yarn 51 by knit stitches and miss stitches. This configuration allows the portions where the elastic yarn 52 is knitted into the loops of the base yarn 51 to have reduced stretchability, while allowing the portions where the elastic yarn 52 is missed to be easily stretchable and preventing the elastic yarn 52 from being exposed on the inner surface. Accordingly, both comfortable compression and prevention of skin damage caused by the elastic yarn 52 can be achieved.
The elastic yarn 52 is knitted into the loops of the base yarn 51 forming the stitches of the outer raised portions 31 and the inner recessed portions 42, which appear concave on the inner surface and convex on the outer surface in rib knitting. Meanwhile, the elastic yarn 52 is missed with respect to the loops of the base yarn 51 forming the stitches of the outer recessed portions 41 and the inner raised portions 32, which appear convex on the inner surface and concave on the outer surface. This configuration allows the portions where the elastic yarn 52 is knitted into the loops of the base yarn 51 to have reduced stretchability, while allowing the portions where the elastic yarn 52 is missed to be easily stretchable and preventing the elastic yarn 52 from being exposed at the inner convex portions 32. As a result, contact of the elastic yarn 52 with the skin is eliminated, thereby suppressing skin damage caused by the elastic yarn 52. Therefore, the garment 1 is less likely to cause irritation to the wearer due to the material, stretchability, friction, or the like of the elastic yarn 52, and even during long-term wear, to cause skin inflammation due to the elastic yarn 52. Accordingly, the garment 1 achieves wearing comfort. Furthermore, achieving both appropriate stretchability and stiffness enables both wearing comfort and enhanced promotion of lymph flow through a muscle pump effect and massage effect.

In the above embodiment, the elastic yarn 52 is interknitted with the rib-knit base structure formed of the base yarn 51. In addition to elastic deformation due to rib knitting, the interknitting of the elastic yarn 52 increases elasticity and compression force, thereby facilitating donning and enabling application of stable compression force. Alternatively, compression force and elasticity may be enhanced by using a highly elastic yarn as the base yarn 51.

In the above embodiment, an example of the girdle-type garment as the pants-type compression garment is described. In another embodiment, the compression garment may also be applied to a stocking-type or pantyhose-type garment.

In the above embodiment, a WHOLEGARMENT^{®} (registered trademark) flat knitting machine is employed as an example. In another embodiment, the seamless flat knitting machine is not limited thereto, and may be of any type, including a computer-controlled type or a manual operated type.

### REFERENCE SIGNS LIST

- 1: a pants-type compression garment
- 10R, 10L: a pair of left and right leg-side knitted fabric portions
- 20: a crotch-side knitted fabric portion
- 30: a body-side knitted fabric portion
- 31: outer raised portions
- 32: inner raised portions
- 41: outer recessed portions
- 42: inner recessed portions
- 51: a base yarn
- 52: an elastic yarn
- 71: a low-stretch region

## Claims

1. A pants-type compression garment, comprising:
a pair of left and right leg-side knitted fabric portions each having a small-diameter tubular shape;
a crotch-side knitted fabric portion connecting the pair of left and right leg-side knitted fabric portions; and
a body-side knitted fabric portion having a large-diameter tubular shape and continuously extending from the pair of left and right leg-side knitted fabric portions and the crotch-side knitted fabric portion,
wherein the pair of left and right leg-side knitted fabric portions, the crotch-side knitted fabric portion, and the body-side knitted fabric portion are integrally knitted by a seamless flat knitting machine.

2. The pants-type compression garment according to claim 1, wherein
the pair of left and right leg-side knitted fabric portions and the body-side knitted fabric portion are knitted by textured knitting forming raised portions and recessed portions that are alternately arranged in a circumferential direction and continuously extend in a wale direction.

3. The pants-type compression garment according to claim 1, wherein
the pair of left and right leg-side knitted fabric portions and the body-side knitted fabric portion are continuously knitted, on a side of the crotch-side knitted fabric portion, by gradually decreasing stitches from each of the leg-side knitted fabric portions toward the body-side knitted fabric portion.

4. The pants-type compression garment according to claim 1, wherein
the body-side knitted fabric portion has a low-stretch region having a lower elongation rate than other regions, the low-stretch region configured to cover a region extending from groin and genital regions to a lower abdominal region of a human body.

5. The pants-type compression garment according to claim 1, wherein
the pair of left and right leg-side knitted fabric portions and the body-side knitted fabric portion are knitted using a base yarn and an elastic yarn, the elastic yarn interknitted with loops of the base yarn by knit stitches and miss stitches.

6. The pants-type compression garment according to claim 1, wherein
the pair of left and right leg-side knitted fabric portions and the body-side knitted fabric portion are knitted using a base yarn and an elastic yarn, the elastic yarn interknitted with loops of the base yarn by knit stitches and miss stitches;
the body-side knitted fabric portion has a low-stretch region having a lower elongation rate than other regions, the low-stretch region to cover a region extending from groin and genital regions to a lower abdominal region of a human body; and
the low-stretch region has a lower elongation rate by having a smaller proportion of transferred stitches, in portions where the elastic yarn is knitted into the loops of the base yarn, than in the other regions.

7. The pants-type compression garment according to claim 1, wherein
the body-side knitted fabric portion has a front portion shorter in length than a back portion.

8. The pants-type compression garment according to claim 1, wherein
the pair of left and right leg-side knitted fabric portions and the body-side knitted fabric portion are knitted using a base yarn and an elastic yarn, the elastic yarn interknitted with loops of the base yarn by knit stitches and miss stitches; and
the body-side knitted fabric portion has a front portion shorter in length than a back portion by having, in portions where the elastic yarn is knitted into the loops of the base yarn, a smaller proportion of transferred stitches on the front portion than on the back portion.

9. The pants-type compression garment according to claim 1, wherein
the pair of left and right leg-side knitted fabric portions, the crotch-side knitted fabric portion, and the body-side knitted fabric portion are knitted using a base yarn having a fineness in a range of 1000 to 3000 denier.

10. The pants-type compression garment according to claim 2, wherein
the textured knitting is rib knitting.

11. The pants-type compression garment according to claim 2, wherein
the pair of left and right leg-side knitted fabric portions and the body-side knitted fabric portion are knitted using a base yarn and an elastic yarn, the elastic yarn interknitted with loops of the base yarn by knit stitches and miss stitches;
the elastic yarn is knitted into the loops of the base yarn that appear concave on an inner surface and convex on an outer surface; and
the elastic yarn is missed with respect to the loops of the base yarn that appear convex on the inner surface and concave on the outer surface.

12. The pants-type compression garment according to claim 2, wherein
the crotch-side knitted fabric portion is a discontinuous region in which continuity of the raised portions and the recessed portions is interrupted.

13. The pants-type compression garment according to claim 12, wherein
the crotch-side knitted fabric portion is knitted using a base yarn and an elastic yarn, the elastic yarn interknitted with loops of the base yarn by knit stitches.
